# EUROPEAN PATENT APPLICATION

(11) **EP 4 541 893 A1**
(43) Date of publication of application: **23.04.2025**
(21) Application number: 23823159.1
(22) Date of filing: 13.06.2023
(51) Int. Cl.: C12N 15/113, A61K 31/713, A61P 1/16, A61P 3/06, A61K 48/00, A61K 31/7105, A61P 9/00

(54) **SIRNA MOLECULE FOR REGULATING PCSK9 GENE ACTIVITY**

(30) Priority: 14.06.2022 CN 202210678937
(71) Applicant: Rona Bioscience, Limited, Admiralty, Hong Kong (HK)
(72) Inventor: HUANG, Jinyu, Shanghai 201315 (CN); GUO, Hongli, Shanghai 201315 (CN); CAI, Guoqing, Shanghai 201315 (CN); YANG, Xiaoyan, Shanghai 201315 (CN)
(74) Representative: Cabinet Becker et Associés
(86) International application number: PCT/CN2023/100015
(87) International publication number: WO 2023/241591

(57) **Abstract**

Provided are a small interfering RNA (siRNA) used to inhibit the expression of proprote in convertase subtilisin/kexin type 9 (PCSK9) in a cell, a vector comprising nucleotides encoding same, and a cell, as well as a method using the siRNA, the vector or the cell for the treatment of diseases or symptoms associated with the expression of PCSK9 in a subject.

## Description

### FIELD OF TECHNOLOGY

The present invention relates to the field of RNA interference.

### BACKGROUND

Proprotein convertase subtilisin/kexin type 9 (PCSK9) is a serine protease, which is mainly expressed in livers, small intestines and kidneys, is a regulatory targeting substance for a low density lipoprotein, and mainly plays a role by regulating expression and secretion activity of a low density lipoprotein receptor on the livers.

Cholesterol in the blood is mainly synthesized in the livers, and decomposition of excess cholesterol is also mainly carried out in the livers. The low density lipoprotein receptor (LDLR) that exists on surfaces of the livers can bind to the cholesterol circulating back to the livers and decompose the cholesterol into bile acid which is then excreted out of the body through intestines. As the protease synthesized in the livers, the PCSK9 can bind to the LDLR and promote the LDLR to enter a hepatocyte lysosome for decomposition, thereby decreasing the amount of the LDLR. On the contrary, inhibition of PSCK9 enzyme activity can increase the amount of the LDLR, thereby enhancing the abilities to uptake and decompose the cholesterol, and ultimately reducing the content of the cholesterol.

The PCSK9 can bind to the low density lipoprotein receptor through an extracellular pathway and an intracellular pathway and promote degradation of the LDLR. In the extracellular pathway, the PCSK9 secreted into an extracellular cell binds to the LDLR on a cell membrane, such that the LDLR is endocytosed to form an endosome and finally enters a lysosome for degradation. In the intracellular pathway, the PCSK9 secreted from a Golgi apparatus enters cytoplasm and directly binds to the LDLR, such that the LDLR directly enters a lysosomal degradation pathway in a cell.

Therefore, the PCSK9 is an effective target for treatment of hyperlipidemia and atherosclerosis. Although hypercholesterolemia itself is asymptomatic, long-term elevated serum cholesterol can lead to atherosclerosis. After decades, chronically elevated cholesterol leads to the formation of atherosclerotic plaques in arteries, and the atherosclerosis can lead to progressive stenosis or even complete occlusion of the arteries involved. In addition, smaller plaques may rupture and cause clot formation and vascular occlusion, leading to, for example, myocardial infarction and/or stroke. When the stenosis or the occlusion is gradually formed, blood supply to tissues and organs is slowly reduced until organ functions are impaired.

In recent years, inhibitors with the protease PCSK9 as a target have become novel therapeutic drugs for these diseases. As a new treatment method, an siRNA has great development potential. The siRNA acts on an mRNA in a cell and can directly silence a target gene compared with traditional small molecule drugs, and thus can fundamentally prevent occurrence and development of diseases more efficiently. However, due to poor stability, easy degradation by a nuclease in vivo, difficult absorption by tissues, difficult uptake by cells, easy generation of an off-target effect and other defects, the siRNA is limited in clinical application. At present, an siRNA capable of effectively inhibiting expression of a PCSK9 gene in a cell is required urgently.

### SUMMARY

The present invention provides a novel small interfering RNA (siRNA) for inhibiting expression of proprotein convertase subtilisin/kexin type 9 (PCSK9) in a cell, a vector, a kit and a pharmaceutical composition thereof, as well as a method for inhibiting or reducing expression of a PCSK9 gene or treating diseases or symptoms associated with the expression of PCSK9 by using the siRNA, the vector, the kit or the pharmaceutical composition.

In a first aspect, the present invention provides a small interfering RNA (siRNA) for inhibiting expression of proprotein convertase subtilisin/kexin type 9 (PCSK9) in a cell. The siRNA includes a sense strand and an antisense strand that form a double-stranded region, where lengths of the sense strand and the antisense strand are each independently 15-30 nucleotides, and the antisense strand includes a nucleotide sequence of at least 15 consecutive nucleotides of a nucleotide sequence shown in any one of SEQ ID NO: 105-208. In some specific embodiments, the sense strand includes a nucleotide sequence of at least 15 consecutive nucleotides of a nucleotide sequence shown in any one of SEQ ID NO: 1-104.

In some embodiments, the lengths of the sense strand and the antisense strand are each independently 17-27 nucleotides, preferably 19-25 nucleotides, more preferably 19-23 nucleotides.

In some embodiments, a length of the double-stranded region is 15-25 base pairs, preferably 17-21 base pairs, more preferably 19-21 base pairs.

In some embodiments, one or two of the sense strand and the antisense strand include a 3' overhang and/or a 5' overhang having at least 1 nucleotide, for example, one or two of the sense strand and the antisense strand include a 3' overhang and/or a 5' overhang having at least 1 nucleotide. In some preferred embodiments, the antisense strand includes a 3' overhang and/or a 5' overhang having at least 2 nucleotides, and preferably, the antisense strand includes a 3' overhang and/or a 5' overhang having 2 nucleotides.

In some embodiments, the antisense strand includes a nucleotide sequence of at least 16 consecutive nucleotides, a nucleotide sequence of at least 17 consecutive nucleotides, a nucleotide sequence of at least 18 consecutive nucleotides, a nucleotide sequence of at least 19 consecutive nucleotides, or a nucleotide sequence of at least 20 consecutive nucleotides of the nucleotide sequence shown in any one of SEQ ID NO: 105-208, and preferably, the antisense strand includes the nucleotide sequence shown in any one of SEQ ID NO: 105-208.

In some embodiments, the sense strand includes a nucleotide sequence of at least 16 consecutive nucleotides, a nucleotide sequence of at least 17 consecutive nucleotides, a nucleotide sequence of at least 18 consecutive nucleotides, a nucleotide sequence of at least 19 consecutive nucleotides, or a nucleotide sequence of at least 20 consecutive nucleotides of the nucleotide sequence shown in any one of SEQ ID NO: 1-104, and preferably, the sense strand includes the nucleotide sequence shown in any one of SEQ ID NO: 1-104.

In some embodiments, the siRNA includes a sense strand sequence and an antisense strand sequence that are paired as shown in Table 3.

In some embodiments, the antisense strand includes a nucleotide sequence of at least 15 consecutive nucleotides, a nucleotide sequence of at least 16 consecutive nucleotides, a nucleotide sequence of at least 17 consecutive nucleotides, a nucleotide sequence of at least 18 consecutive nucleotides, a nucleotide sequence of at least 19 consecutive nucleotides, or a nucleotide sequence of at least 20 consecutive nucleotides of a nucleotide sequence shown in any one of SEQ ID NO: 195, 203, 204, 142, 207, 143, 197, 205, 206, 144, 147, 148, 149, and 150, and preferably, the antisense strand includes the nucleotide sequence shown in any one of SEQ ID NO: 195, 203, 204, 142, 207, 143, 197, 205, 206, 144, 147, 148, 149, and 150.

In some embodiments, the sense strand includes a nucleotide sequence of at least 15 consecutive nucleotides, a nucleotide sequence of at least 16 consecutive nucleotides, a nucleotide sequence of at least 17 consecutive nucleotides, a nucleotide sequence of at least 18 consecutive nucleotides, a nucleotide sequence of at least 19 consecutive nucleotides, or a nucleotide sequence of at least 20 consecutive nucleotides of a nucleotide sequence shown in any one of SEQ ID NO: 91, 99, 100, 38, 103, 39, 93, 101, 102, 40, 43, 44, 45, and 46, and preferably, the sense strand includes the nucleotide sequence shown in any one of SEQ ID NO: 91, 99, 100, 38, 103, 39, 93, 101, 102, 40, 43, 44, 45, and 46.

In some embodiments, (a) the antisense strand includes a nucleotide sequence shown in SEQ ID NO: 195, and the sense strand includes a nucleotide sequence shown in SEQ ID NO: 91;
(b) the antisense strand includes a nucleotide sequence shown in SEQ ID NO: 203, and the sense strand includes a nucleotide sequence shown in SEQ ID NO: 99;
(c) the antisense strand includes a nucleotide sequence shown in SEQ ID NO: 204, and the sense strand includes a nucleotide sequence shown in SEQ ID NO: 100;
(d) the antisense strand includes a nucleotide sequence shown in SEQ ID NO: 142, and the sense strand includes a nucleotide sequence shown in SEQ ID NO: 38;
(e) the antisense strand includes a nucleotide sequence shown in SEQ ID NO: 207, and the sense strand includes a nucleotide sequence shown in SEQ ID NO: 103;
(f) the antisense strand includes a nucleotide sequence shown in SEQ ID NO: 143, and the sense strand includes a nucleotide sequence shown in SEQ ID NO: 39;
(g) the antisense strand includes a nucleotide sequence shown in SEQ ID NO: 197, and the sense strand includes a nucleotide sequence shown in SEQ ID NO: 93;
(h) the antisense strand includes a nucleotide sequence shown in SEQ ID NO: 205, and the sense strand includes a nucleotide sequence shown in SEQ ID NO: 101;
(i) the antisense strand includes a nucleotide sequence shown in SEQ ID NO: 206, and the sense strand includes a nucleotide sequence shown in SEQ ID NO: 102;
(j) the antisense strand includes a nucleotide sequence shown in SEQ ID NO: 144, and the sense strand includes a nucleotide sequence shown in SEQ ID NO: 40; or
(k) the antisense strand includes a nucleotide sequence shown in SEQ ID NO: 147, and the sense strand includes a nucleotide sequence shown in SEQ ID NO: 43;
(l) the antisense strand includes a nucleotide sequence shown in SEQ ID NO: 148, and the sense strand includes a nucleotide sequence shown in SEQ ID NO: 44;
(m) the antisense strand includes a nucleotide sequence shown in SEQ ID NO: 149, and the sense strand includes a nucleotide sequence shown in SEQ ID NO: 45; or
(n) the antisense strand includes a nucleotide sequence shown in SEQ ID NO: 150, and the sense strand includes a nucleotide sequence shown in SEQ ID NO: 46.

In some embodiments, substantially all nucleotides of the sense strand and substantially all nucleotides of the antisense strand are modified nucleotides, or all the nucleotides of the sense strand and all the nucleotides of the antisense strand are modified nucleotides.

In some specific embodiments, the sense strand and the antisense strand each independently include one or more nucleotide modifications selected from the following group: 2'-O-methyl modified nucleotides, 2'-fluoro modified nucleotides, 2'-deoxy- modified nucleotides, inosine ribonucleotides, abasic nucleotides, reverse abasic deoxyribonucleotides, nucleotides containing a thiophosphate group, vinyl phosphate modified nucleotides, locked nucleotides, 2'-amino- modified nucleotides, 2'-alkyl- modified nucleotides, morpholino nucleotides, aminophosphates, unnatural bases containing nucleotides, and terminal nucleotides or deoxyribonucleotides linked to cholesterol-based derivatives or a sebacamide dodecanoate group.

In some preferred embodiments, the sense strand and the antisense strand each independently include one or more nucleotide modifications selected from the following group: 2'-O-methyl modified nucleotides, 2'-fluoro modified nucleotides, abasic nucleotides, or nucleotides containing a thiophosphate group. In some preferred embodiments, the sense strand and/or the antisense strand includes at least 2 2'-fluoro modified nucleotides. In some preferred embodiments, the sense strand and/or the antisense strand includes at least 8 2'-O-methyl modified nucleotides. In some preferred embodiments, a 3' end and/or a 5' end of the sense strand and/or the antisense strand includes 1-5 thiophosphate groups, preferably 2-3 thiophosphate groups.

In some preferred embodiments, the antisense strand includes a modified nucleotide sequence shown in Table 5, and/or the sense strand includes a modified nucleotide sequence shown in Table 4. In some preferred embodiments, the siRNA includes a modified sense strand sequence and a modified antisense strand sequence that are paired as shown in Table 6.

In some specific embodiments, (a) the sense strand includes CmsUmsAmGmAmCmCfUmGfUmdTUmUmGmCmUmUmUmUmGmUm, and the antisense strand includes AmsCfsAmAfAfAfGmCfAmAfAmAfCmAfGmGfUmCfUmAmGmsAmsAm;
(b) the sense strand includes CmsUmsAmGmAmCmCfUmGfUmdTUmUmGmCmUmUmUmUmGmsUm, and the antisense strand includes AmsCfsAmAfAfAfGmCfAmAfAmAfCmAfGmGfUmCfUmAmGmsAmsAm;
(c) the sense strand includes UmsGmsUmUmUmUmGfCfUfUmUmUmGmUmAmAmCmsUmsUm, and the antisense strand includes AmsAfsGmUfUmAfCmAfAmAfAmGfCmAfAmAfAmCfAmsGfsGm;
(d) the sense strand includes UmsGmsUmUmUmUmGfCfUfUmUmUmGmUmAmAmCmUmsUm, and the antisense strand includes AmsAfsGmUfUmAfCmAfAmAfAmGfCmAfAmAfAmCfAmsGfsGm;
(e) the sense strand includes GmsUmsUmUmUmGmCfUfUfUmUmGmUmAmAmCmUmsUmsAm, and the antisense strand includes UmsAfsAmGfUmUfAmCfAmAfAmAfGmCfAmAfAmAfCmsAfsGm;
(f) the sense strand includes GmsUmsUmUmUmGmCfUfUfUmUmGmUmAmAmCmUmsUmsAm, and the antisense strand includes UmsAfsAmGfUmUfAmCfAmAfAmAfGmCfAmAfAmAfCmsAfsGm;
(g) the sense strand includes GmsUmsUmUmUmGmCfUfUfUmUmGmUmAmAmCmUmUmsAm, and the antisense strand includes UmsAfsAmGfUmUfAmCfAmAfAmAfGmCfAmAfAmAfCmsAfsGm;
(h) the sense strand includes GmsUmsUmUmUmGmCfUfUfUmUmGmUmAmAmCmUmUmAm, and the antisense strand includes UmsAfsAmGfUmUfAmCfAmAfAmAfGmCfAmAfAmAfCmsAfsGm;
(i) the sense strand includes UmsUmsUmUmGmCmUfUfUfUmGmUmAmAmCmUmUmsGmsAm, and the antisense strand includes UmsCfsAmAfGmUfUmAfCmAfAmAfAmGfCmAfAmAfAmsCfsAm;
(j) the sense strand includes UmsUmsUmUmGmCmUfUfUfUmGmUmAmAmCmUmUmsGmsAm, and the antisense strand includes UmsCfsAmAfGmUfUmAfCmAfAmAfAmGfCmAfAmAfAmsCfsAm;
(k) the sense strand includes UmsGmsCmUmUmUmUfGfUfAmAmCmUmUmGmAmAmsGmsAm, and the antisense strand includes UmsCfsUmUfCmAfAmGfUmUfAmCfAmAfAmAfGmCfAmsAfsAm;
(l) the sense strand includes GmsCmsUmUmUmUmGfUfAfAmCmUmUmGmAmAmGmsAmsUm, and the antisense strand includes AmsUfsCmUfUmCfAmAfGmUfUmAfCmAfAmAfAmGfCmsAfsAm;
(m) the sense strand includes CmsUmsUmUmUmGmUfAfAfCmUmUmGmAmAmGmAmsUmsAm, and the antisense strand includes UmsAfsUmCfUmUfCmAfAmGfUmUfAmCfAmAfAmAfGmsCfsAm; or
(n) the sense strand includes UmsUmsUmUmGmUmAfAfCfUmUmGmAmAmGmAmUmsAmsUm, and the antisense strand includes AmsUfsAmUfCmUfUmCfAmAfGmUfUmAfCmAfAmAfAmsGfsCm.

In some embodiments, the siRNA is further conjugated with a ligand moiety containing N-acetylgalactosamine through a phosphate group or a thiophosphate group, and preferably, the sense strand of the siRNA is conjugated with the ligand moiety through the phosphate group or the thiophosphate group. In some preferred embodiments, a 3' end of the sense strand is conjugated with the ligand moiety through the phosphate group or the thiophosphate group. In other preferred embodiments, a 5' end of the sense strand is conjugated with the ligand moiety through the phosphate group or the thiophosphate group.

In some embodiments, the ligand moiety includes a conjugated group shown in Formula (X'): 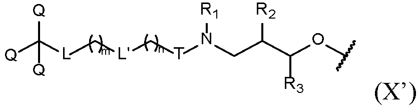 where, 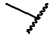 represents a position connected with a biomolecule;
Q is independently H, 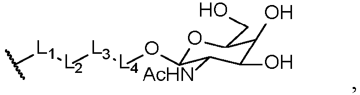 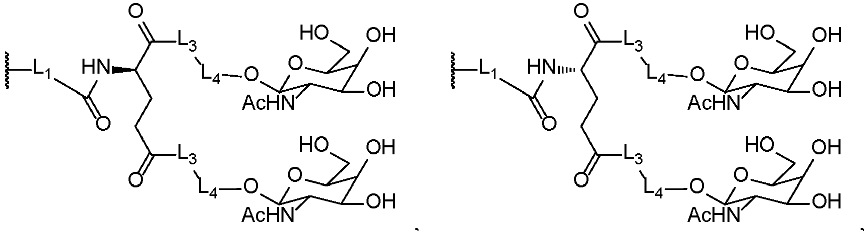 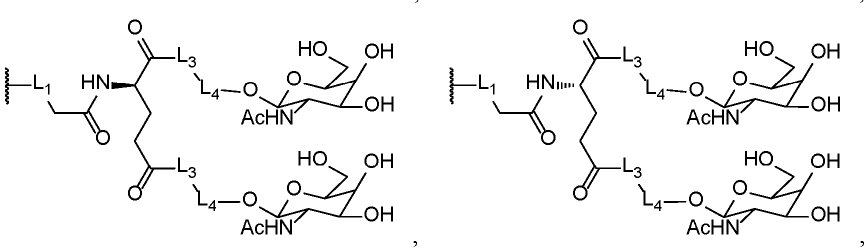 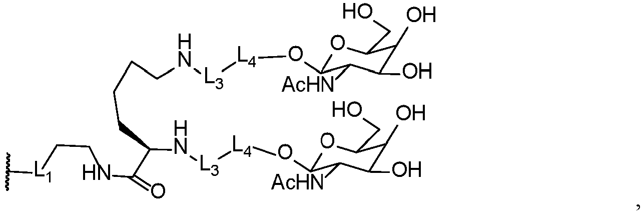 or 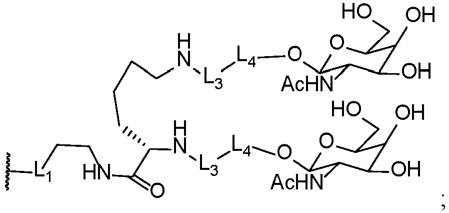
where Li is a bond, -CH₂-, -CH₂CH₂-, -C(O)-, -CH₂O-, -CH₂O-CH₂CH₂O-, or - NHC(O)-(CH₂NHC(O))ₐ-;
L₂ is a bond or -CH₂CH₂C(O)-;
L₃ is a bond, -(NHCH₂CH₂)_{b}-, -(NHCH₂CH₂CH₂)_{b}-, or -C(O)CH₂-;
L₄ is -(OCH₂CH₂)_{c}-, -(OCH₂CH₂CH₂)_{c}-, -(OCH₂CH₂CH₂CH₂)_{c}-, - (OCH₂CH₂CH₂CH₂CH₂)_{c}-, or -NHC(O)-(CH₂)_{d}-;
where a is 0, 1, 2, or 3;
b is 1, 2, 3, 4, or 5;
c is 1, 2, 3, 4, or 5;
d is 1, 2, 3, 4, 5, 6, 7, or 8;
L is a bond, -CH₂O-, or -NHC(O)-;
L' is a bond, -C(O)NH-, -NHC(O)-, or -O(CH₂CH₂O)ₑ-;
where e is 1, 2, 3, 4, or 5;
T is a bond, -CH₂-, -C(O)-, -M-, -CH₂-M-, or -C(O)-M-;
where M is 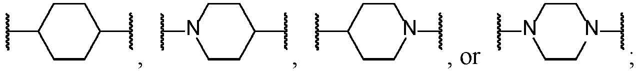
R₁ and R₂ together form -CH₂CH₂O- or -CH₂CH(R)-O-, and R₃ is H;
or the R₁ and the R₃ together form -C₁₋₂ alkylene-, and the R₂ is H;
where R is -OR', -CH₂OR', or -CH₂CH₂OR', where R' is H, a hydroxyl protective group, or a solid support, and the hydroxyl protective group is preferably - C(O)CH₂CH₂C(O)OH or 4,4'-dimethoxytriphenylmethyl;
m is 0, 1, 2, 3, 4, 5, 6, 7, 8, 9, or 10; and
n is 0, 1, 2, 3, 4, 5, 6, 7, 8, 9, or 10.

In some embodiments, the conjugated ligand targets an asialoglycoprotein receptor (ASGPR).

In some preferred embodiments, the conjugated group is selected from Table 1:

**Table 1. Structures of the conjugated group**

| Nu mbe r | Structure |
|---|---|
| 1 | 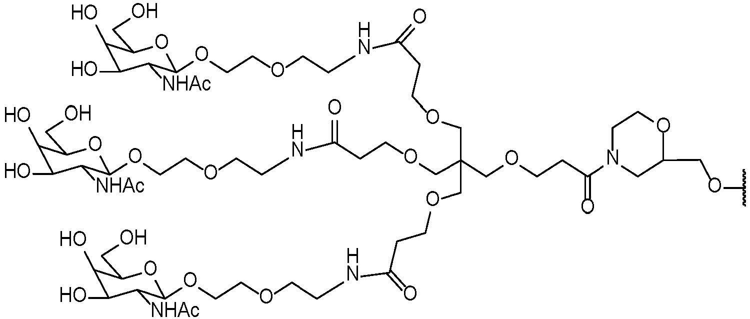 |
| 2 | 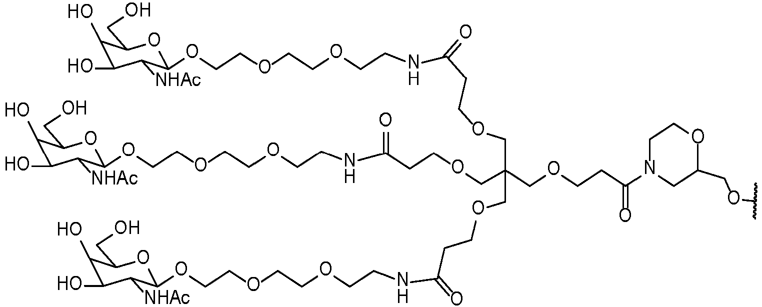 |
| 3 | 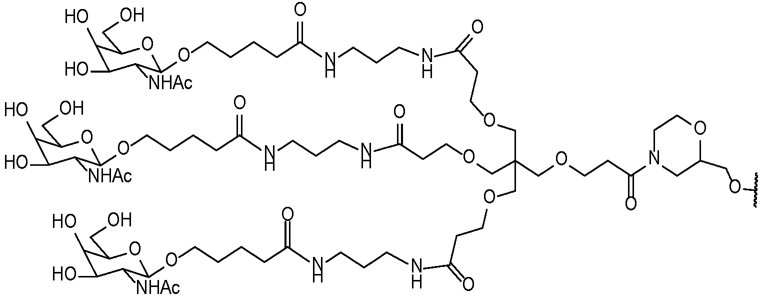 |
| 4 | 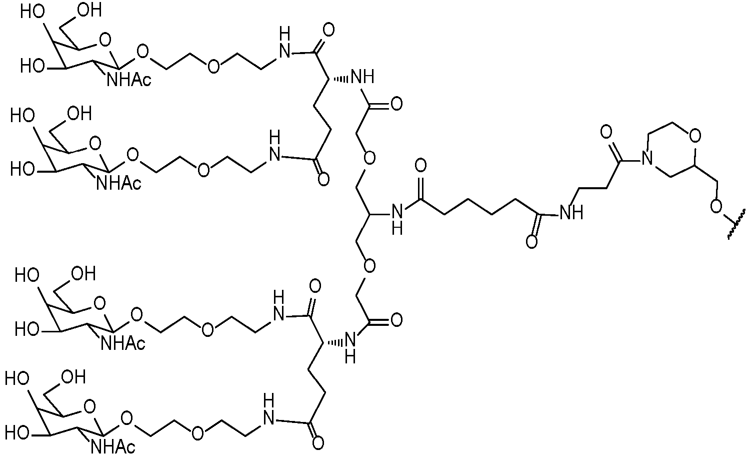 |
| 5 | 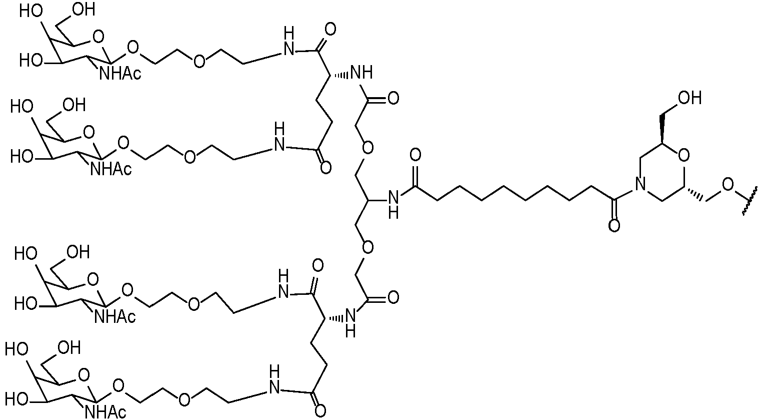 |
| 6 | 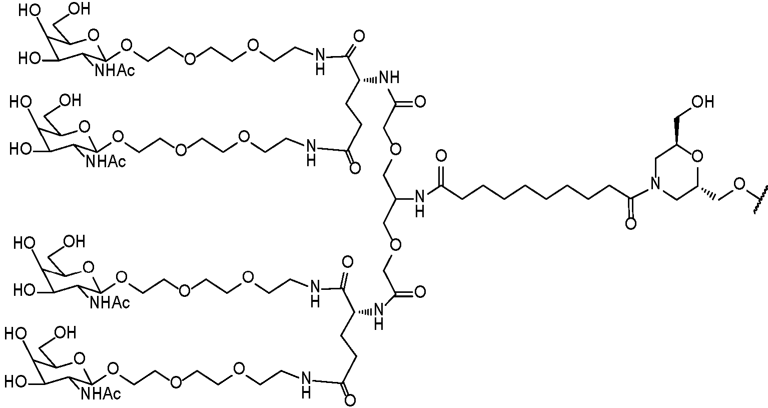 |
| 7 | 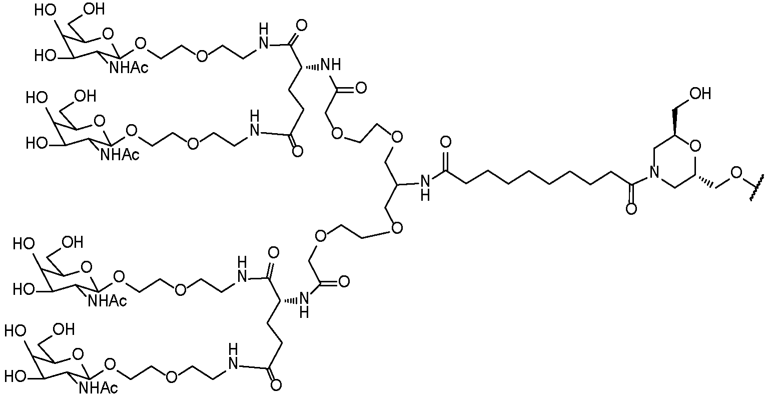 |
| 8 | 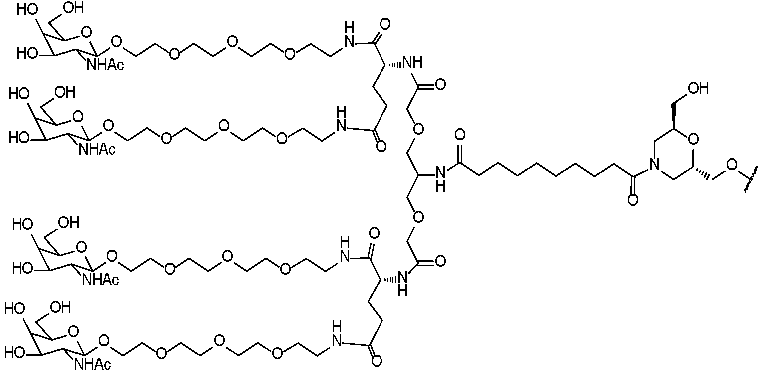 |
| 9 | 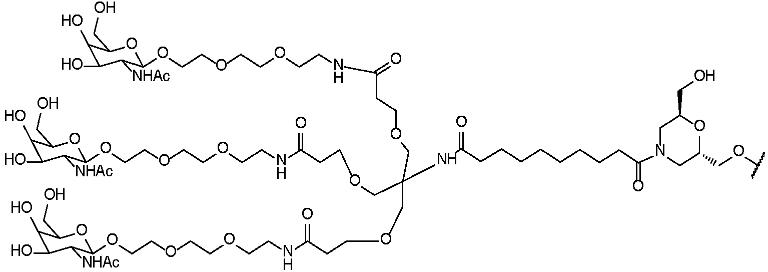 |
| 10 | 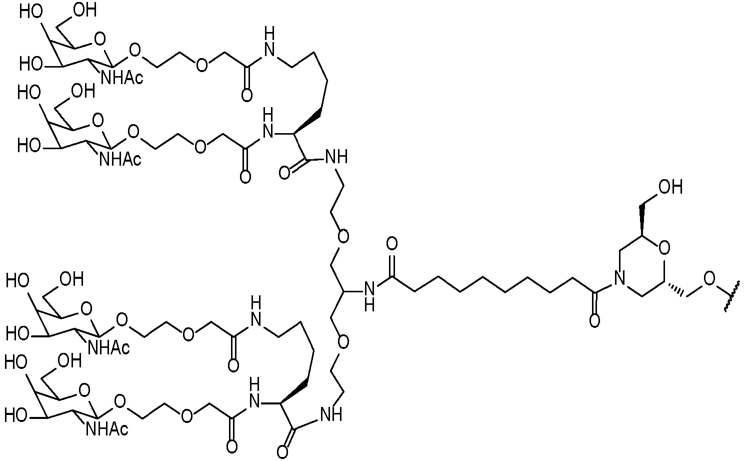 |
| 11 | 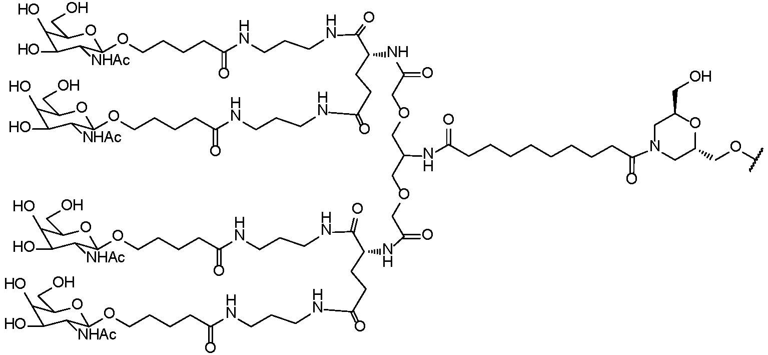 |
| 12 | 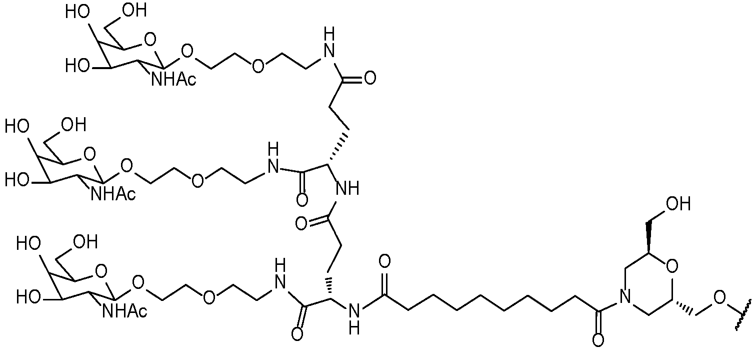 |
| 13 | 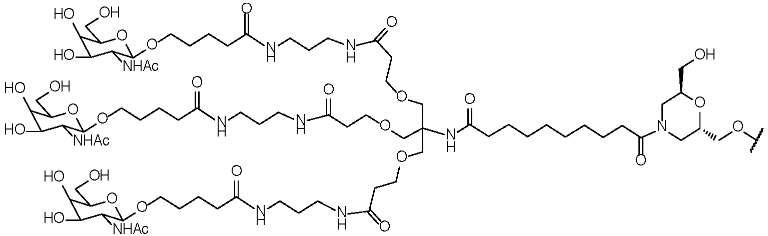 |
| 14 | 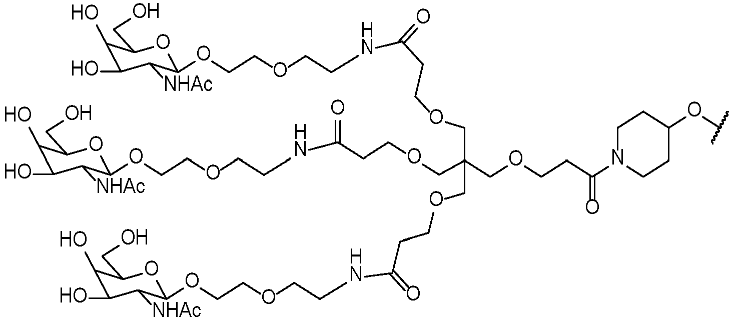 |
| 15 | 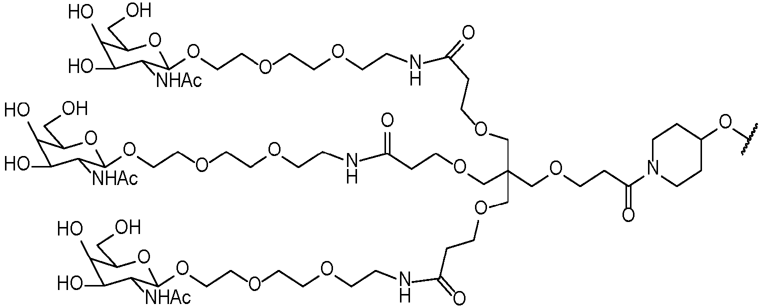 |
| 16 | 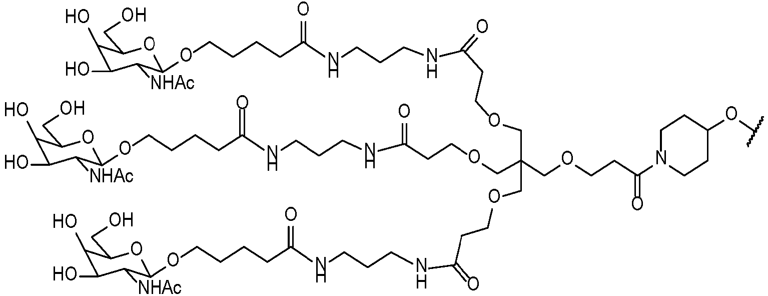 ∘ |

In some preferred embodiments, the conjugated group is selected from Table 2:

**Table 2. Structures of the conjugated group**

| Nu mbe r | Structure |
|---|---|
| 17 | 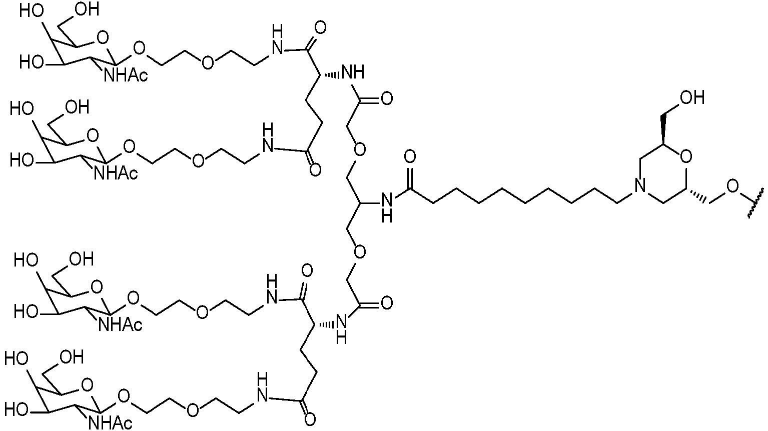 |
| 18 | 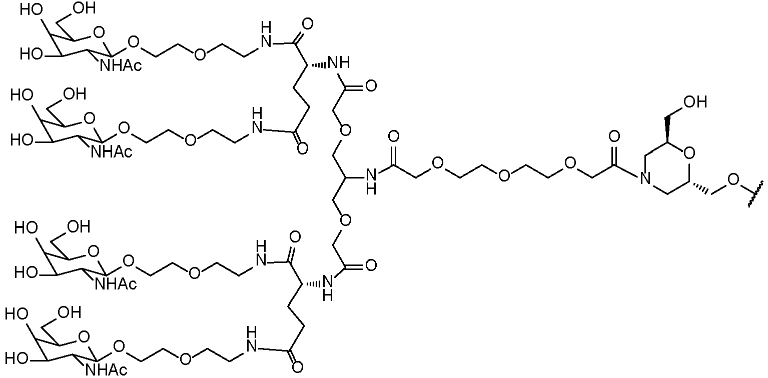 |
| 19 | 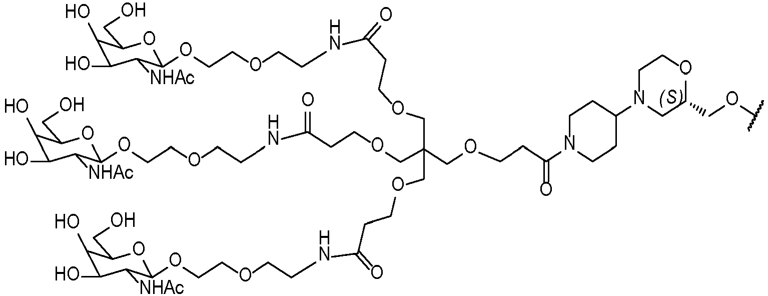 |
| 20 | 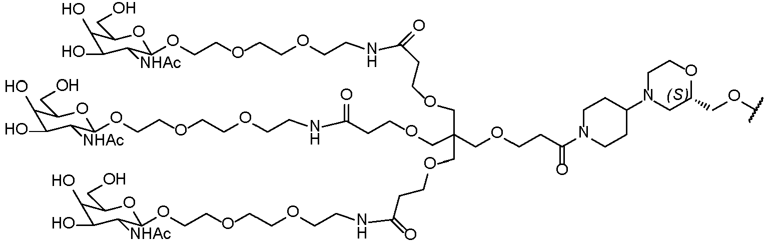 |
| 21 | 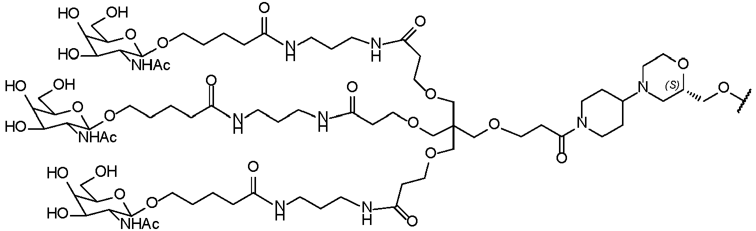 |
| 22 | 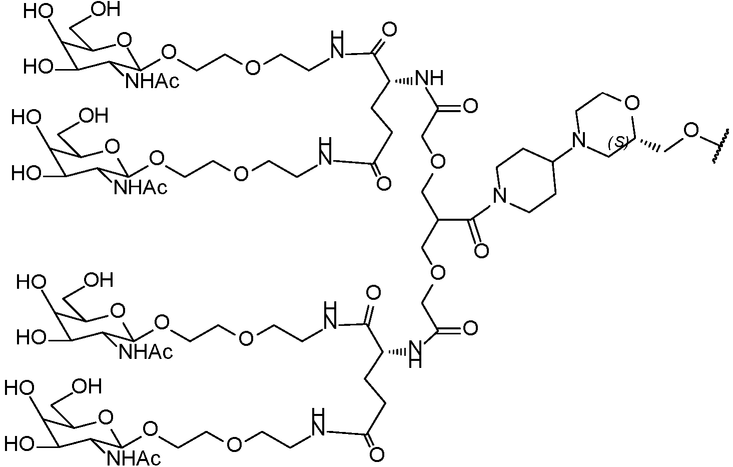 |
| 23-1 | 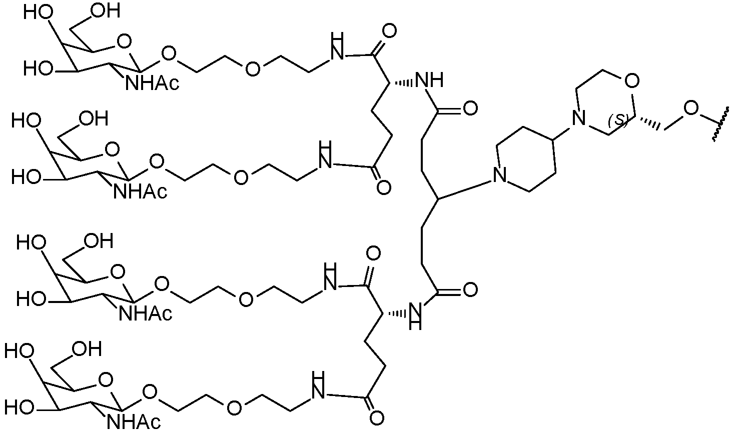 |
| 23-2 | 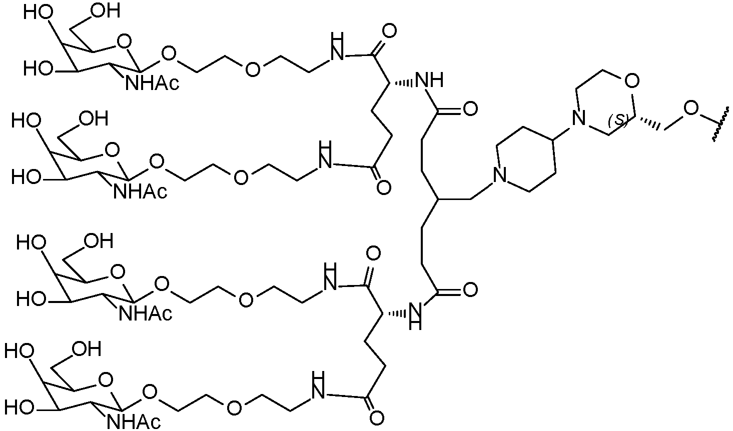 |
| 24 | 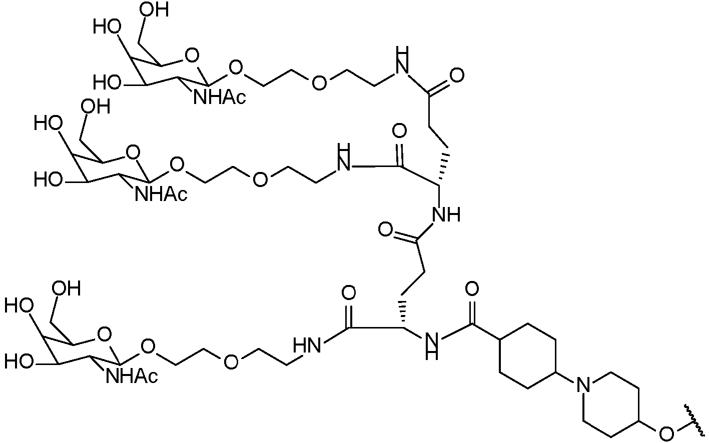 |
| 25 | 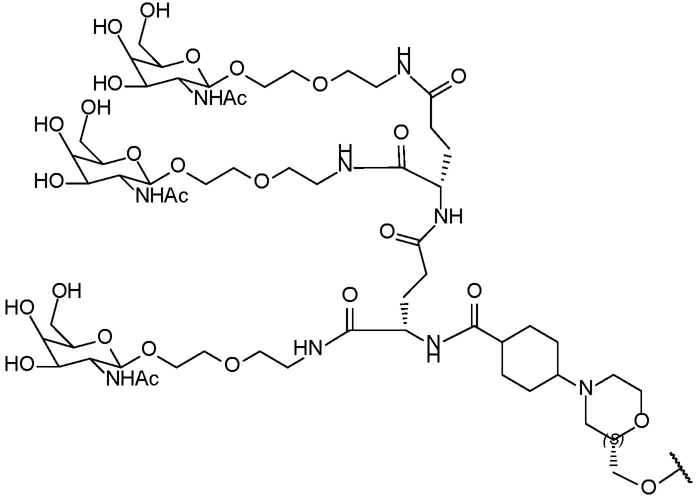 |
| 26 | 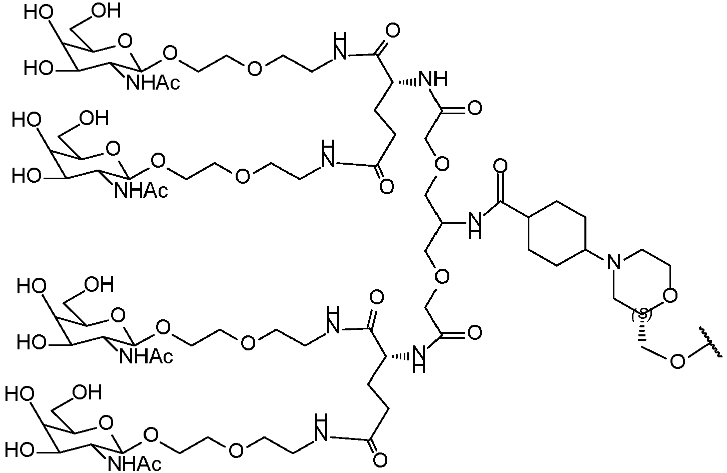 |
| 27 | 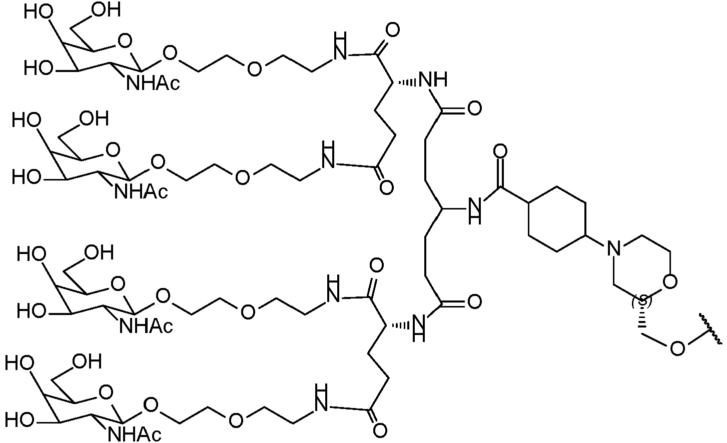 |
| 28 | 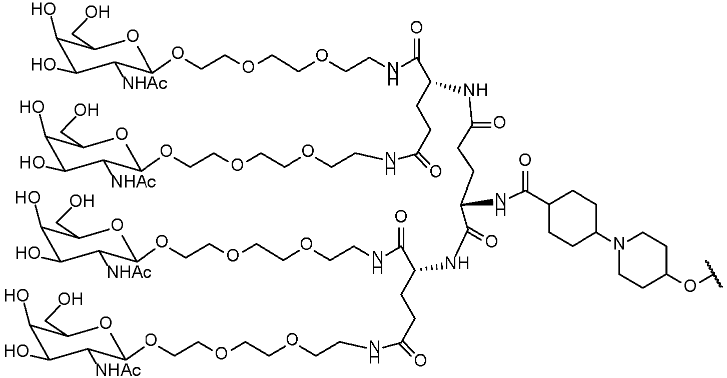 |
| 29 | 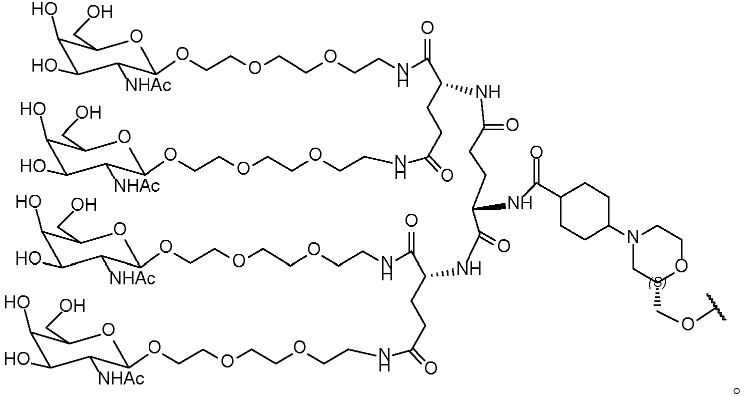 |

In some embodiments, the ligand contained in the siRNA has the following structure: 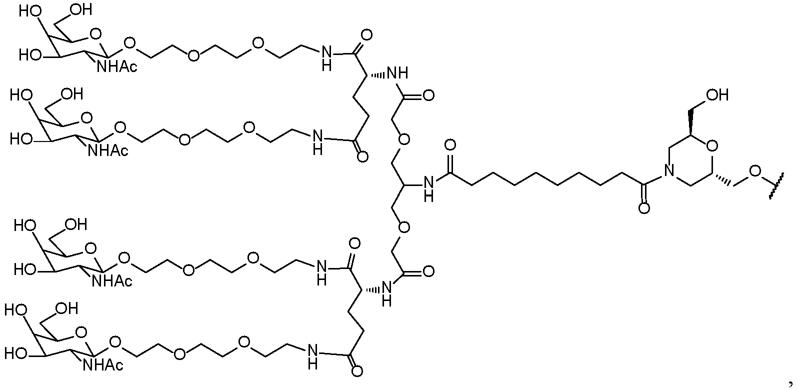 where 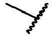 represents a position connected with the sense strand of the siRNA through the phosphate group or the thiophosphate group.

In some embodiments, the ligand contained in the siRNA has the following structure: 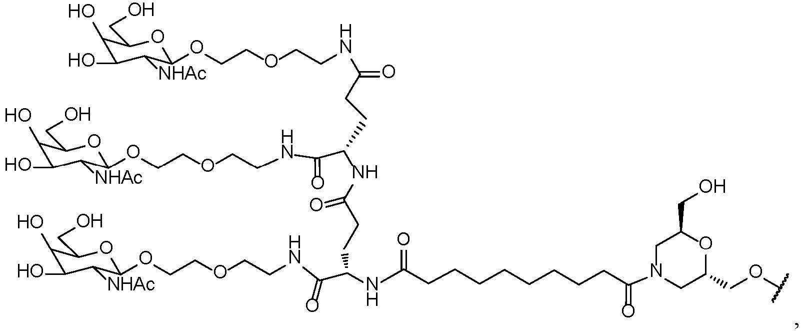 where 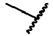 represents a position connected with the sense strand of the siRNA through the phosphate group or the thiophosphate group.

In some embodiments, the ligand contained in the siRNA of the present invention has the following structure: 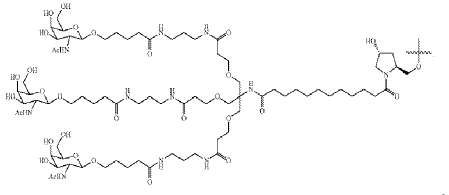 where 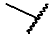 , represents a position connected with the siRNA through the phosphate group or the thiophosphate group.

In a second aspect, the present invention provides a vector, which includes a nucleotide sequence encoding the siRNA of the present invention.

In a third aspect, the present invention provides a cell, which includes the siRNA or the vector of the present invention.

In a fourth aspect, the present invention provides a pharmaceutical composition, which includes the siRNA, the vector or the cell of the present invention, and an optional pharmaceutically acceptable carrier or excipient.

In a fifth aspect, the present invention provides a kit, which includes the siRNA, the vector or the cell of the present invention.

In a sixth aspect, the present invention provides a method for treating diseases or symptoms associated with expression of PCSK9 in a subject, and the method includes a step of administering the siRNA, the vector, the cell or the pharmaceutical composition of the present invention to the subject.

In some embodiments, the diseases associated with expression of PCSK9 are cardiovascular diseases. In some preferred embodiments, the cardiovascular diseases are selected from hyperlipidemia, hypercholesterolemia, nonfamilial hypercholesterolemia, polygenic hypercholesterolemia, familial hypercholesterolemia, homozygous familial hypercholesterolemia, heterozygous familial hypercholesterolemia, coronary heart disease, myocardial infarction, stroke, or atherosclerosis.

In some embodiments, the diseases associated with expression of PCSK9 are neoplastic diseases. In some preferred embodiments, the neoplastic diseases are selected from melanoma, hepatocellular carcinoma, or metastatic hepatic cancer.

In some embodiments, the diseases associated with expression of PCSK9 are T cell-mediated inflammatory immune diseases. In some preferred embodiments, the T cell-mediated inflammatory immune diseases are selected from psoriasis, psoriatic arthritis, eczema or atopic dermatitis, urticaria, hormone-dependent dermatitis, rheumatoid arthritis, scleroderma or diabetes mellitus, chronic liver diseases, or lymphoma.

In some embodiments, the method for treating diseases or symptoms associated with expression of PCSK9 in a subject in the present invention includes administering the siRNA, the vector, the cell or the pharmaceutical composition to the subject, including subcutaneous administration, topical administration, or intravenous administration. In some embodiments, the subject is a human patient.

### DETAILED DESCRIPTION OF THE INVENTION

The embodiments of the present invention are described below through particular specific examples, and other advantages and effects of the present invention can be easily understood by persons skilled in the art based on the contents disclosed in the specification. The present invention may also be implemented or applied in other different specific embodiments, and various modifications or changes of various details in the specification may also be made without departing from the spirit of the present invention based on different viewpoints and applications.

It should be understood that the scope of protection of the present invention is not limited to the following specific embodiments. It should also be understood that terms used in the examples of the present invention are intended to describe specific embodiments, rather than to limit the scope of protection of the present invention.

In the specification and claims of the present invention, unless otherwise explicitly stated herein, the singular forms "a", "one" and "the" include plural forms.

When numerical ranges are given in the examples, it should be understood that, unless otherwise stated in the present invention, two endpoints of each numerical range and any value between the two endpoints may be selected. Unless otherwise defined, meanings of all technical terms and scientific terms used in the present invention are the same as those usually understood by persons skilled in the technical field. In addition to specific methods, apparatuses and materials used in the examples, any methods, apparatuses and materials in the prior art similar to or equivalent to the methods, the apparatuses and the materials in the examples of the present invention may also be used to realize the present invention according to understanding of the prior art by persons skilled in the technical field and documentation of the present invention, which shall fall within the scope of protection of the present invention. The embodiments of the present invention are described in more specific detail below.

### Definition

The term "siRNA" herein is a double-stranded RNA molecule that can mediate silencing of a complementary target RNA (such as an mRNA, such as a transcript of a gene that encodes a protein). The siRNA is usually double-stranded and includes an antisense strand complementary to the target RNA and a sense strand complementary to the antisense strand. For convenience, such mRNA is also called an mRNA to be silenced herein. Such gene is also called a target gene. Usually, an RNA to be silenced is an endogenous gene or a pathogen gene. In addition, an RNA except for the mRNA (such as a tRNA) and a viral RNA can also be targeted.

As used herein, the term "antisense strand" refers to such a strand of the siRNA that includes a region that is completely or substantially complementary to a target sequence.

As used herein, the term "complementary region" refers to a region, on the antisense strand, that is completely or substantially complementary to a target mRNA sequence. In the case that the complementary region is not completely complementary to the target sequence, mispairing can exist in an internal or terminal region of a molecule. Usually, mispairing with highest tolerance exists in the terminal region, for example, within 5, 4, 3, 2, or 1 nucleotide at a 5' and/or 3' end. A part, most sensitive to mispairing, of the antisense strand is called a "seed region". For example, in an siRNA including a 19nt strand, some mispairings can be tolerated at position 19 (counting from 5' to 3').

As used herein, the term "complementary" refers to the ability of a first polynucleotide to hybridize with a second polynucleotide under certain conditions such as strict conditions. For example, the strict conditions may include 400 mM NaCl, 40 mM PIPES with a pH value of 6.4, and 1 mM EDTA that are lasted at 50°C or 70°C for 12-16 hours.

As used herein, in order to meet the above requirements with respect to the hybridize ability, a "complementary" sequence may further include or completely form base pairs that are formed from non-Watson-Crick base pairs and/or from unnatural and modified nucleotides. Such non-Watson-Crick base pairs include, but are not limited to, G:U Wobble base pairs or Hoogstein base pairs.

As used herein, a polynucleotide that is "at least partially complementary" or "substantially complementary" to a messenger RNA (mRNA) refers to a polynucleotide that is substantially complementary to a continuous part of an mRNA of interest (such as, an mRNA encoding PCSK9). For example, when a sequence is substantially complementary to a non-interrupted part of an mRNA encoding PCSK9, a polynucleotide is at least partially complementary to the PCSK9 mRNA.

Herein, the terms "complementary", "completely complementary" and "substantially complementary" may be used relative to base pairs between the sense strand and the antisense strand of the siRNA, or between an antisense strand of an siRNA reagent and a target sequence.

As used herein, the term "sense strand" refers to such a strand of the siRNA that includes a region that is substantially complementary to the region of the term antisense strand defined herein.

A "nucleoside" is a compound composed of two components including a purine base or a pyrimidine base, and ribose or deoxyribose. A "nucleotide" is a compound composed of three components including a purine base or a pyrimidine base, ribose or deoxyribose, and phosphoric acid. An "oligonucleotide" refers to, for example, a nucleic acid molecule (RNA or DNA) that has a length of less than 100, 200, 300, or 400 nucleotides.

The "base" is a basic composition unit for synthesis of nucleosides, nucleotides, and nucleic acids, and due to nitrogen in composition elements, is also called "nitrogenous base". Herein, unless otherwise specified, capital letters A, U, T, G and C represent base composition of nucleotides, which are adenine, uracil, thymine, guanine, and cytosine, respectively.

As used herein, the term "nucleotide overhang" refers to at least one unpaired nucleotide that protrudes from a double-stranded structure of an siRNA (such as, an siRNA). For example, when a 3'-end of one strand of the siRNA extends a 5'-end of another strand or vice versa, a nucleotide overhang exists. The siRNA may include a overhang having at least one nucleotide; and alternatively, the overhang may include at least two nucleotides, at least three nucleotides, at least four nucleotides, at least five nucleotides, or more. The nucleotide overhang may include or be composed of nucleotides/nucleoside analogs (including deoxynucleotides/nucleosides). One or more overhangs may be located at the sense strand, the antisense strand, or any combination thereof. In addition, one or more nucleotides at the overhang may exist at the 5'-end, the 3'-end, or the two ends of the antisense strand or the sense strand of the siRNA.

A "flat end" or "flat terminal end" means that no unpaired nucleotides exist at the end of the double-stranded siRNA, namely, no nucleotide overhang. A "flat end" siRNA refers to an siRNA that is double-stranded throughout a length, namely, no nucleotide overhang at any end of a molecule. The siRNA of the present invention includes an siRNA having a nucleotide overhang at one end (namely, a reagent having one overhang and one flat end) or having nucleotide overhangs at two ends.

Substantially all nucleotides of the siRNA of the present invention are modified. For example, substantially all nucleotides of the sense strand are modified nucleotides, and/or substantially all nucleotides of the antisense strand are modified nucleotides, and/or substantially all the nucleotides of the sense strand and the antisense strand are modified nucleotides. In other embodiments of the present invention, all the nucleotides of the siRNA of the present invention are modified nucleotides. For example, all the nucleotides of the sense strand are modified nucleotides, and/or all the nucleotides of the antisense strand are modified nucleotides, and/or all the nucleotides of the sense strand and the antisense strand are modified nucleotides. The "substantially all nucleotides are modified" means that the siRNA of the present invention is mostly but not completely modified and may include no more than 5, 4, 3, 2, or 1 unmodified nucleotide.

Herein, the "modified nucleotides" include, but are not limited to, 2'-O-methyl modified nucleotides, 2'-fluoro modified nucleotides, 2'-deoxy- modified nucleotides, inosine ribonucleotides, abasic nucleotides, reverse abasic deoxyribonucleotides, nucleotides containing a thiophosphate group, vinyl phosphate modified nucleotides, locked nucleotides, 2'-amino- modified nucleotides, 2'-alkyl- modified nucleotides, morpholino nucleotides, aminophosphates, unnatural bases containing nucleotides, and terminal nucleotides, deoxyribonucleotides or conventional protective groups linked to cholesterol-based derivatives or a sebacamide dodecanoate group, etc. For example, the 2'-fluoro modified nucleotides refer to nucleotides formed by substitution of hydroxyl at site 2' of ribosyl of nucleotides with fluorine. The 2'-deoxy- modified nucleotides refer to nucleotides formed by substitution of 2'-hydroxyl of ribosyl with methoxyl.

As used herein, the "ligand moiety" refers to a chemical portion that is conjugated with the siRNA and can change the distribution, targeting or life of the siRNA. In preferred embodiments, compared with, for example, an siRNA without the ligand, the ligand provides enhanced affinity for a selected target (such as a molecule, a cell or cell type, a compartment (such as a cell or organ compartment, a tissue, an organ, or a region of the body).

As used herein, the terms "inhibit", "reduce", "silence', "down-regulate" and other similar terms can be used interchangeably and include inhibition at any level.

The phrase "inhibit expression of PCSK9" is intended to inhibit expression of any PCSK9 gene as well as variants or mutants of the PCSK9 gene. Thus, the PCSK9 gene may be a wild type PCSK9 gene, a mutant PCSK9 gene, or a transgenic PCSK9 gene in the case of genetically manipulated cells, cell population s, or organisms.

"Inhibition of PCSK9 gene expression" includes inhibition of the PCSK9 gene at any level, such as at least partial inhibition of the PCSK9 gene expression. The PCSK9 gene expression can be evaluated based on levels or changes in levels of any variables associated with the PCSK9 gene expression, such as a PCSK9 mRNA level, a PCSK9 protein level, or a lipid level. The levels can be evaluated in individual cells or in a group of cells (including, for example, a sample derived from a subject).

The inhibition can be evaluated through a decrease of an absolute or relative level of one or more variables associated with the PCSK9 expression compared with a control level. The control level may be any type of control level utilized in the field, such as a baseline level before administration or a level determined from a similarly untreated or treated subject, cell, or sample with a control (such as, an only buffer control or an inert agent control).

The "hydroxyl protective group" refers to a group that can prevent hydroxyl from a chemical reaction and can also be removed under certain conditions to restore the hydroxyl. The hydroxyl protective group mainly includes a silane protective group, an acyl protective group, or am ether protective group, preferably including the following: trimethylsilyl (TMS), triethylsilyl (TES), dimethylisopropylsilyl (DMIPS), diethylisopropylsilyl (DEIPS), tert-butyldimethylsilyl (TBDMS), tert-butyldiphenylsilyl (TBDPS), triisopropylsilyl (TIPS), acetyl (Ac), chloroacetyl, dichloroacetyl, trichloroacetyl, trifluoroacetyl (TFA), benzoyl, p-methoxybenzoyl, 9-fluorenylmethoxycarbonyl (Fmoc), allyloxycarbonyl (Alloc), 2,2,2-trichloroethoxycarbonyl (Troc), carbobenzoxy (Cbz), tert-butoxycarbonyl (Boc), phenylmethyl (Bn), p-methoxybenzyl (PMB), allyl, triphenylmethyl (Tr), di-p-methoxytriphenylmethyl (DMTr), methoxymethyl (MOM), phenoxymethyl (BOM), 2,2,2-trichloroethoxymethyl, 2-methoxyethoxymethyl (MEM), methylthiomethyl (MTM), or p-methoxybenzyloxymethyl (PMBM).

The term "halo" or "halogen" refers to fluorine (F), chlorine (Cl), bromine (Br), and iodine (I).

"C₁₋₆ haloalkyl" refers to the "C₁₋₆ alkyl" that is substituted with one or more halogen groups. In some embodiments, C₁₋₄ haloalkyl is particularly preferred, and C₁₋₂ haloalkyl is more preferred. Exemplary haloalkyl includes, but is not limited to: -CF₃, -CH₂F, -CHF₂, -CHFCH₂F, -CH₂CHF₂, -CF₂CF₃, -CCl₃, -CH₂Cl, -CHCl₂, 2,2,2-trifluoro-1,1-dimethyl-ethyl, etc. A haloalkyl group can be substituted at any available connection point with, for example, 1 to 5 substituents, 1 to 3 substituents, or 1 substituent.

"C₁₋₆ alkylene" refers to a divalent group formed by removing another hydrogen of C₁₋₆ alkyl, and can be substituted or unsubstituted. In some embodiments, C₁₋₄ alkylene, C₂₋₄ alkylene, and C₁₋₂ alkylene are preferred. Unsubstituted alkylene includes, but is not limited to: methylene (-CH₂-), ethylidene (-CH₂CH₂-), propylidene (-CH₂CH₂CH₂-), butylidene (-CH₂CH₂CH₂CH₂-), pentylidene (-CH₂CH₂CH₂CH₂CH₂-), hexylidene (-CH₂CH₂CH₂CH₂CH₂CH₂-), etc. Exemplary substituted alkylene, such as, alkylene substituted with one or more alkyls (methyl), includes, but is not limited to: substituted methylene (-CH(CH₃)-, -C(CH₃)₂-), substituted ethylidene (-CH(CH₃)CH₂-, - CH₂CH(CH₃)-, -C(CH₃)₂CH₂-, -CH₂C(CH₃)₂-), substituted propylidene (-CH(CH₃)CH₂CH₂-, -CH₂CH(CH₃)CH₂-, -CH₂CH₂CH(CH₃)-, -C(CH₃)₂CH₂CH₂-, - CH₂C(CH₃)₂CH₂-, -CH₂CH₂C(CH₃)₂-), etc.

As used herein, the term "vector" refers to such a nucleic acid molecule that can amplify or express another nucleic acid connected thereto.

### I. siRNA

The present invention provides a small interfering RNA (siRNA) for inhibiting expression of proprotein convertase subtilisin/kexin type 9 (PCSK9) in a cell. The siRNA includes a sense strand and an antisense strand that form a double-stranded region, where lengths of the sense strand and the antisense strand are each independently 15-30 nucleotides, and the antisense strand includes a nucleotide sequence of at least 15 consecutive nucleotides of a nucleotide sequence shown in any one of SEQ ID NO: 105-208.

In some embodiments, the double-stranded region formed by the sense strand and the antisense strand is completely complementary. In other embodiments, the double-stranded region formed by the sense strand and the antisense strand is substantially complementary, which may include 1, 2, 3, 4, or 5 non-complementary sites.

In some specific embodiments, the sense strand includes a nucleotide sequence of at least 15 consecutive nucleotides of a nucleotide sequence shown in any one of SEQ ID NO: 1-104.

In some embodiments, the lengths of the sense strand and the antisense strand are each independently 17-27 nucleotides, preferably 19-25 nucleotides, more preferably 19-23 nucleotides.

In some embodiments, a length of the double-stranded region is 15-25 base pairs, preferably 17-21 base pairs, more preferably 19-21 base pairs.

One or two of the sense strand and the antisense strand include a 3' overhang and/or a 5' overhang having at least 1 nucleotide, for example, one or two of the sense strand and the antisense strand include a 3' overhang and/or a 5' overhang having at least 1 nucleotide. In some preferred embodiments, the antisense strand includes a 3' overhang and/or a 5' overhang having at least 2 nucleotides, and preferably, the antisense strand includes a 3' overhang and/or a 5' overhang having 2 nucleotides. In some embodiments, the lengths of the sense strand and the antisense strand are the same. In some embodiments, the full length of the sense strand and the full length of the antisense strand are complementary to form a double strand, namely, having a flat terminal end. In other embodiments, the lengths of the sense strand and the antisense strand are the same, and a part of the sense strand is complementary to a part of the antisense strand, namely, both the sense strand and the antisense strand have a 5' overhang. In some embodiments, the lengths of the sense strand and the antisense strand are different. In preferred embodiments, a 5' end of the antisense strand is a overhang having at least 1 nucleotide, more preferably a overhang having 2 or 3 nucleotides.

In some embodiments, the antisense strand includes a nucleotide sequence of at least 16 consecutive nucleotides, a nucleotide sequence of at least 17 consecutive nucleotides, a nucleotide sequence of at least 18 consecutive nucleotides, a nucleotide sequence of at least 19 consecutive nucleotides, or a nucleotide sequence of at least 20 consecutive nucleotides of the nucleotide sequence shown in any one of SEQ ID NO: 105-208, and preferably, the antisense strand includes the nucleotide sequence shown in any one of SEQ ID NO: 105-208.

In some embodiments, the sense strand includes a nucleotide sequence of at least 16 consecutive nucleotides, a nucleotide sequence of at least 17 consecutive nucleotides, a nucleotide sequence of at least 18 consecutive nucleotides, a nucleotide sequence of at least 19 consecutive nucleotides, or a nucleotide sequence of at least 20 consecutive nucleotides of the nucleotide sequence shown in any one of SEQ ID NO: 1-104, and preferably, the sense strand includes the nucleotide sequence shown in any one of SEQ ID NO: 1-104.

In some embodiments, the siRNA includes a sense strand sequence and an antisense strand sequence that are paired as shown in Table 3.

In some embodiments, the antisense strand includes a nucleotide sequence of at least 15 consecutive nucleotides, a nucleotide sequence of at least 16 consecutive nucleotides, a nucleotide sequence of at least 17 consecutive nucleotides, a nucleotide sequence of at least 18 consecutive nucleotides, a nucleotide sequence of at least 19 consecutive nucleotides, or a nucleotide sequence of at least 20 consecutive nucleotides of a nucleotide sequence shown in any one of SEQ ID NO: 195/203/204, 142/207, 143/197/205/206, 144, 147, 148, 149, and 150, and preferably, the antisense strand includes the nucleotide sequence shown in any one of SEQ ID NO: 195/203/204, 142/207, 143/197/205/206, 144, 147, 148, 149, and 150.

In some embodiments, the sense strand includes a nucleotide sequence of at least 15 consecutive nucleotides, a nucleotide sequence of at least 16 consecutive nucleotides, a nucleotide sequence of at least 17 consecutive nucleotides, a nucleotide sequence of at least 18 consecutive nucleotides, a nucleotide sequence of at least 19 consecutive nucleotides, or a nucleotide sequence of at least 20 consecutive nucleotides of a nucleotide sequence shown in any one of SEQ ID NO: 91/99/100, 38/103, 39/93/101/102, 40, 43, 44, 45, and 46, and preferably, the sense strand includes the nucleotide sequence shown in any one of SEQ ID NO: 91/99/100, 38/92, 39/93/101/102, 40, 43, 44, 45, and 46.

In some embodiments, (a) the antisense strand includes a nucleotide sequence shown in SEQ ID NO: 195, and the sense strand includes a nucleotide sequence shown in SEQ ID NO: 91;
(b) the antisense strand includes a nucleotide sequence shown in SEQ ID NO: 203, and the sense strand includes a nucleotide sequence shown in SEQ ID NO: 99;
(c) the antisense strand includes a nucleotide sequence shown in SEQ ID NO: 204, and the sense strand includes a nucleotide sequence shown in SEQ ID NO: 100;
(d) the antisense strand includes a nucleotide sequence shown in SEQ ID NO: 142, and the sense strand includes a nucleotide sequence shown in SEQ ID NO: 38;
(e) the antisense strand includes a nucleotide sequence shown in SEQ ID NO: 207, and the sense strand includes a nucleotide sequence shown in SEQ ID NO: 103;
(f) the antisense strand includes a nucleotide sequence shown in SEQ ID NO: 143, and the sense strand includes a nucleotide sequence shown in SEQ ID NO: 39;
(g) the antisense strand includes a nucleotide sequence shown in SEQ ID NO: 197, and the sense strand includes a nucleotide sequence shown in SEQ ID NO: 93;
(h) the antisense strand includes a nucleotide sequence shown in SEQ ID NO: 205, and the sense strand includes a nucleotide sequence shown in SEQ ID NO: 101;
(i) the antisense strand includes a nucleotide sequence shown in SEQ ID NO: 206, and the sense strand includes a nucleotide sequence shown in SEQ ID NO: 102;
(j) the antisense strand includes a nucleotide sequence shown in SEQ ID NO: 144, and the sense strand includes a nucleotide sequence shown in SEQ ID NO: 40; or
(k) the antisense strand includes a nucleotide sequence shown in SEQ ID NO: 147, and the sense strand includes a nucleotide sequence shown in SEQ ID NO: 43;
(l) the antisense strand includes a nucleotide sequence shown in SEQ ID NO: 148, and the sense strand includes a nucleotide sequence shown in SEQ ID NO: 44;
(m) the antisense strand includes a nucleotide sequence shown in SEQ ID NO: 149, and the sense strand includes a nucleotide sequence shown in SEQ ID NO: 45; or
(n) the antisense strand includes a nucleotide sequence shown in SEQ ID NO: 150, and the sense strand includes a nucleotide sequence shown in SEQ ID NO: 46.

### II. Modifications of nucleotides

In some embodiments, substantially all nucleotides of the sense strand and substantially all nucleotides of the antisense strand are modified nucleotides. In some embodiments, at least 80% of the nucleotides of the sense strand are modified nucleotides, and/or at least 80% of the nucleotides of the antisense strand are modified nucleotides.

In some embodiments, all the nucleotides of the sense strand and/or all the nucleotides of the antisense strand are modified nucleotides.

Modifications of the nucleotides in the present invention may be modifications on phosphate groups, ribose groups and/or base groups of nucleotides.

In some specific embodiments, the sense strand and the antisense strand each independently include one or more nucleotide modifications selected from the following group: 2'-O-methyl modified nucleotides, 2'-fluoro modified nucleotides, 2'-deoxy- modified nucleotides, inosine ribonucleotides, abasic nucleotides, reverse abasic deoxyribonucleotides, nucleotides containing a thiophosphate group, vinyl phosphate modified nucleotides, locked nucleotides, 2'-amino- modified nucleotides, 2'-alkyl- modified nucleotides, morpholino nucleotides, aminophosphates, unnatural bases containing nucleotides, and terminal nucleotides or deoxyribonucleotides linked to cholesterol-based derivatives or a sebacamide dodecanoate group.

In some preferred embodiments, the sense strand and the antisense strand each independently include one or more nucleotide modifications selected from the following group: 2'-O-methyl modified nucleotides, 2'-fluoro modified nucleotides, deoxyribonucleotides, or nucleotides containing a thiophosphate group. In some preferred embodiments, the sense strand and/or the antisense strand includes at least 2 2'-fluoro modified nucleotides. In some preferred embodiments, the sense strand and/or the antisense strand includes at least 8 2'-O-methyl modified nucleotides. In some preferred embodiments, a 3' end and/or a 5' end of the sense strand and/or the antisense strand includes 1-5 thiophosphate groups, preferably 2-3 thiophosphate groups. In some preferred embodiments, the sense strand and/or the antisense strand includes an adenine deoxyribonucleotide, a thymine deoxyribonucleotide, a guanine deoxyribonucleotide, and/or a cytosine deoxyribonucleotide. In more preferred embodiments, the sense strand and/or the antisense strand includes a thymine deoxyribonucleotide. In most preferred embodiments, the sense strand includes a thymine deoxyribonucleotide.

In some preferred embodiments, the antisense strand includes a modified nucleotide sequence shown in Table 5 of the specification, and/or the sense strand includes a modified nucleotide sequence shown in Table 4 of the specification. In some preferred embodiments, the siRNA includes a modified sense strand sequence and a modified antisense strand sequence that are paired as shown in Table 6 of the specification.

In some specific embodiments, (a) the sense strand includes CmsUmsAmGmAmCmCfUmGfUmdTUmUmGmCmUmUmUmUmGmUm, and the antisense strand includes AmsCfsAmAfAfAfGmCfAmAfAmAfCmAfGmGfUmCfUmAmGmsAmsAm;
(b) the sense strand includes CmsUmsAmGmAmCmCfUmGfUmdTUmUmGmCmUmUmUmUmGmsUm, and the antisense strand includes AmsCfsAmAfAfAfGmCfAmAfAmAfCmAfGmGfUmCfUmAmGmsAmsAm;
(c) the sense strand includes UmsGmsUmUmUmUmGfCfUfUmUmUmGmUmAmAmCmsUmsUm, and the antisense strand includes AmsAfsGmUfUmAfCmAfAmAfAmGfCmAfAmAfAmCfAmsGfsGm;
(d) the sense strand includes UmsGmsUmUmUmUmGfCfUfUmUmUmGmUmAmAmCmUmsUm, and the antisense strand includes AmsAfsGmUfUmAfCmAfAmAfAmGfCmAfAmAfAmCfAmsGfsGm;
(e) the sense strand includes GmsUmsUmUmUmGmCfUfUfUmUmGmUmAmAmCmUmsUmsAm, and the antisense strand includes UmsAfsAmGfUmUfAmCfAmAfAmAfGmCfAmAfAmAfCmsAfsGm;
(f) the sense strand includes GmsUmsUmUmUmGmCfUfUfUmUmGmUmAmAmCmUmsUmsAm, and the antisense strand includes UmsAfsAmGfUmUfAmCfAmAfAmAfGmCfAmAfAmAfCmsAfsGm;
(g) the sense strand includes GmsUmsUmUmUmGmCfUfUfUmUmGmUmAmAmCmUmUmsAm, and the antisense strand includes UmsAfsAmGfUmUfAmCfAmAfAmAfGmCfAmAfAmAfCmsAfsGm;
(h) the sense strand includes GmsUmsUmUmUmGmCfUfUfUmUmGmUmAmAmCmUmUmAm, and the antisense strand includes UmsAfsAmGfUmUfAmCfAmAfAmAfGmCfAmAfAmAfCmsAfsGm;
(i) the sense strand includes UmsUmsUmUmGmCmUfUfUfUmGmUmAmAmCmUmUmsGmsAm, and the antisense strand includes UmsCfsAmAfGmUfUmAfCmAfAmAfAmGfCmAfAmAfAmsCfsAm;
(j) the sense strand includes UmsUmsUmUmGmCmUfUfUfUmGmUmAmAmCmUmUmsGmsAm, and the antisense strand includes UmsCfsAmAfGmUfUmAfCmAfAmAfAmGfCmAfAmAfAmsCfsAm;
(k) the sense strand includes UmsGmsCmUmUmUmUfGfUfAmAmCmUmUmGmAmAmsGmsAm, and the antisense strand includes UmsCfsUmUfCmAfAmGfUmUfAmCfAmAfAmAfGmCfAmsAfsAm;
(l) the sense strand includes GmsCmsUmUmUmUmGfUfAfAmCmUmUmGmAmAmGmsAmsUm, and the antisense strand includes AmsUfsCmUfUmCfAmAfGmUfUmAfCmAfAmAfAmGfCmsAfsAm;
(m) the sense strand includes CmsUmsUmUmUmGmUfAfAfCmUmUmGmAmAmGmAmsUmsAm, and the antisense strand includes UmsAfsUmCfUmUfCmAfAmGfUmUfAmCfAmAfAmAfGmsCfsAm; or
(n) the sense strand includes UmsUmsUmUmGmUmAfAfCfUmUmGmAmAmGmAmUmsAmsUm, and the antisense strand includes AmsUfsAmUfCmUfUmCfAmAfGmUfUmAfCmAfAmAfAmsGfsCm.

### III. Ligand

The siRNA of the present invention is further conjugated with a ligand moiety containing N-acetylgalactosamine through a phosphate group or a thiophosphate group. In preferred embodiments, the sense strand of the siRNA is conjugated with the ligand moiety through the phosphate group or the thiophosphate group. In some preferred embodiments, a 3' end of the sense strand is conjugated with the ligand moiety through the phosphate group or the thiophosphate group. In other preferred embodiments, a 5' end of the sense strand is conjugated with the ligand moiety through the phosphate group or the thiophosphate group.

In some embodiments, the ligand moiety includes a conjugated group shown in Formula (X'): 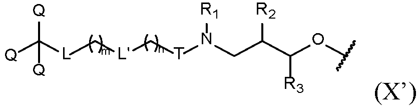 where, 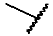 represents a position connected with a biomolecule;
Q is independently H, 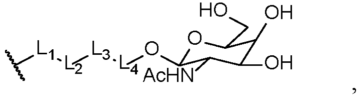 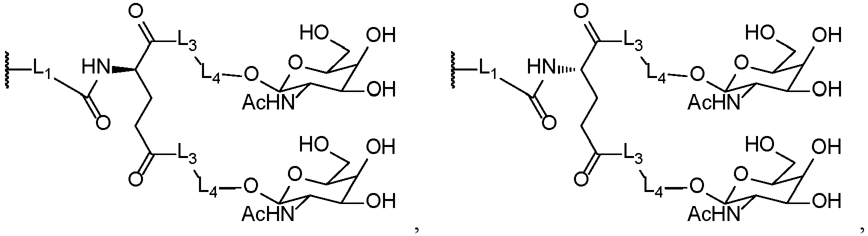 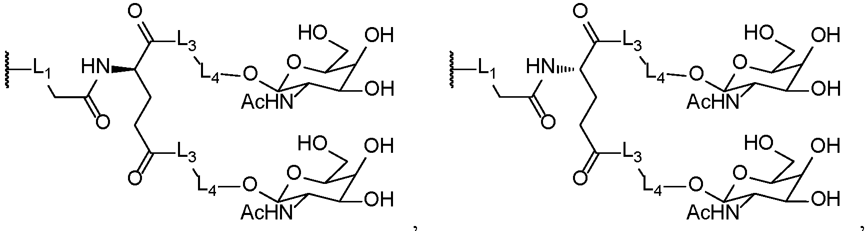 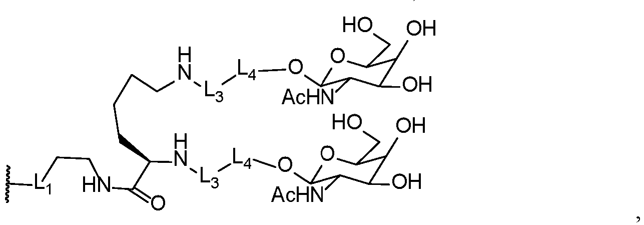 or 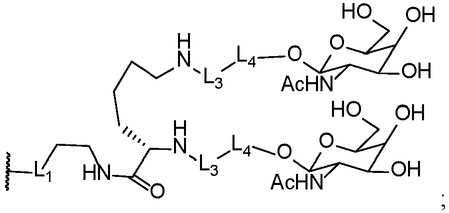
where Li is a bond, -CH₂-, -CH₂CH₂-, -C(O)-, -CH₂O-, -CH₂O-CH₂CH₂O-, or -NHC(O)-(CH₂NHC(O))ₐ-;
L₂ is a bond or -CH₂CH₂C(O)-;
L₃ is a bond, -(NHCH₂CH₂)_{b}-, -(NHCH₂CH₂CH₂)_{b}-, or -C(O)CH₂-;
L₄ is -(OCH₂CH₂)_{c}-, -(OCH₂CH₂CH₂)_{c}-, -(OCH₂CH₂CH₂CH₂)_{c}-, - (OCH₂CH₂CH₂CH₂CH₂)_{c}-, or -NHC(O)-(CH₂)_{d}-;
   where a is 0, 1, 2, or 3;
   b is 1, 2, 3, 4, or 5;
   c is 1, 2, 3, 4, or 5;
   d is 1, 2, 3, 4, 5, 6, 7, or 8;
L is a bond, -CH₂O-, or -NHC(O)-;
L' is a bond, -C(O)NH-, -NHC(O)-, or -O(CH₂CH₂O)ₑ-;
   where e is 1, 2, 3, 4, or 5;
T is a bond, -CH₂-, -C(O)-, -M-, -CH₂-M-, or -C(O)-M-;
   where M is 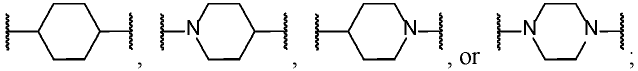
R₁ and R₂ together form -CH₂CH₂O- or -CH₂CH(R)-O-, and R₃ is H;
or the R₁ and the R₃ together form -C₁₋₂ alkylene-, and the R₂ is H;
   where R is -OR', -CH₂OR', or -CH₂CH₂OR', where R' is H, a hydroxyl protective group, or a solid support, and the hydroxyl protective group is preferably -C(O)CH₂CH₂C(O)OH or 4,4'-dimethoxytriphenylmethyl;
m is 0, 1, 2, 3, 4, 5, 6, 7, 8, 9, or 10; and
n is 0, 1, 2, 3, 4, 5, 6, 7, 8, 9, or 10.

In some embodiments, the conjugated group is shown in Formula (I'): 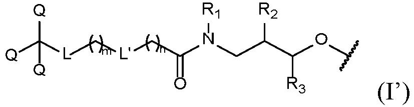 where, 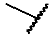 represents a position connected with a biomolecule;
Q is independently H, 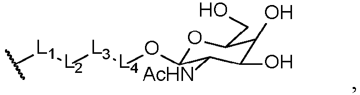 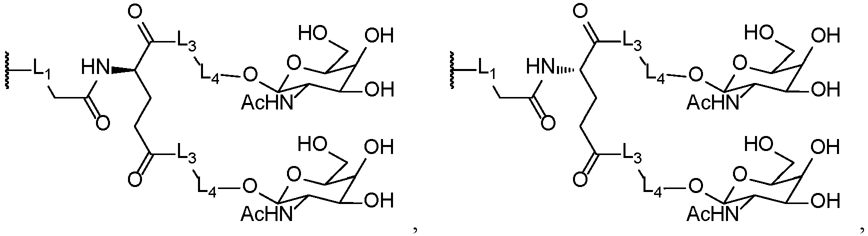 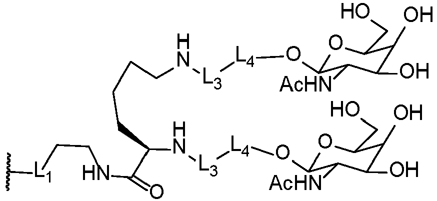 or 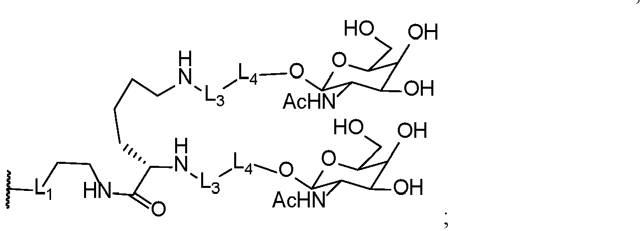
where Li is a bond, -CH₂-, -CH₂CH₂-, -C(O)-, -CH₂O-, -CH₂O-CH₂CH₂O-, or -NHC(O)-(CH₂NHC(O))ₐ-;
L₂ is a bond or -CH₂CH₂C(O)-;
L₃ is a bond, -(NHCH₂CH₂)_{b}-, -(NHCH₂CH₂CH₂)_{b}-, or -C(O)CH₂-;
L₄ is -(OCH₂CH₂)_{c}-, -(OCH₂CH₂CH₂)_{c}-, -(OCH₂CH₂CH₂CH₂)_{c}-, - (OCH₂CH₂CH₂CH₂CH₂)_{c}-, or -NHC(O)-(CH₂)_{d}-;
   where a is 0, 1, 2, or 3;
   b is 1, 2, 3, 4, or 5;
   c is 1, 2, 3, 4, or 5;
   d is 1, 2, 3, 4, 5, 6, 7, or 8;
L is -CH₂O- or -NHC(O)-;
L' is a bond, -C(O)NH-, or -NHC(O)-;
R₁ and R₂ together form -CH₂CH₂O- or -CH₂CH(R)-O-, and R₃ is H;
or the R₁ and the R₃ together form -C₁₋₂ alkylene-, and the R₂ is H;
   where R is -OR', -CH₂OR', or -CH₂CH₂OR', where R' is H, a hydroxyl protective group, or a solid support, and the hydroxyl protective group is preferably -C(O)CH₂CH₂C(O)OH or 4,4'-dimethoxytriphenylmethyl;
m is 0, 1, 2, 3, 4, 5, 6, 7, 8, 9, or 10; and
n is 0, 1, 2, 3, 4, 5, 6, 7, 8, 9, or 10.

In some specific embodiments, where,
the Q is independently H or 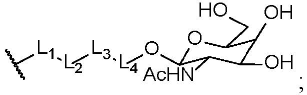
   where the Li is -CH₂O- or -NHC(O)-(CH₂NHC(O))ₐ-;
   the L₂ is -CH₂CH₂C(O)-;
   the L₃ is -(NHCH₂CH₂)_{b}- or -(NHCH₂CH₂CH₂)_{b}-;
   the L₄ is -(OCH₂CH₂)_{c}- or -NHC(O)-(CH₂)_{d}-;
      where the a is 0, 1, 2, or 3;
      the b is 1, 2, 3, 4, or 5;
      the c is 1, 2, 3, 4, or 5;
      the d is 1, 2, 3, 4, 5, 6, 7, or 8;
the L is -CH₂O-;
the L' is a bond;
the R₁ and the R₂ together form -CH₂CH₂O- or -CH₂CH(R)-O-, and the R₃ is H;
or the R₁ and the R₃ together form -C₁₋₂ alkylene-, and the R₂ is H;
   where the R is -OR', -CH₂OR', or -CH₂CH₂OR', where the R' is H, a hydroxyl protective group, or a solid support, and the hydroxyl protective group is preferably -C(O)CH₂CH₂C(O)OH or 4,4'-dimethoxytriphenylmethyl;
the m is 0, 1, 2, 3, 4, 5, 6, 7, 8, 9, or 10; and
the n is 0, 1, 2, 3, 4, 5, 6, 7, 8, 9, or 10.

In some embodiments, the conjugated group is shown in Formula (I'-1), Formula (I'-2), or Formula (I'-3): 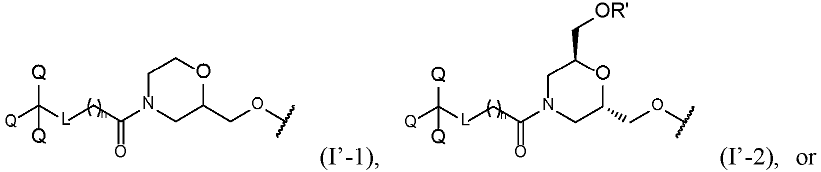 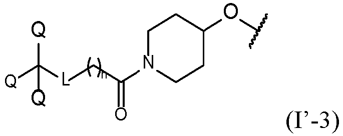 where, 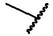 represents a position connected with a biomolecule;
Q is 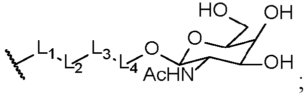
   where L₁ is -CH₂O- or -NHC(O)-;
   L₂ is -CH₂CH₂C(O)-;
   L₃ is -(NHCH₂CH₂)_{b}- or -(NHCH₂CH₂CH₂)_{b}-;
   L₄ is -(OCH₂CH₂)_{c}- or -NHC(O)-(CH₂)_{d}-;
      where b is 1, 2, 3, 4, or 5;
      c is 1, 2, 3, 4, or 5;
      d is 1, 2, 3, 4, 5, 6, 7, or 8;
L is -CH₂O-;
R' is H, a hydroxyl protective group, or a solid support, and the hydroxyl protective group is preferably -C(O)CH₂CH₂C(O)OH or 4,4'-dimethoxytriphenylmethyl; and
n is 0, 1, 2, 3, 4, 5, 6, 7, 8, 9, or 10.

In some specific embodiments, where,
the Q is independently H, 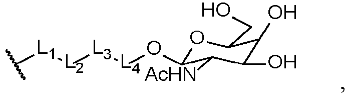 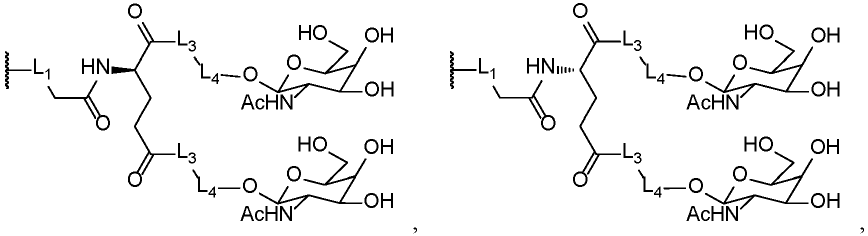 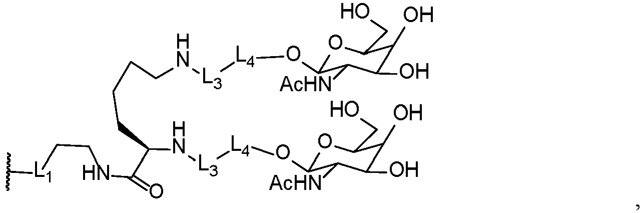 or 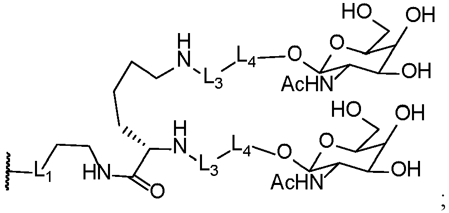
   where the L₁ is -CH₂O-, -CH₂O-CH₂CH₂O-, or -NHC(O)-(CH₂NHC(O))ₐ-;
   the L₂ is -CH₂CH₂C(O)-;
   the L₃ is -(NHCH₂CH₂)_{b}-, -(NHCH₂CH₂CH₂)_{b}-, or -C(O)CH₂-;
   the L₄ is -(OCH₂CH₂)_{c}- or -NHC(O)-(CH₂)_{d}-;
      where the a is 0, 1, 2, or 3;
      the b is 1, 2, 3, 4, or 5;
      the c is 1, 2, 3, 4, or 5;
      the d is 1, 2, 3, 4, 5, 6, 7, or 8;
the L is -CH₂O- or -NHC(O)-;
the L' is a bond or -C(O)NH-;
the R₁ and the R₂ together form -CH₂CH₂O- or -CH₂CH(R)-O-, and the R₃ is H;
or the R₁ and the R₃ together form -C₁₋₂ alkylene-, and the R₂ is H;
   where the R is -OR', -CH₂OR', or -CH₂CH₂OR', where the R' is H, a hydroxyl protective group, or a solid support, and the hydroxyl protective group is preferably -C(O)CH₂CH₂C(O)OH or 4,4'-dimethoxytriphenylmethyl;
the m is 0, 1, 2, 3, 4, 5, 6, 7, 8, 9, or 10; and
the n is 0, 1, 2, 3, 4, 5, 6, 7, 8, 9, or 10.

In some embodiments, the conjugated group is shown in Formula (II'-1) or Formula (II'-2): 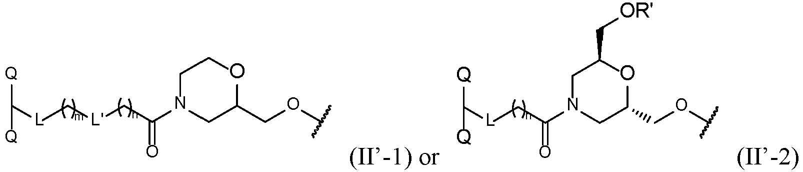 where, 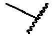 represents a position connected with a biomolecule;
Q is independently 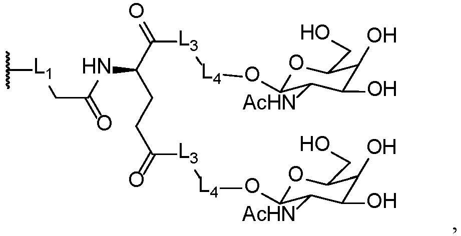 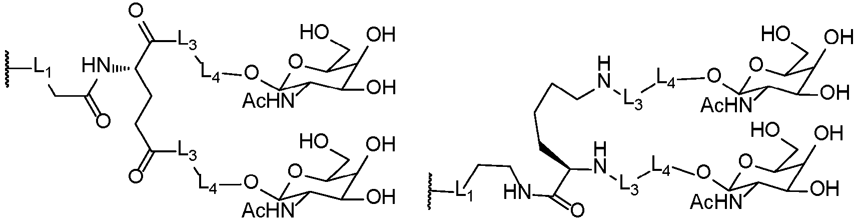 or 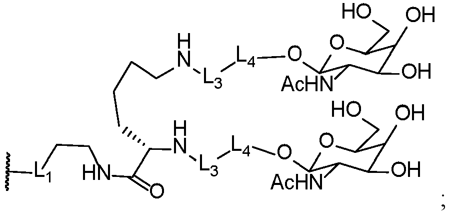
   where L₁ is -CH₂O- or -CH₂O-CH₂CH₂O-;
   L₃ is -(NHCH₂CH₂)_{b}-, -(NHCH₂CH₂CH₂)_{b}-, or -C(O)CH₂-;
   L₄ is -(OCH₂CH₂)_{c}- or -NHC(O)-(CH₂)_{d}-;
      where b is 1, 2, 3, 4, or 5;
      c is 1, 2, 3, 4, or 5;
      d is 1, 2, 3, 4, 5, 6, 7, or 8;
L is -NHC(O)-;
L' is a bond or -C(O)NH-;
R' is H, a hydroxyl protective group, or a solid support, and the hydroxyl protective group is preferably -C(O)CH₂CH₂C(O)OH or 4,4'-dimethoxytriphenylmethyl;
m is 0, 1, 2, 3, 4, 5, 6, 7, 8, 9, or 10; and
n is 0, 1, 2, 3, 4, 5, 6, 7, 8, 9, or 10.

In some specific embodiments, where,
the Q is independently H, 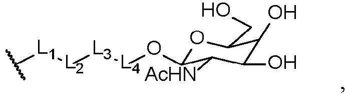 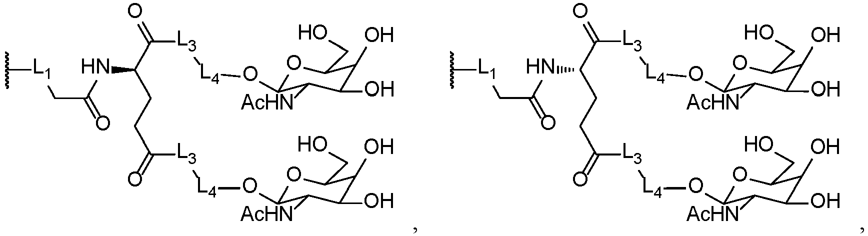 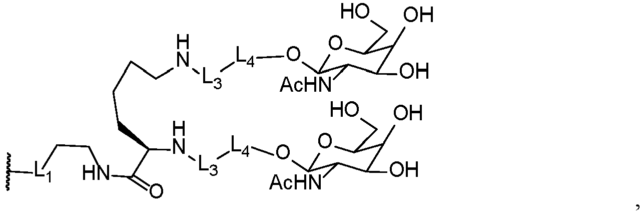 or 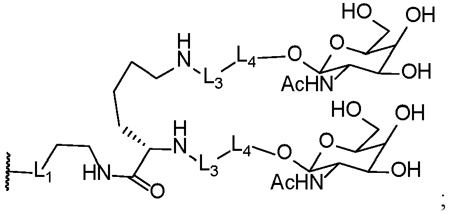
   where the Li is -CH₂-, -C(O)-, -CH₂O-, -CH₂O-CH₂CH₂O-, or -NHC(O)-(CH₂NHC(O))ₐ-;
   the L₂ is a bond;
   the L₃ is -(NHCH₂CH₂)_{b}-, -(NHCH₂CH₂CH₂)_{b}-, or -C(O)CH₂-;
   the L₄ is -(OCH₂CH₂)_{c}- or -NHC(O)-(CH₂)_{d}-;
      where the a is 0, 1, 2, or 3;
      the b is 1, 2, 3, 4, or 5;
      the c is 1, 2, 3, 4, or 5;
      the d is 1, 2, 3, 4, 5, 6, 7, or 8;
the L is -CH₂O- or -NHC(O)-;
the L' is a bond or -C(O)NH-;
the R₁ and the R₂ together form -CH₂CH₂O- or -CH₂CH(R)-O-, and the R₃ is H;
or the R₁ and the R₃ together form -C₁₋₂ alkylene-, and the R₂ is H;
   where the R is -OR', -CH₂OR', or -CH₂CH₂OR', where the R' is H, a hydroxyl protective group, or a solid support, and the hydroxyl protective group is preferably -C(O)CH₂CH₂C(O)OH or 4,4'-dimethoxytriphenylmethyl;
the m is 0, 1, 2, 3, 4, 5, 6, 7, 8, 9, or 10; and
the n is 0, 1, 2, 3, 4, 5, 6, 7, 8, 9, or 10.

In some embodiments, the conjugated group is shown in Formula (II'-2): 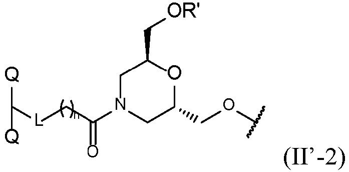 where, 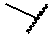 represents a position connected with a biomolecule;
Q is independently 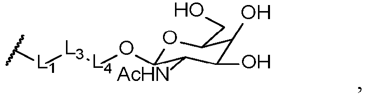 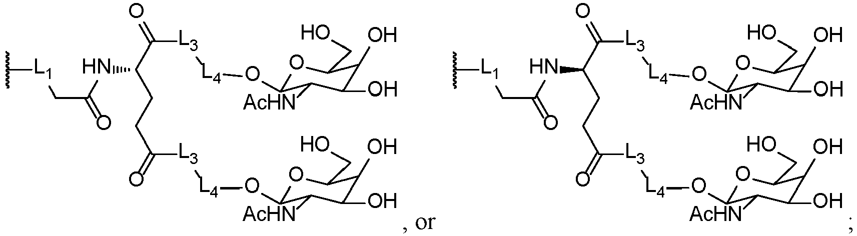
   where L₁ is -CH₂- or -C(O)-;
   L₃ is -(NHCH₂CH₂)_{b}-;
   L₄ is -(OCH₂CH₂)_{c}-;
      where b is 1, 2, 3, 4, or 5;
      c is 1, 2, 3, 4, or 5;
L is -CH₂O- or -NHC(O)-;
R' is H, a hydroxyl protective group, or a solid support, and the hydroxyl protective group is preferably -C(O)CH₂CH₂C(O)OH or 4,4'-dimethoxytriphenylmethyl; and
n is 0, 1, 2, 3, 4, 5, 6, 7, 8, 9, or 10.

In some specific embodiments, where,
the Q is independently H, 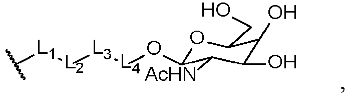 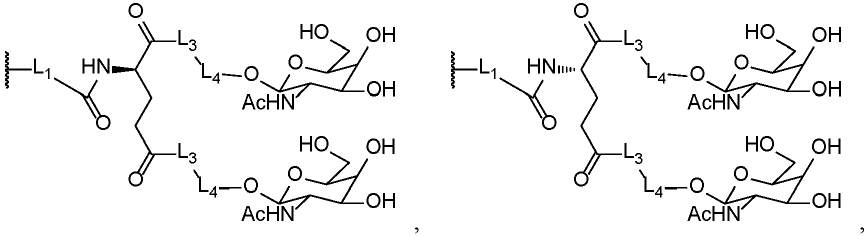 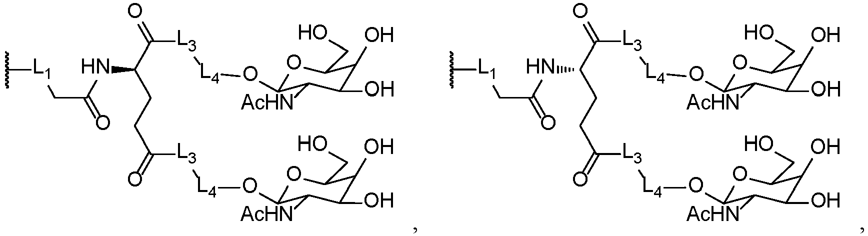 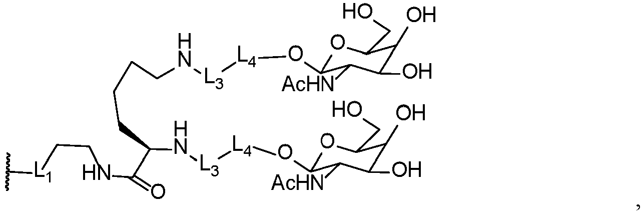 or 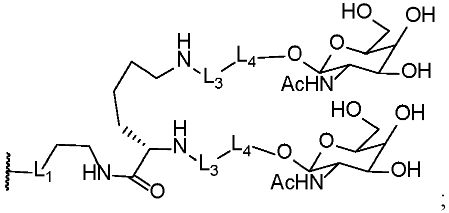
   where the L₁ is a bond, -CH₂-, -CH₂CH₂-, -C(O)-, -CH₂O-, -CH₂O-CH₂CH₂O-, or -NHC(O)-(CH₂NHC(O))ₐ-;
   the L₂ is a bond or -CH₂CH₂C(O)-;
   the L₃ is a bond, -(NHCH₂CH₂)_{b}-, -(NHCH₂CH₂CH₂)_{b}-, or -C(O)CH₂-;
   the L₄ is -(OCH₂CH₂)_{c}-, -(OCH₂CH₂CH₂)_{c}-, -(OCH₂CH₂CH₂CH₂)_{c}-, - (OCH₂CH₂CH₂CH₂CH₂)_{c}-, or -NHC(O)-(CH₂)_{d}-;
      where the a is 0, 1, 2, or 3;
      the b is 1, 2, 3, 4, or 5;
      the c is 1, 2, 3, 4, or 5;
      the d is 1, 2, 3, 4, 5, 6, 7, or 8;
the L is a bond, -CH₂O-, or -NHC(O)-;
the L' is a bond, -C(O)NH-, -NHC(O)-, or -O(CH₂CH₂O)ₑ-;
   where the e is 1, 2, 3, 4, or 5;
the T is a bond, -CH₂-, -M-, -CH₂-M-, or -C(O)-M-;
   where the M is 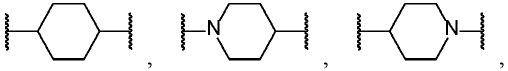 or 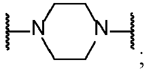
the R₁ and the R₂ together form -CH₂CH₂O- or -CH₂CH(R)-O-, and the R₃ is H;
or the R₁ and the R₃ together form -C₁₋₂ alkylene-, and the R₂ is H;
   where the R is -OR', -CH₂OR', or -CH₂CH₂OR', where the R' is H, a hydroxyl protective group, or a solid support, and the hydroxyl protective group is preferably -C(O)CH₂CH₂C(O)OH or 4,4'-dimethoxytriphenylmethyl;
the m is 0, 1, 2, 3, 4, 5, 6, 7, 8, 9, or 10; and
the n is 0, 1, 2, 3, 4, 5, 6, 7, 8, 9, or 10.

In some specific embodiments, where,
the T is -M-, -CH₂-M-, or -C(O)-M-, where the M is

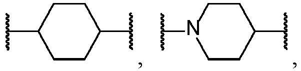

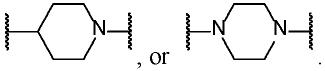

In some specific embodiments, where,
the Q is independently H or 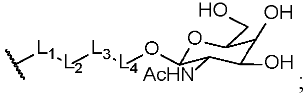
   where the Li is -CH₂O- or -NHC(O)-(CH₂NHC(O))ₐ-;
   the L₂ is -CH₂CH₂C(O)-;
   the L₃ is -(NHCH₂CH₂)_{b}- or -(NHCH₂CH₂CH₂)_{b}-;
   the L₄ is -(OCH₂CH₂)_{c}- or -NHC(O)-(CH₂)_{d}-;
      where the a is 0, 1, 2, or 3;
      the b is 1, 2, 3, 4, or 5;
      the c is 1, 2, 3, 4, or 5;
      the d is 1, 2, 3, 4, 5, 6, 7, or 8;
the L is a bond or -CH₂O-;
the L' is a bond or -O(CH₂CH₂O)ₑ-;
   where the e is 1, 2, 3, 4, or 5;
the R₁ and the R₂ together form -CH₂CH₂O- or -CH₂CH(R)-O-, and the R₃ is H;
or the R₁ and the R₃ together form -C₁₋₂ alkylene-, and the R₂ is H;
   where the R is -OR', -CH₂OR', or -CH₂CH₂OR', where the R' is H, a hydroxyl protective group, or a solid support, and the hydroxyl protective group is preferably -C(O)CH₂CH₂C(O)OH or 4,4'-dimethoxytriphenylmethyl;
the m is 0, 1, 2, 3, 4, 5, 6, 7, 8, 9, or 10;
the n is 0, 1, 2, 3, 4, 5, 6, 7, 8, 9, or 10; and
the T is as defined according to the foregoing embodiments.

In some embodiments, the conjugated group is shown in Formula (III'-1), Formula (III'-2), or Formula (III'-3): 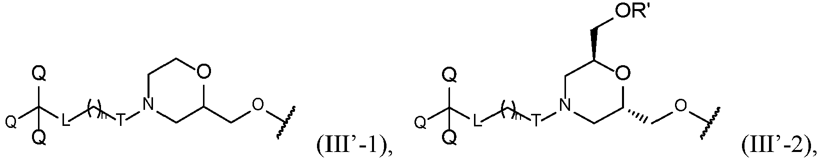 or 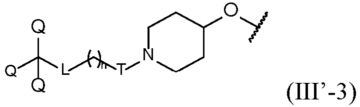 where,
Q is 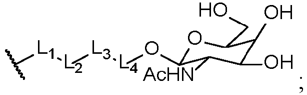
   where L₁ is -CH₂O- or -NHC(O)-;
   L₂ is -CH₂CH₂C(O)-;
   L₃ is -(NHCH₂CH₂)_{b}- or -(NHCH₂CH₂CH₂)_{b}-;
   L₄ is -(OCH₂CH₂)_{c}- or -NHC(O)-(CH₂)_{d}-;
      where b is 1, 2, 3, 4, or 5;
      c is 1, 2, 3, 4, or 5;
      d is 1, 2, 3, 4, 5, 6, 7, or 8;
L is a bond or -CH₂O-;
where R' is H, a hydroxyl protective group, or a solid support, and the hydroxyl protective group is preferably -C(O)CH₂CH₂C(O)OH or 4,4'-dimethoxytriphenylmethyl;
n is 0, 1, 2, 3, 4, 5, 6, 7, 8, 9, or 10; and
T is as defined according to the foregoing embodiments.

In some specific embodiments, where,
the Q is independently H, 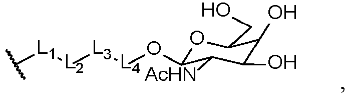 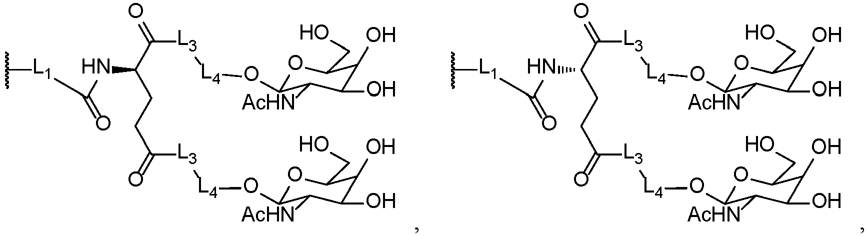 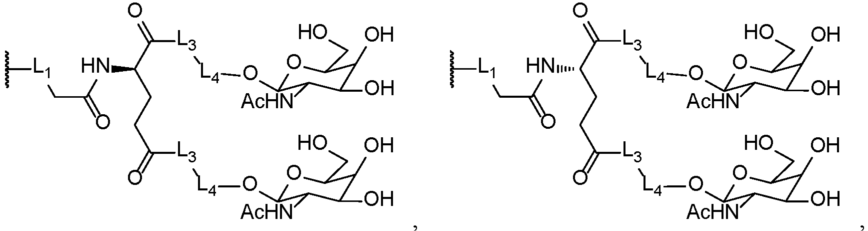 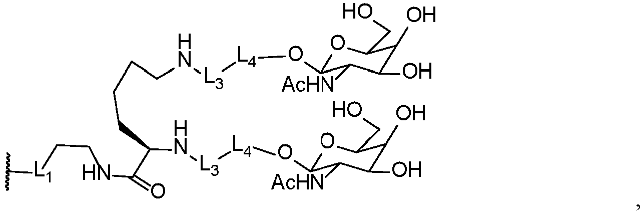 or 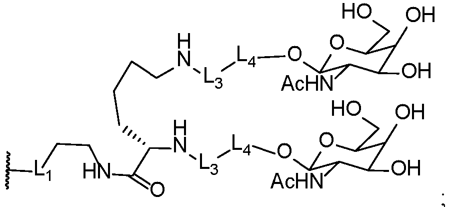
   where the Li is -CH₂-, -CH₂O-, or -C(O)-;
   the L₂ is a bond;
   the L₃ is -(NHCH₂CH₂)_{b}-, -(NHCH₂CH₂CH₂)_{b}-, or -C(O)CH₂-;
   the L₄ is -(OCH₂CH₂)_{c}- or -NHC(O)-(CH₂)_{d}-;
      where the b is 1, 2, 3, 4, or 5;
      the c is 1, 2, 3, 4, or 5;
      the d is 1, 2, 3, 4, 5, 6, 7, or 8;
the L is a bond or -NHC(O)-;
the L' is a bond;
the R₁ and the R₂ together form -CH₂CH₂O- or -CH₂CH(R)-O-, and the R₃ is H;
or the R₁ and the R₃ together form -C₁₋₂ alkylene-, and the R₂ is H;
   where the R is -OR', -CH₂OR', or -CH₂CH₂OR', where the R' is H, a hydroxyl protective group, or a solid support, and the hydroxyl protective group is preferably -C(O)CH₂CH₂C(O)OH or 4,4'-dimethoxytriphenylmethyl;
the m is 0, 1, 2, 3, 4, 5, 6, 7, 8, 9, or 10;
the n is 0, 1, 2, 3, 4, 5, 6, 7, 8, 9, or 10; and
the T is as defined according to the foregoing embodiments.

In some embodiments, the conjugated group is shown in Formula (IV-1) or Formula (IV-2): 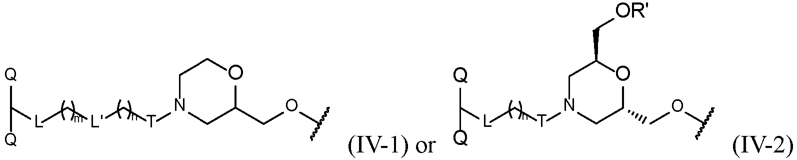 where,
Q is independently 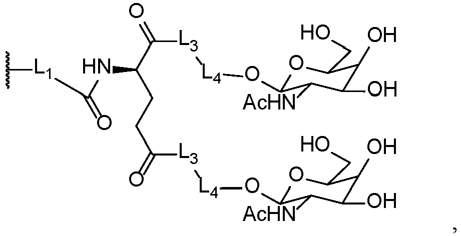 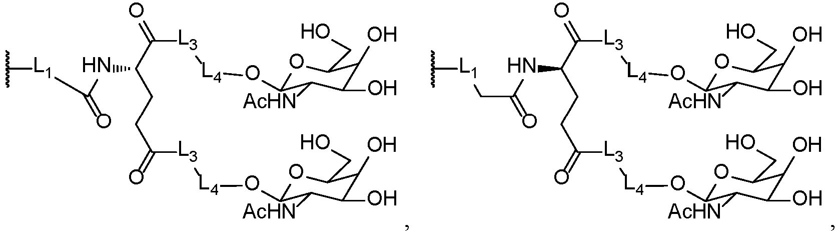 or 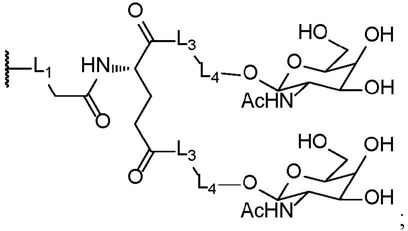
   where Li is -CH₂-, -CH₂O-, or -C(O)-;
   L₃ is -(NHCH₂CH₂)_{b}-, -(NHCH₂CH₂CH₂)_{b}-, or -C(O)CH₂-;
   L₄ is -(OCH₂CH₂)_{c}- or -NHC(O)-(CH₂)_{d}-;
      where b is 1, 2, 3, 4, or 5;
      c is 1, 2, 3, 4, or 5;
      d is 1, 2, 3, 4, 5, 6, 7, or 8;
L is a bond or -NHC(O)-;
L' is a bond;
where R' is H, a hydroxyl protective group, or a solid support, and the hydroxyl protective group is preferably -C(O)CH₂CH₂C(O)OH or 4,4'-dimethoxytriphenylmethyl;
m is 0, 1, 2, 3, 4, 5, 6, 7, 8, 9, or 10;
n is 0, 1, 2, 3, 4, 5, 6, 7, 8, 9, or 10; and
T is as defined according to the foregoing embodiments.

In some specific embodiments, where,
the Q is independently H, 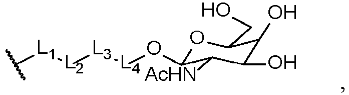 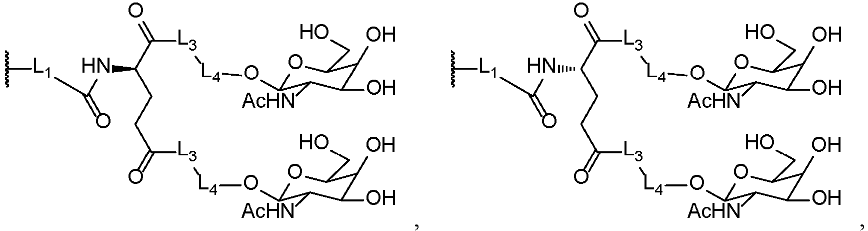 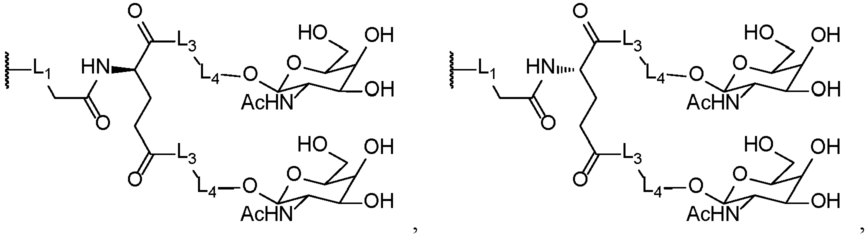 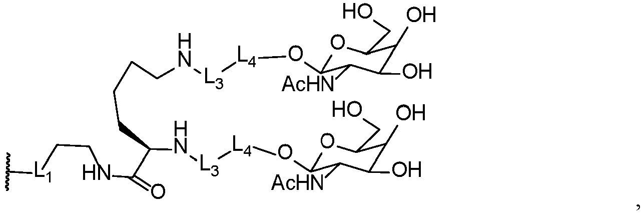 or 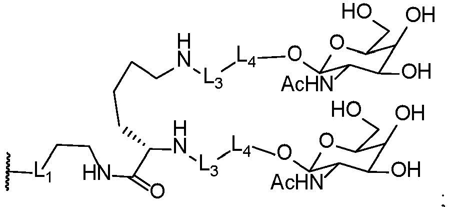
   where the L₁ is a bond, -CH₂-, -CH₂CH₂-, -C(O)-, -CH₂O-, -CH₂O-CH₂CH₂O-, or -NHC(O)-(CH₂NHC(O))ₐ-;
   the L₂ is a bond or -CH₂CH₂C(O)-;
   the L₃ is a bond, -(NHCH₂CH₂)_{b}-, -(NHCH₂CH₂CH₂)_{b}-, or -C(O)CH₂-;
   the L₄ is -(OCH₂CH₂)_{c}-, -(OCH₂CH₂CH₂)_{c}-, -(OCH₂CH₂CH₂CH₂)_{c}-, - (OCH₂CH₂CH₂CH₂CH₂)_{c}-, or -NHC(O)-(CH₂)_{d}-;
      where the a is 0, 1, 2, or 3;
      the b is 1, 2, 3, 4, or 5;
      the c is 1, 2, 3, 4, or 5;
      the d is 1, 2, 3, 4, 5, 6, 7, or 8;
the L is a bond, -CH₂O-, or -NHC(O)-;
the L' is -O(CH₂CH₂O)ₑ-;
   where the e is 1, 2, 3, 4, or 5;
the T is a bond, -CH₂-, -C(O)-, -M-, -CH₂-M-, or -C(O)-M-;
   where the M is 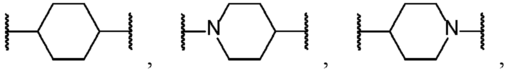 or 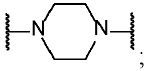
the R₁ and the R₂ together form -CH₂CH₂O- or -CH₂CH(R)-O-, and the R₃ is H;
or the R₁ and the R₃ together form -C₁₋₂ alkylene-, and the R₂ is H;
   where the R is -OR', -CH₂OR', or -CH₂CH₂OR', where the R' is H, a hydroxyl protective group, or a solid support, and the hydroxyl protective group is preferably -C(O)CH₂CH₂C(O)OH or 4,4'-dimethoxytriphenylmethyl;
the m is 0, 1, 2, 3, 4, 5, 6, 7, 8, 9, or 10; and
the n is 0, 1, 2, 3, 4, 5, 6, 7, 8, 9, or 10.

In some preferred embodiments, the conjugated group is selected from the following:

| Nu mbe r | Structure |
|---|---|
| 1 | 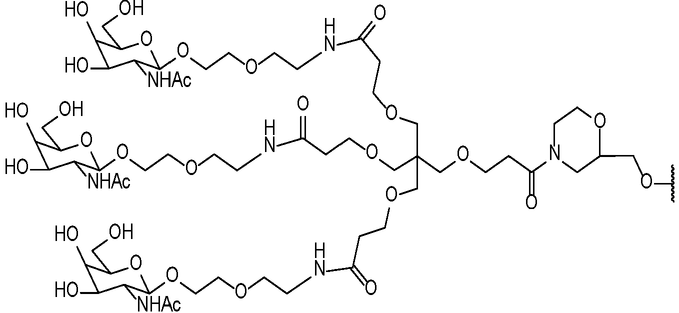 |
| 2 | 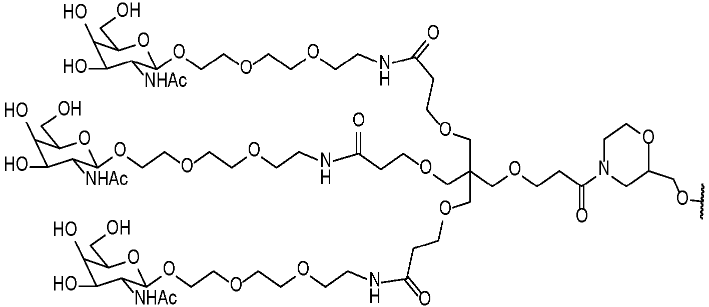 |
| 3 | 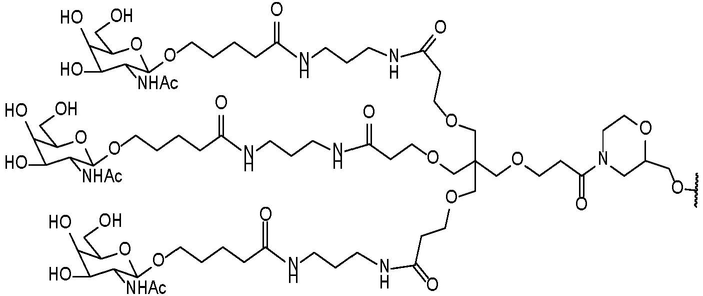 |
| 4 | 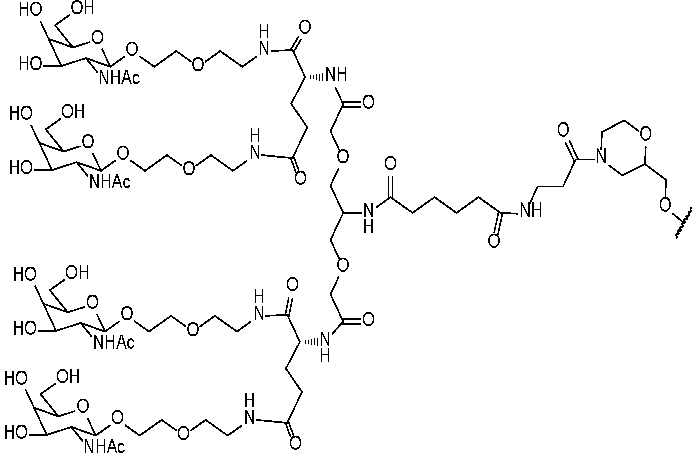 |
| 5 | 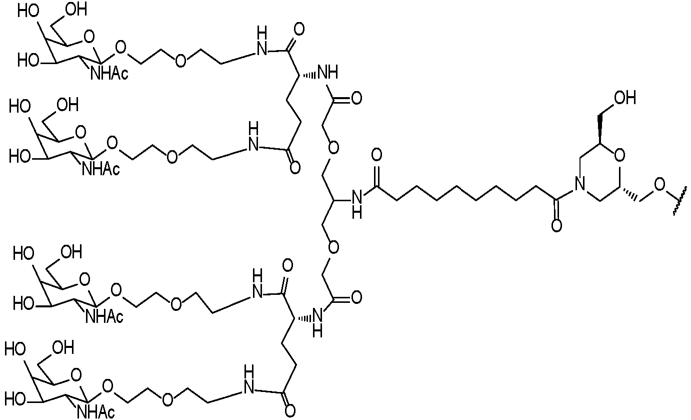 |
| 6 | 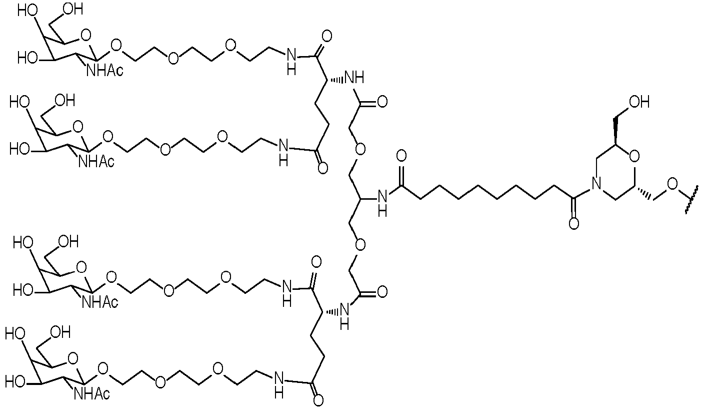 |
| 7 | 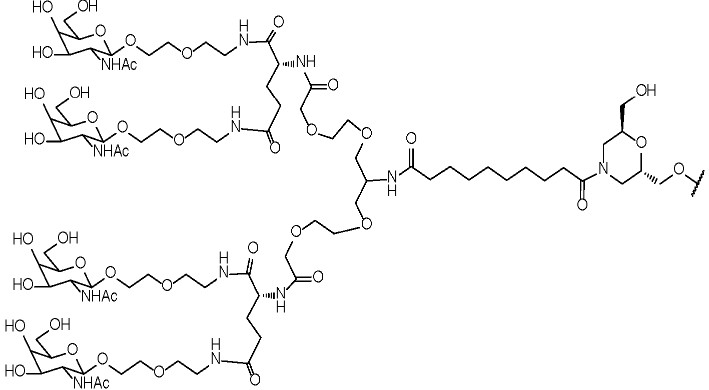 |
| 8 | 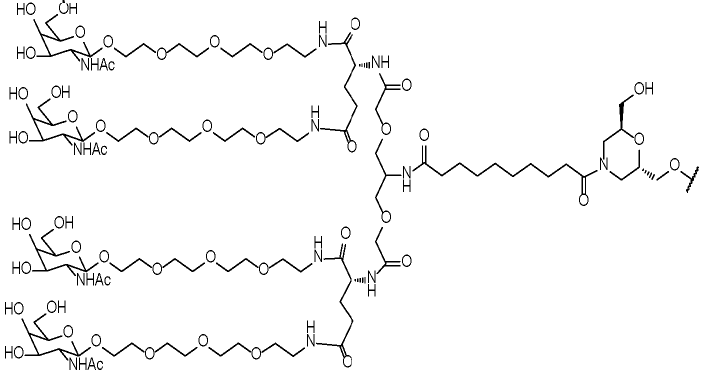 |
| 9 | 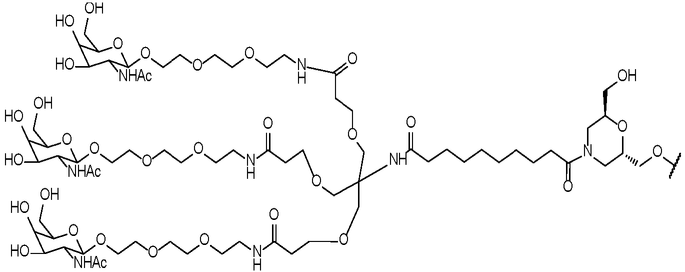 |
| 10 | 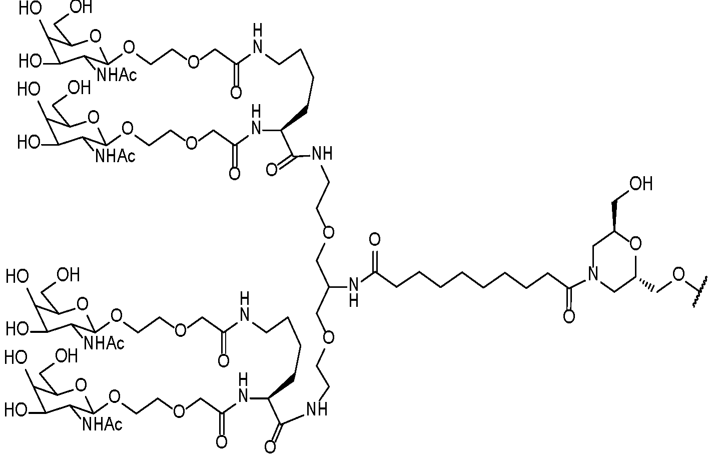 |
| 11 | 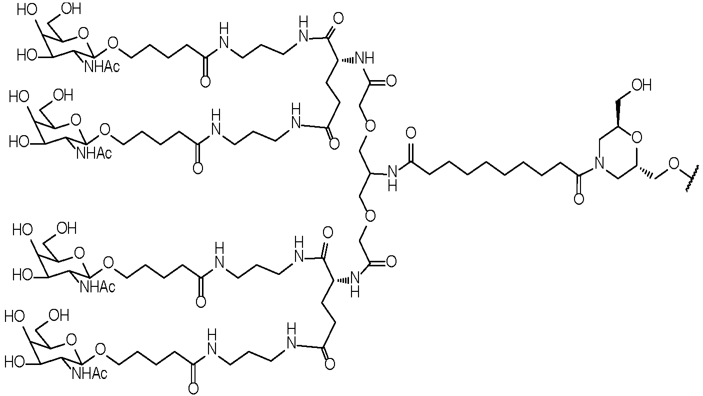 |
| 12 | 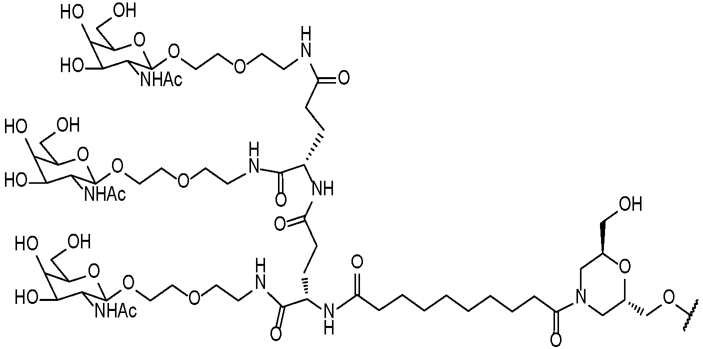 |
| 13 | 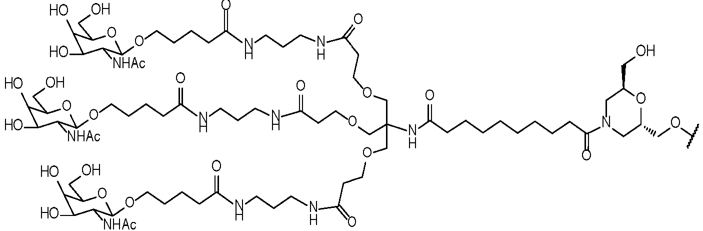 |
| 14 | 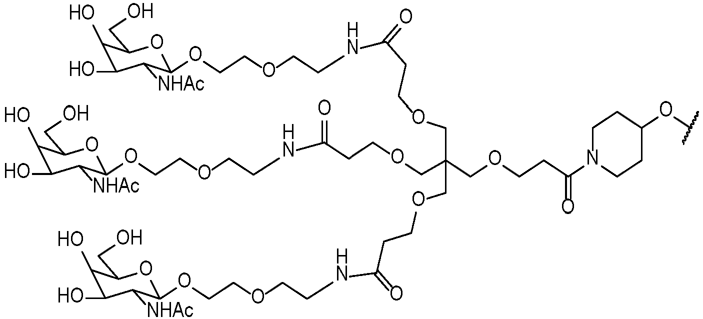 |
| 15 | 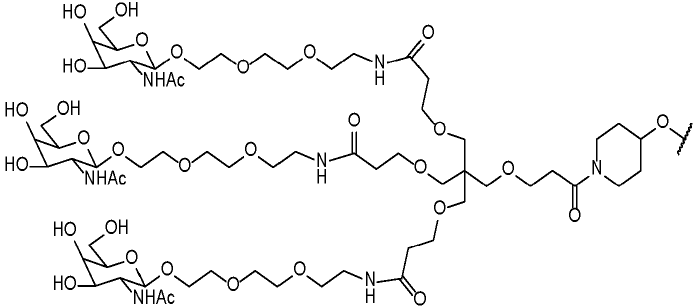 |
| 16 | 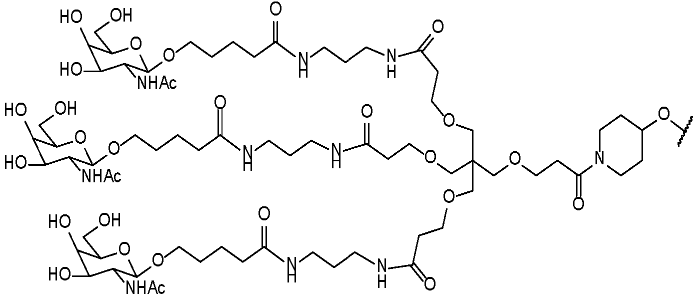 ∘ |

In some preferred embodiments, the conjugated group is selected from the following:

| Number | Structure |
|---|---|
| 17 | 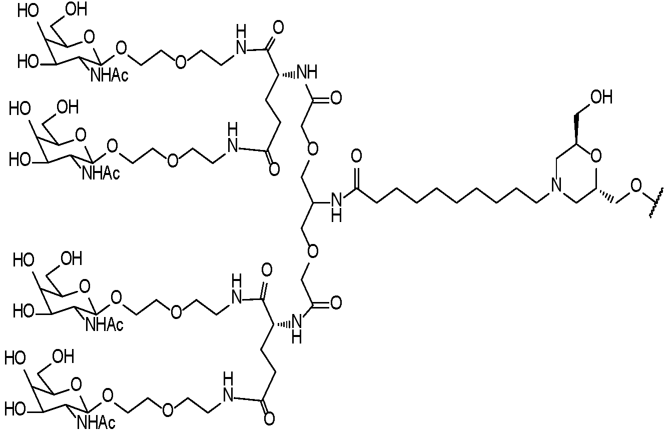 |
| 18 | 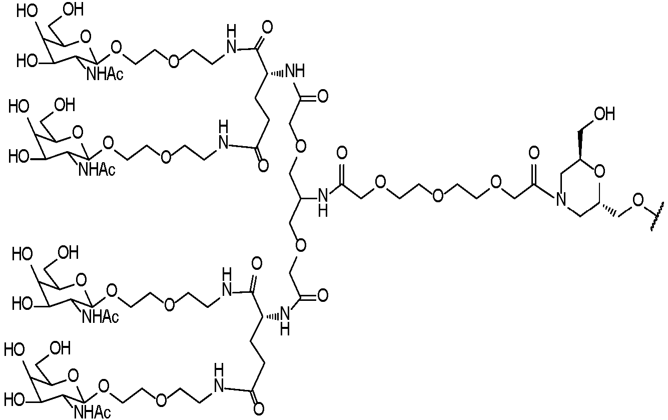 |
| 19 | 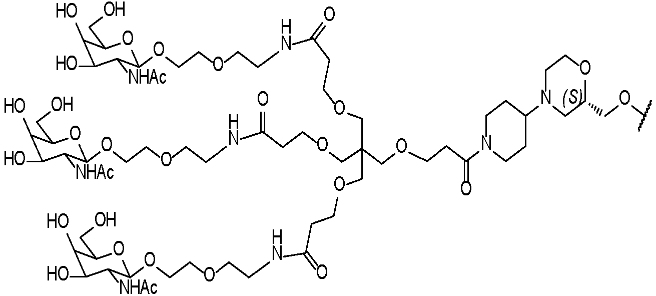 |
| 20 | 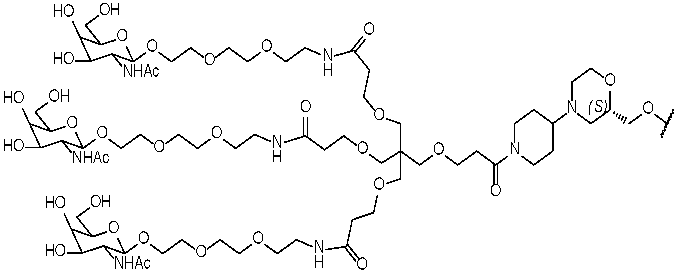 |
| 21 | 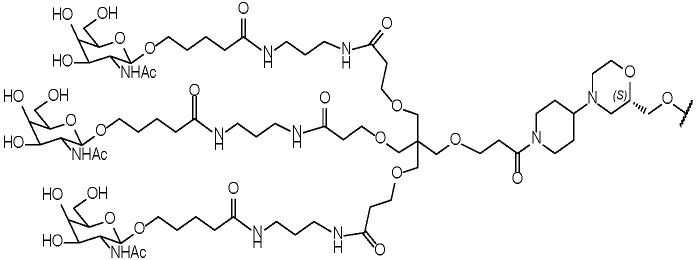 |
| 22 | 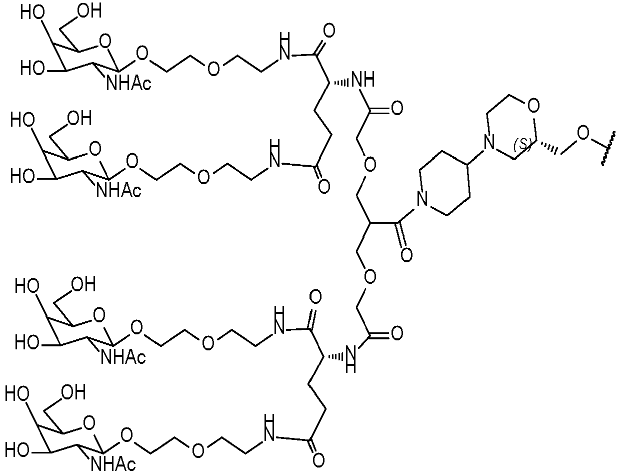 |
| 23-1 | 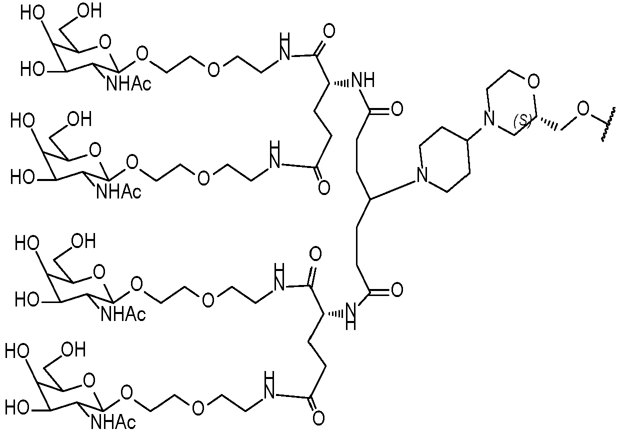 |
| 23-2 | 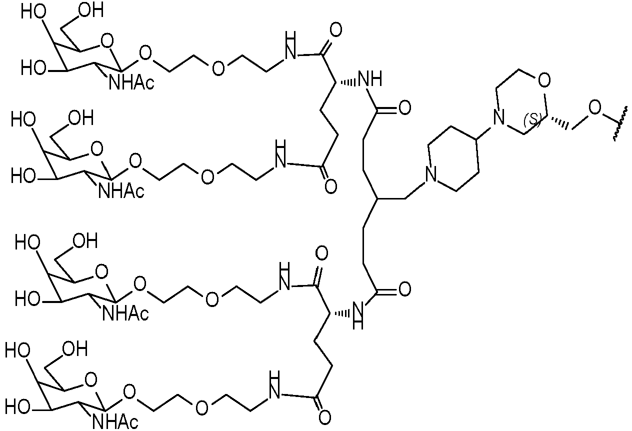 |
| 24 | 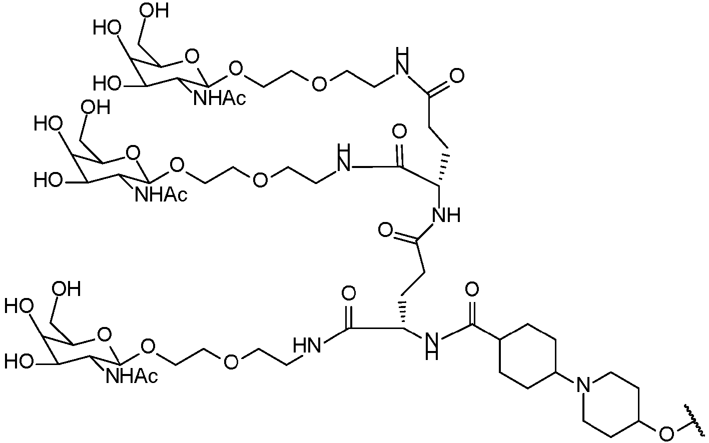 |
| 25 | 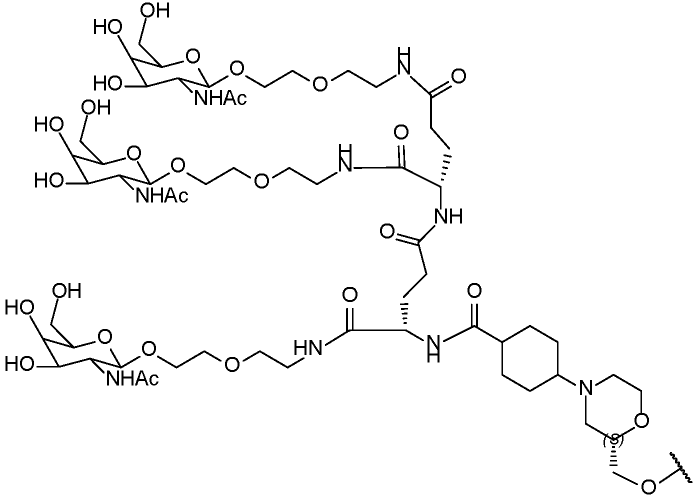 |
| 26 | 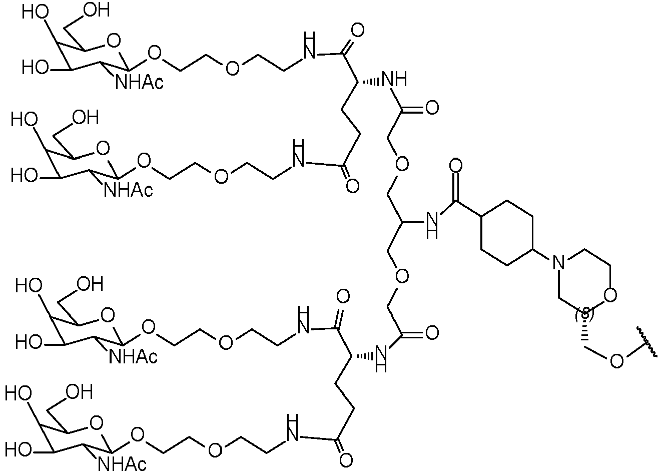 |
| 27 | 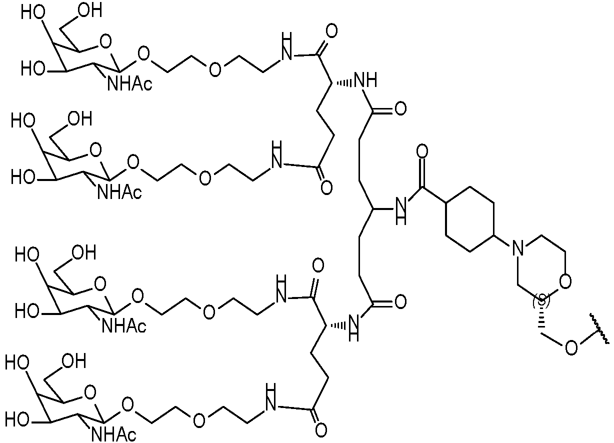 |
| 28 | 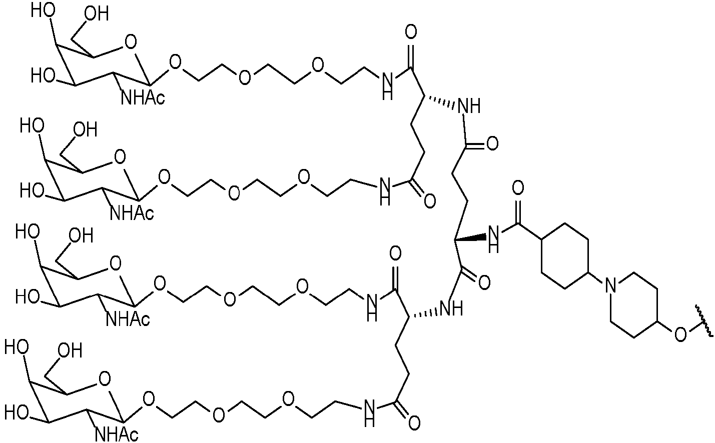 |
| 29 | 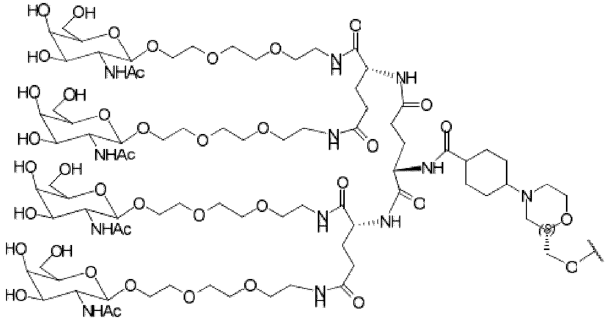 ∘ |

In some embodiments, the ligand targets an asialoglycoprotein receptor (ASGPR).

In one preferred embodiment, the ligand has the following structure: 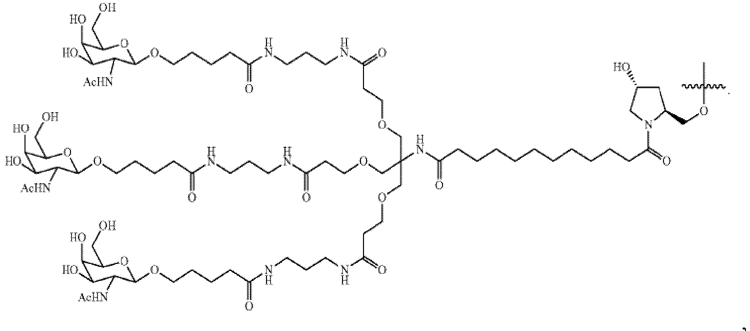 where 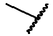 represents a position connected with the siRNA through the phosphate group or the thiophosphate group.

In one preferred embodiment, the ligand has the following structure: 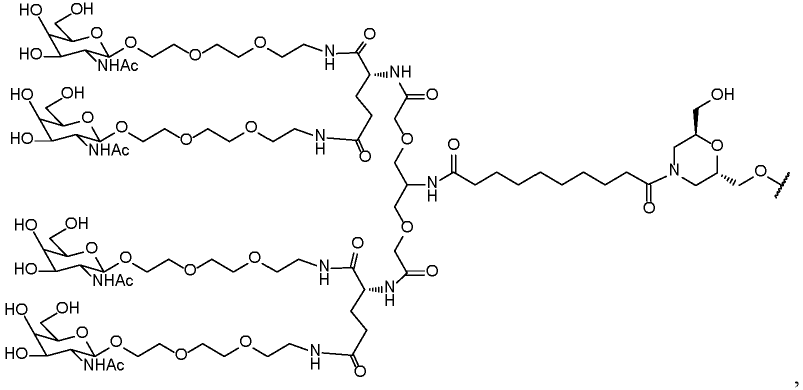 where 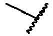 represents a position connected with the sense strand of the siRNA through the phosphate group or the thiophosphate group.

In one preferred embodiment, the ligand has the following structure: 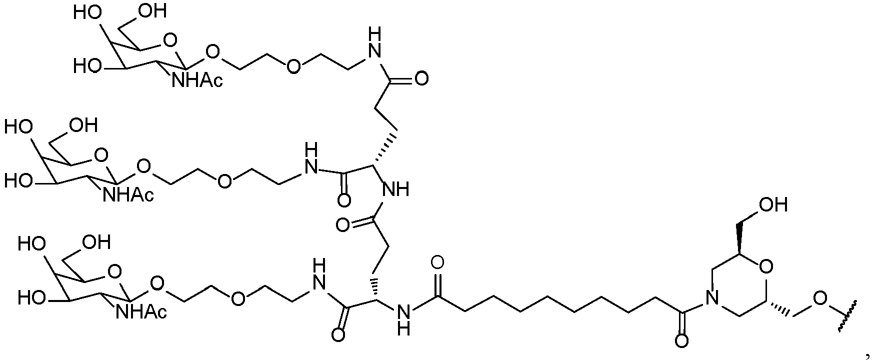 where 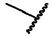 represents a position connected with the sense strand of the siRNA through the phosphate group or the thiophosphate group.

### IV. Inhibition of PCSK9 gene expression

The siRNA of the present invention can inhibit PCSK9 gene expression by at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99%.

Inhibition of PCSK9 gene expression may be demonstrated by a decrease in an amount of an mRNA expressed by a first cell or cell population (such cell may present in, for example, a sample derived from a subject), where the PCSK9 gene is transcribed and the cell or these cells have been treated (for example, by making the cell or these cells contact with the siRNA of the present invention, or by giving the siRNA of the present invention to a subject in which the cells are present or previously present), such that the PCSK9 gene expression is inhibited compared with a second cell or cell population (one or more control cells) that is substantially identical to the first cell or cell population but has not been treated in this way.

In preferred embodiments, the inhibition is evaluated by expressing a level of an mRNA in the treated cell as a percentage of a level of an mRNA in a control cell using a formula below. In some specific embodiments, a 2^{-ΔΔ^{Ct}} value is calculated to compare differences between an experimental group and a control group, where ΔΔCt=[(Ct value of a target gene in the experimental group-Ct value of an internal reference in the experimental group)-(Ct value of a target gene in the control group-Ct value of an internal reference in the control group)].

Alternatively, the PCSK9 gene expression, such as the inhibition of PCSK9 gene expression, can be evaluated based on a decrease of parameters (such as a lipid level, or a cholesterol level, such as an LDLc level) associated with functions of the PCSK9 gene expression. PCSK9 gene silencing can be determined in any cell that expresses PCSK9 through constitutive or genomic engineering and through any known assay in the field. A liver is a main site for PCSK9 expression. Other important expression sites include a pancreas, a kidney and intestines.

Inhibition of PCSK9 protein expression can be demonstrated by a decrease in a PCSK9 protein level (such as a protein level expressed in a sample derived from a subject) expressed by a cell or cell population. As explained above in evaluation of mRNA inhibition, the inhibition of a protein expression level in a treated cell or cell population can be similarly expressed as a percentage of a protein level in a control cell or cell population.

The control cell or cell population that can be used for evaluating the inhibition of PCSK9 gene expression include cells or cell populations that have not yet been getting in contact with the siRNA of the present invention. For example, the control cell or cell population can be derived from individual subjects (such as human beings or animal subjects) before the subjects are treated with the siRNA.

### V. Vector

The present invention provides a vector, which includes a nucleotide sequence encoding the siRNA of the present invention. The vector of the present invention can amplify or express nucleotides that are connected thereto and encode the siRNA of the present invention.

The siRNA targeting the PCSK9 gene can be expressed from a transcription unit inserted into a DNA or RNA vector. Expression can be transient (within several hours to several weeks) or sustained (several weeks to several months or longer), depending on a specific construct used and a target tissue or cell type. Nucleotides encoding the siRNA targeting the PCSK9 gene can be introduced into a linear construct, a circular plastid, or a viral vector. The nucleotides encoding the siRNA targeting the PCSK gene can be incorporated into a cell genome for stable expression, or be stably inherited and expressed outside chromosomes. In general, siRNA expression vectors are usually DNA plasmids or viral vectors.

A viral vector system containing an encoding sequence of the siRNA targeting the PCSK9 gene includes, but is not limited to: (a) adenovirus vectors; (b) retroviral vectors; (c) adeno-associated virus vectors; (d) herpes simplex virus vectors; (e) SV40 vectors; (f) polyomavirus vectors; (g) papillomavirus vectors; (h) picornavirus vectors; (i) poxvirus vectors; and (j) helper virus-dependent adenoviruses or gutless adenoviruses.

### VI. Cell

The present invention provides a cell, which includes the siRNA or the vector of the present invention, where the siRNA or the vector of the present invention can be transcribed in the cell.

### VII. Pharmaceutical composition

The present invention provides a pharmaceutical composition, which includes the siRNA, the vector or the cell of the present invention, and an optional pharmaceutically acceptable carrier or excipient.

The "pharmaceutically acceptable" used herein refers such compounds, materials, compositions and/or dosage forms that are suitable for use in contact with tissues of human subjects and animal subjects without causing excessive toxicity, irritation, anaphylaxis or other problems or complications within a correct medical judgment range, commensurate with a reasonable benefit/risk ratio.

Herein, the pharmaceutically acceptable carrier refers to a drug carrier that facilitates administration of the siRNA or the vector containing the encoding sequence or the cell to the human body and/or facilitates absorption or exertion of functions, for example, a diluent; an excipient, such as water; a filler, such as starch, sucrose, etc.; an adhesive, such as a cellulose derivative, alginate, gelatin, and polyvinylpyrrolidone; a wetting agent, such as glycerol; a disintegrating agent, such as agar, calcium carbonate, and sodium bicarbonate; an absorption enhancer, such as a quaternary ammonium compound; a surfactant, such as cetanol; an adsorption carrier, such as kaolin and bentonite; a lubricant, such as talc powder, calcium/magnesium stearate, polyethylene glycol, etc.. In addition, other adjuvants, such as a flavoring agent, a sweetener, etc., can also be added to the composition.

For example, the pharmaceutical composition including the siRNA, the vector or the cell of the present invention may include a pharmaceutically acceptable diluent or slow-release matrix, and the siRNA or the vector of the present invention is embedded into the slow-release matrix.

### VIII. Kit

The present invention provides a kit, which includes the siRNA, the vector or the cell of the present invention.

The present invention further provides a kit for using the siRNA of the present invention and/or implementing a method of the present invention. Such a kit includes one or more of the siRNA, the vector or the cell of the present invention and may further include an operation instruction. The operation instruction may record an instruction for inhibiting PCSK9 expression in the cell by making the cell contact with the siRNA or the vector of the present invention at an amount that effectively inhibits the PCSK9 expression.

In the case that the siRNA or the vector of the present invention is in contact with the cell in vitro, optionally, the kit of the present invention may also include a tool for making the cell contact with the siRNA or the vector of the present invention (such as, an injection device) or a tool for measuring an inhibitory effect of PCSK9 (such as, a device for measuring the inhibition of a PCSK9 mRNA or protein). Such device for measuring the inhibition of PCSK9 may include a device for obtaining a sample (such as, a plasma sample) from a subject.

In the case that the siRNA or the vector of the present invention or the cell into which the siRNA or the vector has been introduced in vitro is administered to the body, the kit of the present invention may also optionally include a device for administering the siRNA, the vector or the cell of the present invention to a subject or a device for determining an effective therapeutic dose or an effective preventive dose.

### IX. Therapeutic method and pharmaceutical use

The present invention provides a method for treating diseases or symptoms associated with expression of PCSK9 in a subject, and the method includes a step of administering the siRNA, the vector, the cell or the pharmaceutical composition of the present invention to the subject.

In some embodiments, the diseases associated with expression of PCSK9 are cardiovascular diseases. In some preferred embodiments, the cardiovascular diseases are selected from hyperlipidemia, hypercholesterolemia, nonfamilial hypercholesterolemia, polygenic hypercholesterolemia, familial hypercholesterolemia, homozygous familial hypercholesterolemia, heterozygous familial hypercholesterolemia, coronary heart disease, myocardial infarction, stroke, or atherosclerosis.

In some embodiments, the diseases associated with expression of PCSK9 are neoplastic diseases. In some preferred embodiments, the neoplastic diseases are selected from melanoma, hepatocellular carcinoma, or metastatic hepatic cancer.

In some embodiments, the diseases associated with expression of PCSK9 are T cell-mediated inflammatory immune diseases. In some preferred embodiments, the T cell-mediated inflammatory immune diseases are selected from psoriasis, psoriatic arthritis, eczema, atopic dermatitis, urticaria, hormone-dependent dermatitis, rheumatoid arthritis, scleroderma, diabetes mellitus, chronic liver diseases, or lymphoma.

In some embodiments, the method for treating diseases or symptoms associated with expression of PCSK9 in a subject in the present invention includes administering the siRNA or the pharmaceutical composition to the subject, including subcutaneous administration or intravenous administration. In some embodiments, the subject is a human patient.

The present invention also relates to the siRNA, the vector, the cell or the pharmaceutical composition of the present invention that are used for treating diseases or symptoms associated with expression of PCSK9 in a subject.

The present invention also relates to use of the siRNA, the vector, the cell or the pharmaceutical composition of the present invention in preparation of drugs for treatment of diseases or symptoms associated with expression of PCSK9 in a subject. The drugs of the present invention can be prepared into emulsions, micro-emulsions, and micro-particles.

### Sequence

An RNA sequence provided by the present invention targets a human PCSK9 gene (or a target gene, a target mRNA sequence, or a target sequence).

**Table 3. Nucleotide sequences of the sense strand and the antisense strand that target PCSK9 gene as used in the present invention**

| SEQ ID NO: | Sense strand sequence (5' → 3') | SE Q ID NO: | Antisense strand sequence (5' → 3') |
|---|---|---|---|
| 1 | CCUACGUGGUGGUGCUGAA | 105 | UUCAGCACCACCACGUAGGUG |
| 2 | GGUGGAGGUGUAUCUCCUA | 106 | UAGGAGAUACACCUCCACCAG |
| 3 | GGGUCAUGGUCACCGACUU | 107 | AAGUCGGUGACCAUGACCCUG |
| 4 | CCUGGAGUUUAUUCGGAAA | 108 | UUUCCGAAUAAACUCCAGGCC |
| 5 | GCACCUGCUUUGUGUCACA | 109 | UGUGACACAAAGCAGGUGCUG |
| 6 | CCAAAGAUGUCAUCAAUGA | 110 | UCAUUGAUGACAUCUUUGGCA |
| 7 | GCUGUUUUGCAGGACUGUA | 111 | UACAGUCCUGCAAAACAGCUG |
| 8 | GGUCUGGAAUGCAAAGUCA | 112 | UGACUUUGCAUUCCAGACCUG |
| 9 | GGCUGGGGCUGAGCUUUAA | 113 | UUAAAGCUCAGCCCCAGCCCU |
| 10 | GCAGGAACUGAGCCAGAAA | 114 | UUUCUGGCUCAGUUCCUGCUG |
| 11 | CCAAGCAAGCAGACAUUUA | 115 | UAAAUGUCUGCUUGCUUGGGU |
| 12 | CUGUCCUCUCUGUUGCCUU | 116 | AAGGCAACAGAGAGGACAGAC |
| 13 | UGUCCUCUCUGUUGCCUUU | 117 | AAAGGCAACAGAGAGGACAGA |
| 14 | GUCCUCUCUGUUGCCUUUU | 118 | AAAAGGCAACAGAGAGGACAG |
| 15 | UCCUCUCUGUUGCCUUUUU | 119 | AAAAAGGCAACAGAGAGGACA |
| 16 | CCUCUCUGUUGCCUUUUUA | 120 | UAAAAAGGCAACAGAGAGGAC |
| 17 | CUCUCUGUUGCCUUUUUAU | 121 | AUAAAAAGGCAACAGAGAGGA |
| 18 | UCUCUGUUGCCUUUUUACA | 122 | UGUAAAAAGGCAACAGAGAGG |
| 19 | CUCUGUUGCCUUUUUACAA | 123 | UUGUAAAAAGGCAACAGAGAG |
| 20 | UCUGUUGCCUUUUUACAGA | 124 | UCUGUAAAAAGGCAACAGAGA |
| 21 | CUGUUGCCUUUUUACAGCA | 125 | UGCUGUAAAAAGGCAACAGAG |
| 22 | UGUUGCCUUUUUACAGCCA | 126 | UGGCUGUAAAAAGGCAACAGA |
| 23 | GUUGCCUUUUUACAGCCAA | 127 | UUGGCUGUAAAAAGGCAACAG |
| 24 | UUGCCUUUUUACAGCCAAA | 128 | UUUGGCUGUAAAAAGGCAACA |
| 25 | ACUUUUCUAGACCUGUUUU | 129 | AAAACAGGUCUAGAAAAGUUG |
| 26 | CUUUUCUAGACCUGUUUUA | 130 | UAAAACAGGUCUAGAAAAGUU |
| 27 | UUUUCUAGACCUGUUUUGA | 131 | UCAAAACAGGUCUAGAAAAGU |
| 28 | UUUCUAGACCUGUUUUGCU | 132 | AGCAAAACAGGUCUAGAAAAG |
| 29 | UUCUAGACCUGUUUUGCUU | 133 | AAGCAAAACAGGUCUAGAAAA |
| 30 | UCUAGACCUGUUUUGCUUU | 134 | AAAGCAAAACAGGUCUAGAAA |
| 31 | CUAGACCUGUUUUGCUUUU | 135 | AAAAGCAAAACAGGUCUAGAA |
| 32 | UAGACCUGUUUUGCUUUUA | 136 | UAAAAGCAAAACAGGUCUAGA |
| 33 | AGACCUGUUUUGCUUUUGU | 137 | ACAAAAGCAAAACAGGUCUAG |
| 34 | GACCUGUUUUGCUUUUGUA | 138 | UACAAAAGCAAAACAGGUCUA |
| 35 | ACCUGUUUUGCUUUUGUAA | 139 | UUACAAAAGCAAAACAGGUCU |
| 36 | CCUGUUUUGCUUUUGUAAA | 140 | UUUACAAAAGCAAAACAGGUC |
| 37 | CUGUUUUGCUUUUGUAACU | 141 | AGUUACAAAAGCAAAACAGGU |
| 38 | UGUUUUGCUUUUGUAACUU | 142 | AAGUUACAAAAGCAAAACAGG |
| 39 | GUUUUGCUUUUGUAACUUA | 143 | UAAGUUACAAAAGCAAAACAG |
| 40 | UUUUGCUUUUGUAACUUGA | 144 | UCAAGUUACAAAAGCAAAACA |
| 41 | UUUGCUUUUGUAACUUGAA | 145 | UUCAAGUUACAAAAGCAAAAC |
| 42 | UUGCUUUUGUAACUUGAAA | 146 | UUUCAAGUUACAAAAGCAAAA |
| 43 | UGCUUUUGUAACUUGAAGA | 147 | UCUUCAAGUUACAAAAGCAAA |
| 44 | GCUUUUGUAACUUGAAGAU | 148 | AUCUUCAAGUUACAAAAGCAA |
| 45 | CUUUUGUAACUUGAAGAUA | 149 | UAUCUUCAAGUUACAAAAGCA |
| 46 | UUUUGUAACUUGAAGAUAU | 150 | AUAUCUUCAAGUUACAAAAGC |
| 47 | UUUGUAACUUGAAGAUAUU | 151 | AAUAUCUUCAAGUUACAAAAG |
| 48 | UUGUAACUUGAAGAUAUUU | 152 | AAAUAUCUUCAAGUUACAAAA |
| 49 | UGUAACUUGAAGAUAUUUA | 153 | UAAAUAUCUUCAAGUUACAAA |
| 50 | GUAACUUGAAGAUAUUUAU | 154 | AUAAAUAUCUUCAAGUUACAA |
| 51 | UAACUUGAAGAUAUUUAUU | 155 | AAUAAAUAUCUUCAAGUUACA |
| 52 | AACUUGAAGAUAUUUAUUA | 156 | UAAUAAAUAUCUUCAAGUUAC |
| 53 | ACUUGAAGAUAUUUAUUCU | 157 | AGAAUAAAUAUCUUCAAGUUA |
| 54 | CUUGAAGAUAUUUAUUCUA | 158 | UAGAAUAAAUAUCUUCAAGUU |
| 55 | UUGAAGAUAUUUAUUCUGA | 159 | UCAGAAUAAAUAUCUUCAAGU |
| 56 | UGAAGAUAUUUAUUCUGGA | 160 | UCCAGAAUAAAUAUCUUCAAG |
| 57 | GAAGAUAUUUAUUCUGGGU | 161 | ACCCAGAAUAAAUAUCUUCAA |
| 58 | AAGAUAUUUAUUCUGGGUU | 162 | AACCCAGAAUAAAUAUCUUCA |
| 59 | AGAUAUUUAUUCUGGGUUU | 163 | AAACCCAGAAUAAAUAUCUUC |
| 60 | GAUAUUUAUUCUGGGUUUU | 164 | AAAACCCAGAAUAAAUAUCUU |
| 61 | AUAUUUAUUCUGGGUUUUA | 165 | UAAAACCCAGAAUAAAUAUCU |
| 62 | UAUUUAUUCUGGGUUUUGU | 166 | ACAAAACCCAGAAUAAAUAUC |
| 63 | AUUUAUUCUGGGUUUUGUA | 167 | UACAAAACCCAGAAUAAAUAU |
| 64 | UUUAUUCUGGGUUUUGUAA | 168 | UUACAAAACCCAGAAUAAAUA |
| 65 | UUAUUCUGGGUUUUGUAGA | 169 | UCUACAAAACCCAGAAUAAAU |
| 66 | UAUUCUGGGUUUUGUAGCA | 170 | UGCUACAAAACCCAGAAUAAA |
| 67 | AUUCUGGGUUUUGUAGCAU | 171 | AUGCUACAAAACCCAGAAUAA |
| 68 | UUCUGGGUUUUGUAGCAUU | 172 | AAUGCUACAAAACCCAGAAUA |
| 69 | UCUGGGUUUUGUAGCAUUU | 173 | AAAUGCUACAAAACCCAGAAU |
| 70 | CUGGGUUUUGUAGCAUUUU | 174 | AAAAUGCUACAAAACCCAGAA |
| 71 | UGGGUUUUGUAGCAUUUUU | 175 | AAAAAUGCUACAAAACCCAGA |
| 72 | GGGUUUUGUAGCAUUUUUA | 176 | UAAAAAUGCUACAAAACCCAG |
| 73 | GGUUUUGUAGCAUUUUUAU | 177 | AUAAAAAUGCUACAAAACCCA |
| 74 | GUUUUGUAGCAUUUUUAUU | 178 | AAUAAAAAUGCUACAAAACCC |
| 75 | UUUUGUAGCAUUUUUAUUA | 179 | UAAUAAAAAUGCUACAAAACC |
| 76 | UUUGUAGCAUUUUUAUUAA | 180 | UUAAUAAAAAUGCUACAAAAC |
| 77 | UUGUAGCAUUUUUAUUAAU | 181 | AUUAAUAAAAAUGCUACAAAA |
| 78 | UGUAGCAUUUUUAUUAAUA | 182 | UAUUAAUAAAAAUGCUACAAA |
| 79 | GUAGCAUUUUUAUUAAUAU | 183 | AUAUUAAUAAAAAUGCUACAA |
| 80 | UAGCAUUUUUAUUAAUAUA | 184 | UAUAUUAAUAAAAAUGCUACA |
| 81 | AGCAUUUUUAUUAAUAUGA | 185 | UCAUAUUAAUAAAAAUGCUAC |
| 82 | GGUGACUUUUUAAAAUAAA | 186 | UUUAUUUUAAAAAGUCACCAU |
| 83 | GUGACUUUUUAAAAUAAAA | 187 | UUUUAUUUUAAAAAGUCACCA |
| 84 | UGACUUUUUAAAAUAAAAA | 188 | UUUUUAUUUUAAAAAGUCACC |
| 85 | GACUUUUUAAAAUAAAAAU | 189 | AUUUUUAUUUUAAAAAGUCAC |
| 86 | ACUUUUUAAAAUAAAAACA | 190 | UGUUUUUAUUUUAAAAAGUCA |
| 87 | CUUUUUAAAAUAAAAACAA | 191 | UUGUUUUUAUUUUAAAAAGUC |
| 88 | UUUUUAAAAUAAAAACAAA | 192 | UUUGUUUUUAUUUUAAAAAGU |
| 89 | UUUUAAAAUAAAAACAAAU | 193 | AUUUGUUUUUAUUUUAAAAAG |
| 90 | UUUAAAAUAAAAACAAACA | 194 | UGUUUGUUUUUAUUUUAAAAA |
| 91 | CUAGACCUGUdTUUGCUUUUG U | 195 | ACAAAAGCAAAACAGGUCUAGA A |
| 92 | UGUUUUGCUUUUGUAACUU | 196 | AAGUUACAAAAGCAAAACAGG |
| 93 | GUUUUGCUUUUGUAACUUA | 197 | UAAGUUACAAAAGCAAAACAG |
| 94 | UUUUGCUUUUGUAACUUGA | 198 | UCAAGUUACAAAAGCAAAACA |
| 95 | UGCUUUUGUAACUUGAAGA | 199 | UCUUCAAGUUACAAAAGCAAA |
| 96 | GCUUUUGUAACUUGAAGAU | 200 | AUCUUCAAGUUACAAAAGCAA |
| 97 | CUUUUGUAACUUGAAGAUA | 201 | UAUCUUCAAGUUACAAAAGCA |
| 98 | UUUUGUAACUUGAAGAUAU | 202 | AUAUCUUCAAGUUACAAAAGC |
| 99 | CUAGACCUGUdTUUGCUUUUG U | 203 | ACAAAAGCAAAACAGGUCUAGA A |
| 100 | CUAGACCUGUdTUUGCUUUUG U | 204 | ACAAAAGCAAAACAGGUCUAGA A |
| 101 | GUUUUGCUUUUGUAACUUA | 205 | UAAGUUACAAAAGCAAAACAG |
| 102 | GUUUUGCUUUUGUAACUUA | 206 | UAAGUUACAAAAGCAAAACAG |
| 103 | UGUUUUGCUUUUGUAACUU | 207 | AAGUUACAAAAGCAAAACAGG |
| 104 | UGCUUUUGUAACUUGAAGA | 208 | UCUUCAAGUUACAAAAGCAAA |

**Table 4 shows modified RNA sequences used in the present invention.**

Herein, meanings of abbreviation are as follows:
A, U, G and C represents a natural adenine ribonucleotide, a uracil ribonucleotide, a guanine ribonucleotide, and a cytosine ribonucleotide, respectively.

d represents that the right side nucleotide is a deoxyribonucleotide. For example, dA, dT, dG and dC represent an adenine deoxyribonucleotide, a thymine deoxyribonucleotide, a guanine deoxyribonucleotide, and a cytosine deoxyribonucleotide, respectively.

i represents an inosine ribonucleotide.

m represents that the left side nucleotide is a 2'-OCH₃ modified nucleotide. For example, Am, Um, Gm and Cm represent 2'-OCH₃ modified A, U, G, and C.

f represents that the left side nucleotide is a 2'-F modified nucleotide. For example, Af, Uf, Gf and Cf represent 2'-F modified A, U, G, and C, respectively.

"s" or "s-" represents that the flanking two nucleotides and/or delivery vectors are connected by thiophosphate.

VP represents that the right side nucleotide is a vinyl phosphonate modified nucleotide, which is well known in the field, with reference to, for example, PCT publication No. WO2011139702, No. WO2013033230, and No. WO2019105419.

IB represents a reverse abasic deoxyribonucleotide, which can include the following three structures according to its position/connection mode in the siRNA. IB is well known in the field, with reference to, for example, F. Czauderna, Nucleic Acids Res., 2003, 31(11), 2705-16 and PCT publication No. WO2016011123 and No. WO2019051402,

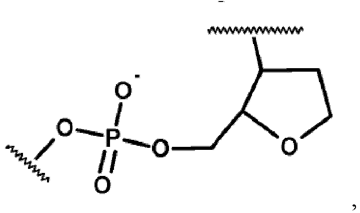

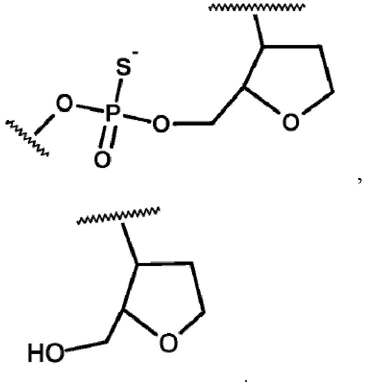

L96 represents a GalNAc delivery moiety having the following structure well known in the field, where 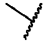 represents a position connected to the siRNA through a phosphate group or a thiophosphate group, with reference to, for example, PCT publication No. WO2009073809 and No. WO2009082607,

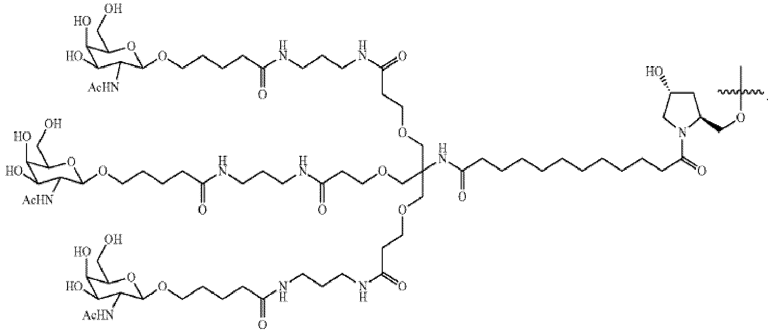

NAG37 represents a GalNAc delivery moiety having the following structure well known in the field, where 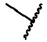 represents a position connected to the siRNA through a phosphate group or a thiophosphate group, with reference to, for example, PCT publication No. WO2018044350,

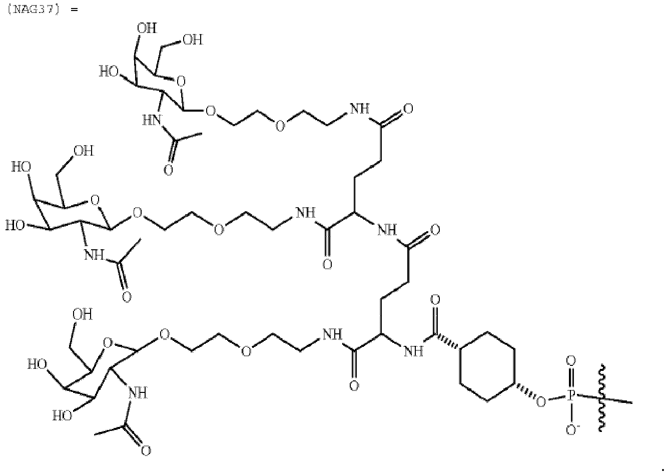

GL6 represents a GalNAc delivery moiety having the following structure, where 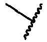 represents a position connected to the siRNA through a phosphate group or a thiophosphate group,

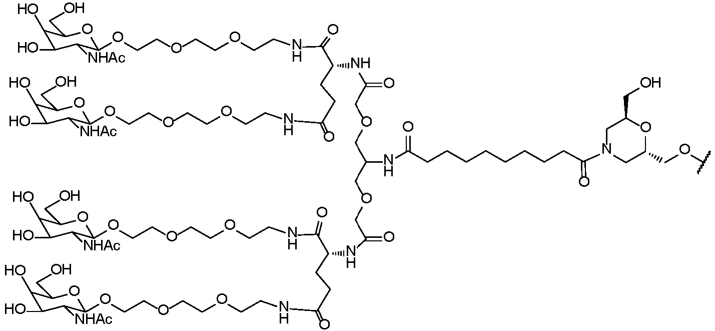

GL12 represents a GalNAc delivery moiety having the following structure, where 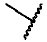 represents a position connected to the siRNA through a phosphate group or a thiophosphate group,

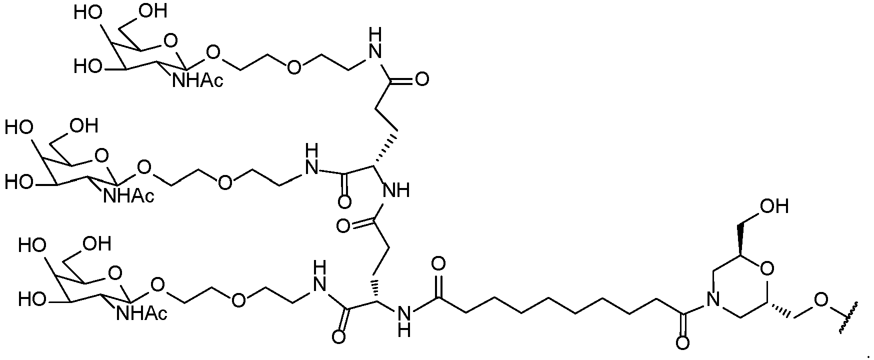

**Table 4 Modified RNA sequences of the sense strand of the siRNA targeting PCSK9 gene as used in the present invention**

| Single strand number | Sequence (5' → 3') | SEQ ID NO: |
|---|---|---|
| SR004482S | CmsCmsUmAmCmGmUfGfGfUmGmGmUmGmCmUmGmsAmsAm | 209 |
| SR004483S | GmsGmsUmGmGmAmGfGfUfGmUmAmUmCmUmCmCmsUmsAm | 210 |
| SR004484S | GmsGmsGmUmCmAmUfGfGfUmCmAmCmCmGmAmCmsUmsUm | 211 |
| SR004485S | CmsCmsUmGmGmAmGfUfUfUmAmUmUmCmGmGmAmsAmsAm | 212 |
| SR004486S | GmsCmsAmCmCmUmGfCfUfUmUmGmUmGmUmCmAmsCmsAm | 213 |
| SR004487S | CmsCmsAmAmAmGmAfUfGfUmCmAmUmCmAmAmUmsGmsAm | 214 |
| SR004488S | GmsCmsUmGmUmUmUfUfGfCmAmGmGmAmCmUmGmsUmsAm | 215 |
| SR004489S | GmsGmsUmCmUmGmGfAfAfUmGmCmAmAmAmGmUmsCmsAm | 216 |
| SR004490S | GmsGmsCmUmGmGmGfGfCfUmGmAmGmCmUmUmUmsAmsAm | 217 |
| SR004491S | GmsCmsAmGmGmAmAfCfUfGmAmGmCmCmAmGmAmsAmsAm | 218 |
| SR004492S | CmsCmsAmAmGmCmAfAfGfCmAmGmAmCmAmUmUmsUmsAm | 219 |
| SR004493S | CmsUmsGmUmCmCmUfCfUfCmUmGmUmUmGmCmCmsUmsUm | 220 |
| SR004494S | UmsGmsUmCmCmUmCfUfCfUmGmUmUmGmCmCmUmsUmsUm | 221 |
| SR004495S | GmsUmsCmCmUmCmUfCfUfGmUmUmGmCmCmUmUmsUmsUm | 222 |
| SR004496S | UmsCmsCmUmCmUmCfUfGfUmUmGmCmCmUmUmUmsUmsUm | 223 |
| SR004497S | CmsCmsUmCmUmCmUfGfUfUmGmCmCmUmUmUmUmsUmsAm | 224 |
| SR004498S | CmsUmsCmUmCmUmGfUfUfGmCmCmUmUmUmUmUmsAmsUm | 225 |
| SR004499S | UmsCmsUmCmUmGmUfUfGfCmCmUmUmUmUmUmAmsCmsAm | 226 |
| SR004500S | CmsUmsCmUmGmUmUfGfCfCmUmUmUmUmUmAmCmsAmsAm | 227 |
| SR004501S | UmsCmsUmGmUmUmGfCfCfUmUmUmUmUmAmCmAmsGmsAm | 228 |
| SR004502S | CmsUmsGmUmUmGmCfCfUfUmUmUmUmAmCmAmGmsCmsAm | 229 |
| SR004503S | UmsGmsUmUmGmCmCfLTfLTfLTmUmUmAmCmAmGmCmsCmsAm | 230 |
| SR004504S | GmsUmsUmGmCmCmUfUfUfUmUmAmCmAmGmCmCmsAmsAm | 231 |
| SR004505S | UmsUmsGmCmCmUmUfUfUfUmAmCmAmGmCmCmAmsAmsAm | 232 |
| SR004506S | AmsCmsUmUmUmUmCfUfAfGmAmCmCmUmGmUmUmsUmsUm | 233 |
| SR004507S | CmsUmsUmUmUmCmUfAfGfAmCmCmUmGmUmUmUmsUmsAm | 234 |
| SR004508S | UmsUmsUmUmCmUmAfGfAfCmCmUmGmUmUmUmUmsGmsAm | 235 |
| SR004509S | UmsUmsUmCmUmAmGfAfCfCmUmGmUmUmUmUmGmsCmsUm | 236 |
| SR004510S | UmsUmsCmUmAmGmAfCfCfUmGmUmUmUmUmGmCmsUmsUm | 237 |
| SR004511S | UmsCmsUmAmGmAmCfCfUfGmUmUmUmUmGmCmUmsUmsUm | 238 |
| SR004512S | CmsUmsAmGmAmCmCfUfGfUmUmUmUmGmCmUmUmsUmsUm | 239 |
| SR004513S | UmsAmsGmAmCmCmUfGfUfUmUmUmGmCmUmUmUmsUmsAm | 240 |
| SR004514S | AmsGmsAmCmCmUmGfUfUfUmUmGmCmUmUmUmUmsGmsUm | 241 |
| SR004515S | GmsAmsCmCmUmGmUfUfUfUmGmCmUmUmUmUmGmsUmsAm | 242 |
| SR004516S | AmsCmsCmUmGmUmUfUfUfGmCmUmUmUmUmGmUmsAmsAm | 243 |
| SR004517S | CmsCmsUmGmUmUmUfUfGfCmUmUmUmUmGmUmAmsAmsAm | 244 |
| SR004518S | CmsUmsGmUmUmUmUfGfCfUmUmUmUmGmUmAmAmsCmsUm | 245 |
| SR004519S | UmsGmsUmUmUmUmGfCfUfUmUmUmGmUmAmAmCmsUmsUm | 246 |
| SR004520S | GmsUmsUmUmUmGmCfUfUfUmUmGmUmAmAmCmUmsUmsAm | 247 |
| SR004521S | UmsUmsUmUmGmCmUfUfUfUmGmUmAmAmCmUmUmsGmsAm | 248 |
| SR004522S | UmsUmsUmGmCmUmUfUfUfGmUmAmAmCmUmUmGmsAmsAm | 249 |
| SR004523S | UmsUmsGmCmUmUmUfUfGfUmAmAmCmUmUmGmAmsAmsAm | 250 |
| SR004524S | UmsGmsCmUmUmUmUfGfUfAmAmCmUmUmGmAmAmsGmsAm | 251 |
| SR004525S | GmsCmsUmUmUmUmGfUfAfAmCmUmUmGmAmAmGmsAmsUm | 252 |
| SR004526S | CmsUmsUmUmUmGmUfAfAfCmUmUmGmAmAmGmAmsUmsAm | 253 |
| SR004527S | UmsUmsUmUmGmUmAfAfCfUmUmGmAmAmGmAmUmsAmsUm | 254 |
| SR004528S | UmsUmsUmGmUmAmAfCfUfUmGmAmAmGmAmUmAmsUmsUm | 255 |
| SR004529S | UmsUmsGmUmAmAmCfUfUfGmAmAmGmAmUmAmUmsUmsUm | 256 |
| SR004530S | UmsGmsUmAmAmCmUfUfGfAmAmGmAmUmAmUmUmsUmsAm | 257 |
| SR004531S | GmsUmsAmAmCmUmUfGfAfAmGmAmUmAmUmUmUmsAmsUm | 258 |
| SR004532S | UmsAmsAmCmUmUmGfAfAfGmAmUmAmUmUmUmAmsUmsUm | 259 |
| SR004533S | AmsAmsCmUmUmGmAfAfGfAmUmAmUmUmUmAmUmsUmsAm | 260 |
| SR004534S | AmsCmsUmUmGmAmAfGfAfUmAmUmUmUmAmUmUmsCmsUm | 261 |
| SR004535S | CmsUmsUmGmAmAmGfAfUfAmUmUmUmAmUmUmCmsUmsAm | 262 |
| SR004536S | UmsUmsGmAmAmGmAfUfAfUmUmUmAmUmUmCmUmsGmsAm | 263 |
| SR004537S | UmsGmsAmAmGmAmUfAfUfUmUmAmUmUmCmUmGmsGmsAm | 264 |
| SR004538S | GmsAmsAmGmAmUmAfUfUfUmAmUmUmCmUmGmGmsGmsUm | 265 |
| SR004539S | AmsAmsGmAmUmAmUfUfUfAmUmUmCmUmGmGmGmsUmsUm | 266 |
| SR004540S | AmsGmsAmUmAmUmUfUfAfUmUmCmUmGmGmGmUmsUmsUm | 267 |
| SR0045415 | GmsAmsUmAmUmUmUfAfUfUmCmUmGmGmGmUmUmsUmsUm | 268 |
| SR004542S | AmsUmsAmUmUmUmAfUfUfCmUmGmGmGmUmUmUmsUmsAm | 269 |
| SR004543S | UmsAmsUmUmUmAmUfUfCfUmGmGmGmUmUmUmUmsGmsUm | 270 |
| SR004544S | AmsUmsUmUmAmUmUfCfUfGmGmGmUmUmUmUmGmsUmsAm | 271 |
| SR004545S | UmsUmsUmAmUmUmCfUfGfGmGmUmUmUmUmGmUmsAmsAm | 272 |
| SR004546S | UmsUmsAmUmUmCmUfGfGfGmUmUmUmUmGmUmAmsGmsAm | 273 |
| SR004547S | UmsAmsUmUmCmUmGfGfGfUmUmUmUmGmUmAmGmsCmsAm | 274 |
| SR004548S | AmsUmsUmCmUmGmGfGfUfUmUmUmGmUmAmGmCmsAmsUm | 275 |
| SR004549S | UmsUmsCmUmGmGmGfUfUfUmUmGmUmAmGmCmAmsUmsUm | 276 |
| SR004550S | UmsCmsUmGmGmGmUfUfUfUmGmUmAmGmCmAmUmsUmsUm | 277 |
| SR004551S | CmsUmsGmGmGmUmUfUfUfGmUmAmGmCmAmUmUmsUmsUm | 278 |
| SR004552S | UmsGmsGmGmUmUmUfUfGfUmAmGmCmAmUmUmUmsUmsUm | 279 |
| SR004553S | GmsGmsGmUmUmUmUfGfUfAmGmCmAmUmUmUmUmsUmsAm | 280 |
| SR004554S | GmsGmsUmUmUmUmGfUfAfGmCmAmUmUmUmUmUmsAmsUm | 281 |
| SR004555S | GmsUmsUmUmUmGmUfAfGfCmAmUmUmUmUmUmAmsUmsUm | 282 |
| SR004556S | UmsUmsUmUmGmUmAfGfCfAmUmUmUmUmUmAmUmsUmsAm | 283 |
| SR004557S | UmsUmsUmGmUmAmGfCfAfUmUmUmUmUmAmUmUmsAmsAm | 284 |
| SR004558S | UmsUmsGmUmAmGmCfAfUfUmUmUmUmAmUmUmAmsAmsUm | 285 |
| SR004559S | UmsGmsUmAmGmCmAfUfUfUmUmUmAmUmUmAmAmsUmsAm | 286 |
| SR004560S | GmsUmsAmGmCmAmUfUfUfUmUmAmUmUmAmAmUmsAmsUm | 287 |
| SR004561S | UmsAmsGmCmAmUmUfUfUfUmAmUmUmAmAmUmAmsUmsAm | 288 |
| SR004562S | AmsGmsCmAmUmUmUfUfUfAmUmUmAmAmUmAmUmsGmsAm | 289 |
| SR004563S | GmsGmsUmGmAmCmUfUfUfUmUmAmAmAmAmUmAmsAmsAm | 290 |
| SR004564S | GmsUmsGmAmCmUmUfUfUfUmAmAmAmAmUmAmAmsAmsAm | 291 |
| SR004565S | UmsGmsAmCmUmUmUfUfUfAmAmAmAmUmAmAmAmsAmsAm | 292 |
| SR004566S | GmsAmsCmUmUmUmUfUfAfAmAmAmUmAmAmAmAmsAmsUm | 293 |
| SR004567S | AmsCmsUmUmUmUmUfAfAfAmAmUmAmAmAmAmAmsCmsAm | 294 |
| SR004568S | CmsUmsUmUmUmUmAfAfAfAmUmAmAmAmAmAmCmsAmsAm | 295 |
| SR004569S | UmsUmsUmUmUmAmAfAfAfUmAmAmAmAmAmCmAmsAmsAm | 296 |
| SR004570S | UmsUmsUmUmAmAmAfAfUfAmAmAmAmAmCmAmAmsAmsUm | 297 |
| SR004571S | UmsUmsUmAmAmAmAfUfAfAmAmAmAmCmAmAmAmsCmsAm | 298 |
| SR004440S | CmsUmsAmGmAmCmCfUmGfUmdTUmUmGmCmUmUmUmUmGmUm -L96 | 299 |
| SR004676S | UmsGmsUmUmUmUmGfCfUfUmUmUmGmUmAmAmCmsUmsUm-L96 | 300 |
| SR004677S | GmsUmsUmUmUmGmCfUfUfUmUmGmUmAmAmCmUmsUmsAm-L96 | 301 |
| SR004678S | UmsUmsUmUmGmCmUfUfUfUmGmUmAmAmCmUmUmsGmsAm-L96 | 302 |
| SR004679S | UmsGmsCmUmUmUmUfGfUfAmAmCmUmUmGmAmAmsGmsAm-L96 | 303 |
| SR004680S | GmsCmsUmUmUmUmGfUfAfAmCmUmUmGmAmAmGmsAmsUm-L96 | 304 |
| SR004681S | CmsUmsUmUmUmGmUfAfAfCmUmUmGmAmAmGmAmsUmsAm-L96 | 305 |
| SR004682S | UmsUmsUmUmGmUmAfAfCfUmUmGmAmAmGmAmUmsAmsUm-L96 | 306 |
| SR011225S | CmsUmsAmGmAmCmCfUmGfUmdTUmUmGmCmUmUmUmUmGmsU ms-GL12 | 307 |
| SR011239S | CmsUmsAmGmAmCmCfUmGfUmdTUmUmGmCmUmUmUmUmGmsU ms-GL6 | 308 |
| SR011269S | GmsUmsUmUmUmGmCfUfUfUmUmGmUmAmAmCmUmUmsAms-GL6 | 309 |
| SR011272S | GmsUmsUmUmUmGmCfUfUfUmUmGmUmAmAmCmUmUmAm-GL6 | 310 |
| SR011271S | UmsGmsUmUmUmUmGfCfUfUmUmUmGmUmAmAmCmUmsUms-GL6 | 311 |
| SR011273S | UmsGmsCmUmUmUmUfGfUfAmAmCmUmUmGmAmAmsGmsAm-GL6 | 312 |

**Table 5 Modified RNA sequences of the antisense strand of the siRNA targeting PCSK9 gene as used in the present invention**

| Single strand number | Sequence (5' → 3') | SEQ ID NO: |
|---|---|---|
| SR004572A | UmsUfsCmAfGmCfAmCfCmAfCmCfAmCfGmUfAmGfGmsUfsGm | 313 |
| SR004573A | UmsAfsGmGfAmGfAmUfAmCfAmCfCmUfCmCfAmCfCmsAfsGm | 314 |
| SR004574A | AmsAfsGmUfCmGfGmUfGmAfCmCfAmUfGmAfCmCfCmsUfsGm | 315 |
| SR004575A | UmsUfsUmCfCmGfAmAfUmAfAmAfCmUfCmCfAmGfGmsCfsCm | 316 |
| SR004576A | UmsGfsUmGfAmCfAmCfAmAfAmGfCmAfGmGfUmGfCmsUfsGm | 317 |
| SR004577A | UmsCfsAmUfUmGfAmUfGmAfCmAfUmCfUmUfUmGfGmsCfsAm | 318 |
| SR004578A | UmsAfsCmAfGmUfCmCfUmGfCmAfAmAfAmCfAmGfCmsUfsGm | 319 |
| SR004579A | UmsGfsAmCfUmUfUmGfCmAfUmUfCmCfAmGfAmCfCmsUfsGm | 320 |
| SR004580A | UmsUfsAmAfAmGfCmUfCmAfGmCfCmCfCmAfGmCfCmsCfsUm | 321 |
| SR004581A | UmsUfsUmCfUmGfGmCfUmCfAmGfUmUfCmCfUmGfCmsUfsGm | 322 |
| SR004582A | UmsAfsAmAfUmGfUmCfUmGfCmUfUmGfCmUfUmGfGmsGfsUm | 323 |
| SR004583A | AmsAfsGmGfCmAfAmCfAmGfAmGfAmGfGmAfCmAfGmsAfsCm | 324 |
| SR004584A | AmsAfsAmGfGmCfAmAfCmAfGmAfGmAfGmGfAmCfAmsGfsAm | 325 |
| SR004585A | AmsAfsAmAfGmGfCmAfAmCfAmGfAmGfAmGfGmAfCmsAfsGm | 326 |
| SR004586A | AmsAfsAmAfAmGfGmCfAmAfCmAfGmAfGmAfGmGfAmsCfsAm | 327 |
| SR004587A | UmsAfsAmAfAmAfGmGfCmAfAmCfAmGfAmGfAmGfGmsAfsCm | 328 |
| SR004588A | AmsUfsAmAfAmAfAmGfGmCfAmAfCmAfGmAfGmAfGmsGfsAm | 329 |
| SR004589A | UmsGfsUmAfAmAfAmAfGmGfCmAfAmCfAmGfAmGfAmsGfsGm | 330 |
| SR004590A | UmsUfsGmUfAmAfAmAfAmGfGmCfAmAfCmAfGmAfGmsAfsGm | 331 |
| SR004591A | UmsCfsUmGfUmAfAmAfAmAfGmGfCmAfAmCfAmGfAmsGfsAm | 332 |
| SR004592A | UmsGfsCmUfGmUfAmAfAmAfAmGfGmCfAmAfCmAfGmsAfsGm | 333 |
| SR004593A | UmsGfsGmCfUmGfUmAfAmAfAmAfGmGfCmAfAmCfAmsGfsAm | 334 |
| SR004594A | UmsUfsGmGfCmUfGmUfAmAfAmAfAmGfGmCfAmAfCmsAfsGm | 335 |
| SR004595A | UmsUfsUmGfGmCfUmGfUmAfAmAfAmAfGmGfCmAfAmsCfsAm | 336 |
| SR004596A | AmsAfsAmAfCmAfGmGfUmCfUmAfGmAfAmAfAmGfUmsUfsGm | 337 |
| SR004597A | UmsAfsAmAfAmCfAmGfGmUfCmUfAmGfAmAfAmAfGmsUfsUm | 338 |
| SR004598A | UmsCfsAmAfAmAfCmAfGmGfUmCfUmAfGmAfAmAfAmsGfsUm | 339 |
| SR004599A | AmsGfsCmAfAmAfAmCfAmGfGmUfCmUfAmGfAmAfAmsAfsGm | 340 |
| SR004600A | AmsAfsGmCfAmAfAmAfCmAfGmGfUmCfUmAfGmAfAmsAfsAm | 341 |
| SR004601A | AmsAfsAmGfCmAfAmAfAmCfAmGfGmUfCmUfAmGfAmsAfsAm | 342 |
| SR004602A | AmsAfsAmAfGmCfAmAfAmAfCmAfGmGfUmCfUmAfGmsAfsAm | 343 |
| SR004603A | UmsAfsAmAfAmGfCmAfAmAfAmCfAmGfGmUfCmUfAmsGfsAm | 344 |
| SR004604A | AmsCfsAmAfAmAfGmCfAmAfAmAfCmAfGmGfUmCfUmsAfsGm | 345 |
| SR004605A | UmsAfsCmAfAmAfAmGfCmAfAmAfAmCfAmGfGmUfCmsUfsAm | 346 |
| SR004606A | UmsUfsAmCfAmAfAmAfGmCfAmAfAmAfCmAfGmGfUmsCfsUm | 347 |
| SR004607A | UmsUfsUmAfCmAfAmAfAmGfCmAfAmAfAmCfAmGfGmsUfsCm | 348 |
| SR004608A | AmsGfsUmUfAmCfAmAfAmAfGmCfAmAfAmAfCmAfGmsGfsUm | 349 |
| SR004609A | AmsAfsGmUfUmAfCmAfAmAfAmGfCmAfAmAfAmCfAmsGfsGm | 350 |
| SR004610A | UmsAfsAmGfUmUfAmCfAmAfAmAfGmCfAmAfAmAfCmsAfsGm | 351 |
| SR004611A | UmsCfsAmAfGmUfUmAfCmAfAmAfAmGfCmAfAmAfAmsCfsAm | 352 |
| SR004612A | UmsUfsCmAfAmGfUmUfAmCfAmAfAmAfGmCfAmAfAmsAfsCm | 353 |
| SR004613A | UmsUfsUmCfAmAfGmUfUmAfCmAfAmAfAmGfCmAfAmsAfsAm | 354 |
| SR004614A | UmsCfsUmUfCmAfAmGfUmUfAmCfAmAfAmAfGmCfAmsAfsAm | 355 |
| SR004615A | AmsUfsCmUfUmCfAmAfGmUfUmAfCmAfAmAfAmGfCmsAfsAm | 356 |
| SR004616A | UmsAfsUmCfUmUfCmAfAmGfUmUfAmCfAmAfAmAfGmsCfsAm | 357 |
| SR004617A | AmsUfsAmUfCmUfUmCfAmAfGmUfUmAfCmAfAmAfAmsGfsCm | 358 |
| SR004618A | AmsAfsUmAfUmCfUmUfCmAfAmGfUmUfAmCfAmAfAmsAfsGm | 359 |
| SR004619A | AmsAfsAmUfAmUfCmUfUmCfAmAfGmUfUmAfCmAfAmsAfsAm | 360 |
| SR004620A | UmsAfsAmAfUmAfUmCfUmUfCmAfAmGfUmUfAmCfAmsAfsAm | 361 |
| SR004621A | AmsUfsAmAfAmUfAmUfCmUfUmCfAmAfGmUfUmAfCmsAfsAm | 362 |
| SR004622A | AmsAfsUmAfAmAfUmAfUmCfUmUfCmAfAmGfUmUfAmsCfsAm | 363 |
| SR004623A | UmsAfsAmUfAmAfAmUfAmUfCmUfUmCfAmAfGmUfUmsAfsCm | 364 |
| SR004624A | AmsGfsAmAfUmAfAmAfUmAfUmCfUmUfCmAfAmGfUmsUfsAm | 365 |
| SR004625A | UmsAfsGmAfAmUfAmAfAmUfAmUfCmUfUmCfAmAfGmsUfsUm | 366 |
| SR004626A | UmsCfsAmGfAmAfUmAfAmAfUmAfUmCfUmUfCmAfAmsGfsUm | 367 |
| SR004627A | UmsCfsCmAfGmAfAmUfAmAfAmUfAmUfCmUfUmCfAmsAfsGm | 368 |
| SR004628A | AmsCfsCmCfAmGfAmAfUmAfAmAfUmAfUmCfUmUfCmsAfsAm | 369 |
| SR004629A | AmsAfsCmCfCmAfGmAfAmUfAmAfAmUfAmUfCmUfUmsCfsAm | 370 |
| SR004630A | AmsAfsAmCfCmCfAmGfAmAfUmAfAmAfUmAfUmCfUmsUfsCm | 371 |
| SR004631A | AmsAfsAmAfCmCfCmAfGmAfAmUfAmAfAmUfAmUfCmsUfsUm | 372 |
| SR004632A | UmsAfsAmAfAmCfCmCfAmGfAmAfUmAfAmAfUmAfUmsCfsUm | 373 |
| SR004633A | AmsCfsAmAfAmAfCmCfCmAfGmAfAmUfAmAfAmUfAmsUfsCm | 374 |
| SR004634A | UmsAfsCmAfAmAfAmCfCmCfAmGfAmAfUmAfAmAfUmsAfsUm | 375 |
| SR004635A | UmsUfsAmCfAmAfAmAfCmCfCmAfGmAfAmUfAmAfAmsUfsAm | 376 |
| SR004636A | UmsCfsUmAfCmAfAmAfAmCfCmCfAmGfAmAfUmAfAmsAfsUm | 377 |
| SR004637A | UmsGfsCmUfAmCfAmAfAmAfCmCfCmAfGmAfAmUfAmsAfsAm | 378 |
| SR004638A | AmsUfsGmCfUmAfCmAfAmAfAmCfCmCfAmGfAmAfUmsAfsAm | 379 |
| SR004639A | AmsAfsUmGfCmUfAmCfAmAfAmAfCmCfCmAfGmAfAmsUfsAm | 380 |
| SR004640A | AmsAfsAmUfGmCfUmAfCmAfAmAfAmCfCmCfAmGfAmsAfsUm | 381 |
| SR004641A | AmsAfsAmAfUmGfCmUfAmCfAmAfAmAfCmCfCmAfGmsAfsAm | 382 |
| SR004642A | AmsAfsAmAfAmUfGmCfUmAfCmAfAmAfAmCfCmCfAmsGfsAm | 383 |
| SR004643A | UmsAfsAmAfAmAfUmGfCmUfAmCfAmAfAmAfCmCfCmsAfsGm | 384 |
| SR004644A | AmsUfsAmAfAmAfAmUfGmCfUmAfCmAfAmAfAmCfCmsCfsAm | 385 |
| SR004645A | AmsAfsUmAfAmAfAmAfUmGfCmUfAmCfAmAfAmAfCmsCfsCm | 386 |
| SR004646A | UmsAfsAmUfAmAfAmAfAmUfGmCfUmAfCmAfAmAfAmsCfsCm | 387 |
| SR004647A | UmsUfsAmAfUmAfAmAfAmAfUmGfCmUfAmCfAmAfAmsAfsCm | 388 |
| SR004648A | AmsUfsUmAfAmUfAmAfAmAfAmUfGmCfUmAfCmAfAmsAfsAm | 389 |
| SR004649A | UmsAfsUmUfAmAfUmAfAmAfAmAfUmGfCmUfAmCfAmsAfsAm | 390 |
| SR004650A | AmsUfsAmUfUmAfAmUfAmAfAmAfAmUfGmCfUmAfCmsAfsAm | 391 |
| SR004651A | UmsAfsUmAfUmUfAmAfUmAfAmAfAmAfUmGfCmUfAmsCfsAm | 392 |
| SR004652A | UmsCfsAmUfAmUfUmAfAmUfAmAfAmAfAmUfGmCfUmsAfsCm | 393 |
| SR004653A | UmsUfsUmAfUmUfUmUfAmAfAmAfAmGfUmCfAmCfCmsAfsUm | 394 |
| SR004654A | UmsUfsUmUfAmUfUmUfUmAfAmAfAmAfGmUfCmAfCmsCfsAm | 395 |
| SR004655A | UmsUfsUmUfUmAfUmUfUmUfAmAfAmAfAmGfUmCfAmsCfsCm | 396 |
| SR004656A | AmsUfsUmUfUmUfAmUfUmUfUmAfAmAfAmAfGmUfCmsAfsCm | 397 |
| SR004657A | UmsGfsUmUfUmUfUmAfUmUfUmUfAmAfAmAfAmGfUmsCfsAm | 398 |
| SR004658A | UmsUfsGmUfUmUfUmUfAmUfUmUfUmAfAmAfAmAfGmsUfsCm | 399 |
| SR004659A | UmsUfsUmGfUmUfUmUfUmAfUmUfUmUfAmAfAmAfAmsGfsUm | 400 |
| SR004660A | AmsUfsUmUfGmUfUmUfUmUfAmUfUmUfUmAfAmAfAmsAfsGm | 401 |
| SR004661A | UmsGfsUmUfUmGfUmUfUmUfUmAfUmUfUmUfAmAfAmsAfsAm | 402 |
| SR004442A | AmsCfsAmAfAfAfGmCfAmAfAmAfCmAfGmGfUmCfUmAmGmsAm sAm | 403 |
| SR004609A | AmsAfsGmUfUmAfCmAfAmAfAmGfCmAfAmAfAmCfAmsGfsGm | 404 |
| SR004610A | UmsAfsAmGfUmUfAmCfAmAfAmAfGmCfAmAfAmAfCmsAfsGm | 405 |
| SR004611A | UmsCfsAmAfGmUfUmAfCmAfAmAfAmGfCmAfAmAfAmsCfsAm | 406 |
| SR004614A | UmsCfsUmUfCmAfAmGfUmUfAmCfAmAfAmAfGmCfAmsAfsAm | 407 |
| SR004615A | AmsUfsCmUfUmCfAmAfGmUfUmAfCmAfAmAfAmGfCmsAfsAm | 408 |
| SR004616A | UmsAfsUmCfUmUfCmAfAmGfUmUfAmCfAmAfAmAfGmsCfsAm | 409 |
| SR004617A | AmsUfsAmUfCmUfUmCfAmAfGmUfUmAfCmAfAmAfAmsGfsCm | 410 |
| SR004442A | AmsCfsAmAfAfAfGmCfAmAfAmAfCmAfGmGfUmCfUmAmGmsAm sAm | 411 |
| SR004442A | AmsCfsAmAfAfAfGmCfAmAfAmAfCmAfGmGfUmCfUmAmGmsAm sAm | 412 |
| SR004610A | UmsAfsAmGfUmUfAmCfAmAfAmAfGmCfAmAfAmAfCmsAfsGm | 413 |
| SR004610A | UmsAfsAmGfUmUfAmCfAmAfAmAfGmCfAmAfAmAfCmsAfsGm | 414 |
| SR004609A | AmsAfsGmUfUmAfCmAfAmAfAmGfCmAfAmAfAmCfAmsGfsGm | 415 |
| SR004614A | UmsCfsUmUfCmAfAmGfUmUfAmCfAmAfAmAfGmCfAmsAfsAm | 416 |

**Table 6 Paired modified sense strands and modified antisense strands of the siRNA targeting PCSK9 gene as used in the present invention**

| Double strand number | Sense strand number | Antisense strand number |
|---|---|---|
| DR002254 | SR004482S | SR004572A |
| DR002255 | SR004483 S | SR004573A |
| DR002256 | SR004484S | SR004574A |
| DR002257 | SR004485S | SR004575A |
| DR002258 | SR004486S | SR004576A |
| DR002259 | SR004487S | SR004577A |
| DR002260 | SR004488S | SR004578A |
| DR002261 | SR004489S | SR004579A |
| DR002262 | SR004490S | SR004580A |
| DR002263 | SR004491S | SR004581A |
| DR002264 | SR004492S | SR004582A |
| DR002265 | SR004493S | SR004583A |
| DR002266 | SR004494S | SR004584A |
| DR002267 | SR004495S | SR004585A |
| DR002268 | SR004496S | SR004586A |
| DR002269 | SR004497S | SR004587A |
| DR002270 | SR004498S | SR004588A |
| DR002271 | SR004499S | SR004589A |
| DR002272 | SR004500S | SR004590A |
| DR002273 | SR004501S | SR004591A |
| DR002274 | SR004502S | SR004592A |
| DR002275 | SR004503S | SR004593A |
| DR002276 | SR004504S | SR004594A |
| DR002277 | SR004505S | SR004595A |
| DR002278 | SR004506S | SR004596A |
| DR002279 | SR004507S | SR004597A |
| DR002280 | SR004508S | SR004598A |
| DR002281 | SR004509S | SR004599A |
| DR002282 | SR004510S | SR004600A |
| DR002283 | SR004511S | SR004601A |
| DR002284 | SR004512S | SR004602A |
| DR002285 | SR004513S | SR004603A |
| DR002286 | SR004514S | SR004604A |
| DR002287 | SR004515S | SR004605A |
| DR002288 | SR004516S | SR004606A |
| DR002289 | SR004517S | SR004607A |
| DR002290 | SR004518S | SR004608A |
| DR002291 | SR004519S | SR004609A |
| DR002292 | SR004520S | SR004610A |
| DR002293 | SR004521S | SR004611A |
| DR002294 | SR004522S | SR004612A |
| DR002295 | SR004523 S | SR004613A |
| DR002296 | SR004524S | SR004614A |
| DR002297 | SR004525S | SR004615A |
| DR002298 | SR004526S | SR004616A |
| DR002299 | SR004527S | SR004617A |
| DR002300 | SR004528S | SR004618A |
| DR002301 | SR004529S | SR004619A |
| DR002302 | SR004530S | SR004620A |
| DR002303 | SR004531S | SR004621A |
| DR002304 | SR004532S | SR004622A |
| DR002305 | SR004533S | SR004623A |
| DR002306 | SR004534S | SR004624A |
| DR002307 | SR004535S | SR004625A |
| DR002308 | SR004536S | SR004626A |
| DR002309 | SR004537S | SR004627A |
| DR002310 | SR004538S | SR004628A |
| DR002311 | SR004539S | SR004629A |
| DR002312 | SR004540S | SR004630A |
| DR002313 | SR004541S | SR004631A |
| DR002314 | SR004542S | SR004632A |
| DR002315 | SR004543S | SR004633A |
| DR002316 | SR004544S | SR004634A |
| DR002317 | SR004545S | SR004635A |
| DR002318 | SR004546S | SR004636A |
| DR002319 | SR004547S | SR004637A |
| DR002320 | SR004548S | SR004638A |
| DR002321 | SR004549S | SR004639A |
| DR002322 | SR004550S | SR004640A |
| DR002323 | SR004551S | SR004641A |
| DR002324 | SR004552S | SR004642A |
| DR002325 | SR004553S | SR004643A |
| DR002326 | SR004554S | SR004644A |
| DR002327 | SR004555S | SR004645A |
| DR002328 | SR004556S | SR004646A |
| DR002329 | SR004557S | SR004647A |
| DR002330 | SR004558S | SR004648A |
| DR002331 | SR004559S | SR004649A |
| DR002332 | SR004560S | SR004650A |
| DR002333 | SR004561S | SR004651A |
| DR002334 | SR004562S | SR004652A |
| DR002335 | SR004563S | SR004653A |
| DR002336 | SR004564S | SR004654A |
| DR002337 | SR004565S | SR004655A |
| DR002338 | SR004566S | SR004656A |
| DR002339 | SR004567S | SR004657A |
| DR002340 | SR004568S | SR004658A |
| DR002341 | SR004569S | SR004659A |
| DR002342 | SR004570S | SR004660A |
| DR002343 | SR004571S | SR004661A |
| DR002221 | SR004440S | SR004442A |
| DR002358 | SR004676S | SR004609A |
| DR002359 | SR004677S | SR004610A |
| DR002360 | SR004678S | SR004611A |
| DR002361 | SR004679S | SR004614A |
| DR002362 | SR004680S | SR004615A |
| DR002363 | SR004681S | SR004616A |
| DR002364 | SR004682S | SR004617A |
| DR005642 | SR011225S | SR004442A |
| DR005656 | SR011239S | SR004442A |
| DR005672 | SR011269S | SR004610A |
| DR005675 | SR011272S | SR004610A |
| DR005674 | SR011271S | SR004609A |
| DR005676 | SR011273S | SR004614A |

### DESCRIPTION OF THE EMBODIMENTS

Unless otherwise specified, sources of raw materials used in the examples are as follows:
Huh7 cell line was purchased from Nanjing Cobioer, with an article No. CBP60202;
Hep3B cell line was purchased from Nanjing Cobioer, with an article No. CBP60197;
PHH cells were purchased from Shanghai Xuanyi, with an article No. QYLF-HPMC;
HEK293A cell line was purchased from Nanjing Cobioer, with an article No. CBP60436; and
Balb/c mice were purchased from Zhejiang Charles River, with an article No. Balb/c.

### Example 1 Preparation of a compound E7

### 1. 1. Preparation of an intermediate 3-4

### 1.1 1.1 Preparation of a compound 2

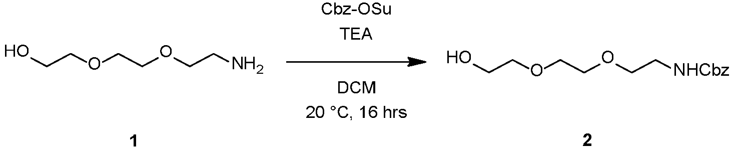

Benzyl(2,5-dioxopyrrolidine-1-yl)carbonate (600 g, 2.40 mol) was slowly added to DCM (1.80 L) of a compound 1 (300 g, 2.01 mol) at 15°C, and TEA (203 g, 2.01 mol, 280 mL) was added dropwise. After the addition, a mixture was stirred at 25°C for 16 h. TLC (dichloromethane:methanol=10:1) showed that the reactant 1 (R_{f}=0.32) was retained and an important new point (R_{f}=0.52) was detected. The reaction mixture was washed with a saturated sodium bicarbonate solution (1.00 L×2), and an organic phase was washed with a salt solution (1.00 L), dried with anhydrous Na₂SO₄ and concentrated under vacuum. A yellow oily compound 2 (about 385 g) was obtained without purification.

### 1.2 1.2 Preparation of a compound 2A

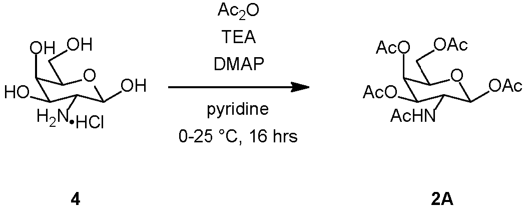

DMAP (19.8 g, 162 mmol) was added at one time to a pyridine solution (1.75 L) of a compound 4 (350 g, 1.62 mol, HCl) and Ac₂O (994 g, 9.74 mol, 912 mL) at 0-15°C, and TEA (164 g, 1.62 mol, 226 mL) was added dropwise. A mixture was stirred at 25°C for 16 h. LCMS (product: RT=0.687 min) showed that initial reactants were completely consumed. EtOAc (1.40 L) was added to the mixture at 25°C and stirred for 30 min, then the mixture was filtered, and a filter cake was cleaned with EtOAc (300 mL). The filter cake was ground with water (1.45 L) at 25°C for 30 min. A mixture was filtered, and a filter cake was cleaned with water (175 mL×3) and then collected to obtain a white solid compound 2A (about 580 g).

### 1.3 Preparation of a compound 2B

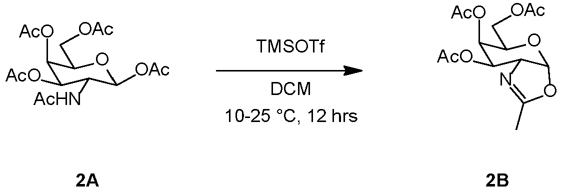

Three reactions were carried out in parallel.

TMSOTf (137 g, 616 mmol, 111 mL) was added dropwise to a DCM solution (800 mL) of the compound 2A (200 g, 514 mmol) at 10-15°C for 0.5 h. Then, a mixture was stirred at 25°C for 3 h. TLC (dichloromethane:methanol=20:1) showed that the compound 2A (R_{f}=-0.54) was completely consumed and a new point (R_{f}=0.24) was formed. The three reactions were combined. The mixture was cooled to 0-15°C and slowly poured to a NaHCO₃ solution (300 g dissolved in 3.00 L of water) at 0-5°C. An organic phase was separated, and an aqueous phase was extracted with DCM (1.00 L×3). Organic layers were combined, dried with Na₂SO₄, filtered and concentrated under vacuum. A yellow oily compound 2B (about 507 g) was obtained and directly used in a next step without purification.

### 1.4 1.4 Preparation of a compound 3

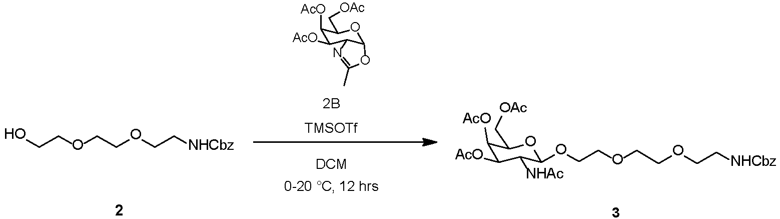

TMSOTf (84.4 g, 380 mmol, 69.0 mL) was added dropwise to a mixture of the compound 2B (250 g, 759 mmol) and the compound 2 (151 g, 531 mmol) in DCM (1.00 L) at 0-10°C, and the mixture was stirred at 20°C for 12 h. TLC (dichloromethane:methanol=20:1) showed that the compound 2 (R_{f}=0.33) was completely consumed and an new point (R_{f}=0.03) was formed. Combined reactants were cooled to 0-5°C, and then the reactants were poured to NaHCO₃ (aqueous solution, 100 g dissolved in 1 L of water) and stirred at 5-10°C for 10 min to separate phases. An aqueous phase was extracted with DCM (500 mL×2), combined organic phases were dried with Na₂SO₄ and filtered, and a filtrate was concentrated under vacuum. A yellow oily compound 3 (about 360 g) was obtained without purification.

¹H NMR: (400 MHz, DMSO).

δ=7.79-7.37 (m, 1H), 7.35-7.26 (m, 5H), 5.21-5.20 (m, 1H), 5.00-4.95 (m, 3H), 4.55-4.53 (m, 1H), 4.03-3.86 (m, 3H), 3.61-3.59 (m, 1H), 3.59-3.57 (m, 1H), 3.48-3.40 (m, 6H), 3.39-3.31 (m, 2H), 3.14-3.13 (m, 2H), 2.09 (s, 3H), 1.99 (s, 3H), 1.88 (s, 3H), 1.76-1.74 (m, 3H).

### 1.5 1.5 Preparation of an intermediate 3-4 (TFA salt)

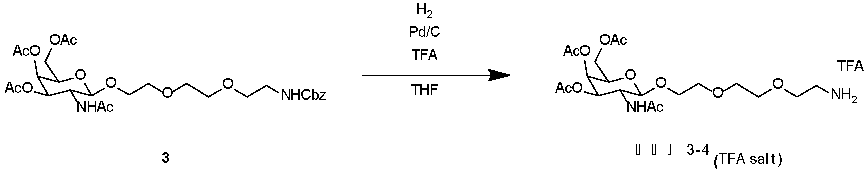

Three reactions were carried out in parallel.

The compound 3 (180 g, 293 mmol) and TFA (33.5 g, 293 mmol, 21.8 mL) were added to a mixture of Pd/C (18.0 g, 16.3 mmol, content: 10%) in THF (1.80 L) in an argon atmosphere. A suspension was subjected to degasification and hydrogen ventilation for three times. The mixture was stirred in H₂ (50 Psi) at 30°C for 2 h. LCMS (product: RT=0.697 min) showed that the compound 3 was consumed and a product peak was detected. The three reactions were combined. The mixture was filtered with celite, and a filtrate was concentrated under reduced pressure to remove a solvent. A yellow solid intermediate 3-4 (TFA salt) (393 g, 660 mmol, yield: 74.8%, purity: 99.6%, TFA) was obtained without purification.

¹H NMR: (400 MHz, DMSO-d6)

δ=7.92 (d, *J =* 9.1 Hz, 4H), 5.27-5.17 (m, 1H), 5.03-4.91 (m, 1H), 4.60-4.50 (m, 1H), 4.09-3.97 (m, 4H), 3.85 (s, 2H), 3.65-3.46 (m, 10H), 3.04-2.92 (m, 2H), 2.10 (s, 3H), 2.00 (s, 3H), 1.94-1.86 (m, 3H), 1.82-1.71 (m, 4H).

### 2. 2. Preparation of an intermediate 3-3

### 2.1 2.1 Preparation of a compound 5

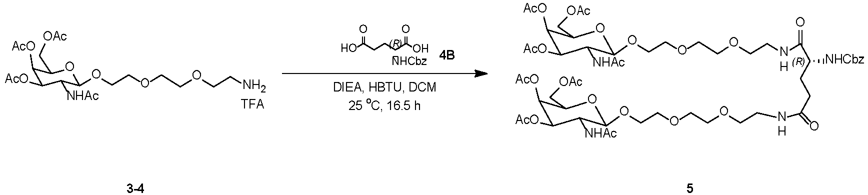

DIEA (30.3 g, 234 mmol, 40.8 mL, 6.60 *eq)* was added at one time to a DCM solution (1.00 L) of a compound 4B (10.0 g, 35.5 mmol, 1.00 *eq)* and the compound 3-4 prepared above (46.3 g, 78.2 mmol, 2.20 *eq,* TFA) at 25°C. Stirring was carried out at 25°C for half an hour. HBTU (30.3 g, 234 mmol, 40.8 mL, 6.60 *eq)* was added to a mixture. Stirring was carried out at 25°C for 16 h. LCMS (product: RT=0.681 mins) showed that a reaction was completed. The mixture was concentrated under vacuum. 0.50 N HCl (200 mL×2) was added to the mixture at 20°C, and the mixture was extracted with DCM (3×500 mL). Organic layers were combined, cleaned with saturated NaHCO₃ (3×800 mL) until a pH value was 8, cleaned with a salt solution (3×500 mL), dried with Na₂SO4, concentrated under vacuum and purified. A residue was purified by column chromatography (SiO₂, DCM: MeOH=50:1 to 15:1). The residue was concentrated under vacuum at 40°C and purified by preparative-MPLC (column: 800 g Agela C18; mobile phase: [water-ACN]; 15-45%, 25 min; 45%, 10 min). Vacuum drying was carried out to obtain a yellow solid compound 5 (about 180 g+75.0 g+87.0 g+40.0 g+38.0 g).

417.0 g of the compound 3-4 was converted into the compound 5 in 9 batches.

### 2.2 Preparation of an intermediate 3-3

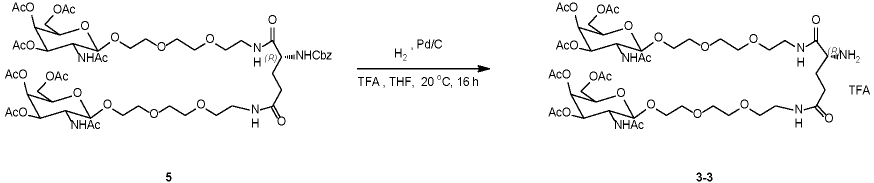

The compound 5 (73.0 g, 61.7 mmol, 1.00 *eq)* and TFA (7.04 g, 61.7 mmol, 4.57 mL, 1.00 *eq)* were added to THF (300 mL) of Pd/C (3.00 g, content: 10%) in an argon atmosphere. A suspension was subjected to degasification and hydrogen ventilation for three times. Stirring was carried out in H₂ (20 Psi) at 20°C for 16 h. TLC (dichloromethane:methanol=8: 1, R_{f}=0.0) showed that a reaction was completed. A mixture was filtered with celite, and a filtrate was concentrated under increased pressure to remove a solvent so as to obtain a white solid compound 3-3 (about 33.4 g+129 g+75.0 g).
¹H NMR: (400 MHz, DMSO)
*δ=8.53 (t, J =* 5.2 Hz, 1H), 8.18 (d, *J =* 2.4 Hz, 3H), 8.03 (t, *J =* 5.2 Hz, 1H), 7.84 (dd, *J =* 3.6 Hz, 2H), 5.22 (d, *J =* 3.2 Hz, 2H), 4.96 (dd, *J =* 3.2 Hz, 2H), 4.55 (d, *J* =8.4 Hz, 2H), 4.02 (t, *J* =8.8 Hz, 6H), 3.77-3.59 (m, 5H), 3.58-3.45 (m, 21H), 3.40-3.20 (m, 4H), 2.18 (t, *J* = 7.6 Hz, 2H), 2.17 (d, *J =*8.0 Hz, 6H), 2.10 (s, 6H), 1.99 (s, 6H), 1.90-1.80 (m, 8H), 1.77 (s, 6H).

### 3. 3. Preparation of a compound E7

### 3.1 Preparation of a compound 3

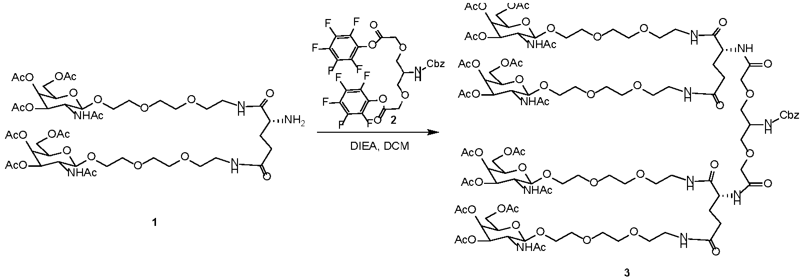

A compound 1 (2.00 g, 1.87 mmol, prepared by a method of the intermediate 3-3) was dissolved in DCM (20.0 mL) at room temperature, DIEA (0.135 mL, 0.814 mmol) and a compound 2 (0.550 g, 0.814 mmol) were sequentially added to a resulting solution, and nitrogen replacement was carried out for 3 times. A reaction mixture was stirred at 25°C for 16 h. Liquid chromatography-mass spectrometry detected an MS response of a product. Thin layer chromatography (dichloromethane/methanol=5/1) showed that raw materials disappeared and a new point was generated. A reaction solution was concentrated under reduced pressure, and a resulting crude product was purified by column chromatography (dichloromethane/methanol=5/1) to obtain a white solid compound 3 (about 780 mg).
¹H NMR (400 MHz, CD₃OD)
*δ*=7.28-7.42 (m, 5H), 5.30-5.34 (m, 4H), 5.04-5.14 (m, 6H), 4.63-4.67 (m, 4H), 4.36-4.44 (m, 2H), 4.00-4.20 (m, 23H), 3.91-3.95 (m, 4H), 3.69-3.77 (m, 9H), 3.52-3.67 (m, 32H), 3.34-3.43 (m, 9H), 2.29-2.31 (m, 4H), 2.14 (s, 12H), 2.03 (s, 12H), 1.92-1.96 (m, 24H). LCMS: m/z=1221.6 (M/2+H)⁺.

### 3.2 3.2 Preparation of a compound 4

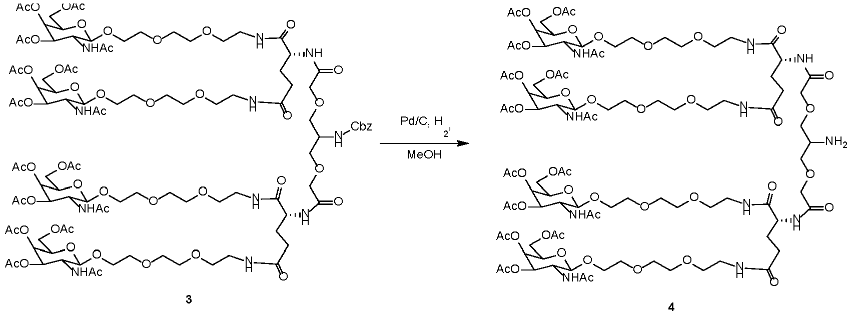

The compound 3 (1.10 g, 0.451 mmol) was dissolved in MeOH (10.0 mL) at room temperature, wet Pd/C (0.050 g, 0.451 mmol) with a mass fraction of 10% was added to a resulting solution, hydrogen replacement was carried out for 3 times, and a reaction mixture was stirred in a hydrogen atmosphere (14.696 psi) at 25°C for 18 h. Liquid chromatography-mass spectrometry detected an MS response of a product. Thin layer chromatography (dichloromethane/methanol=10/1, with phosphomolybdic acid as a color developing agent) showed that raw materials were completely consumed and a new point was generated. A reaction solution was filtered, and a filtrate was concentrated under reduced pressure to obtain a white solid compound 4 (about 840 mg).
¹H NMR (400 MHz, CD₃OD)
*δ*=5.32-5.34 (m 4H), 5.06-5.10 (m, 4H), 4.63-4.65 (m, 4H), 4.38-4.40 (m, 2H), 3.99-4.20 (m, 20H), 3.90-3.97 (m, 4H), 3.69-3.76 (m, 6H), 3.50-3.68 (m, 36H), 3.35-3.44 (m, 11H), 2.28-2.38 (m, 4H), 2.15 (s, 12H), 2.03 (s, 12H), 1.90-1.94 (m, 24H). LCMS: m/z=1154.7 (M/2+H)⁺.

### 3.3 3.3 Preparation of a compound 6

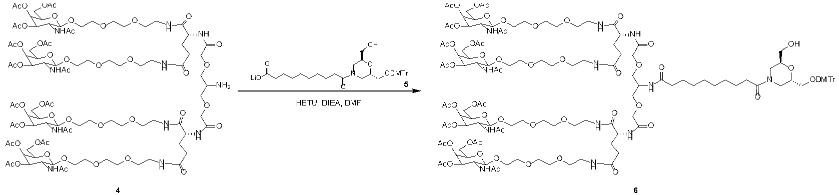

The compound 5 (232 mg, 0.364 mmol) was dissolved in DCM (10.0 mL) at room temperature, HBTU (207 mg, 0.546 mmol), DIEA (0.181 mL, 1.09 mmol) and the compound 4 (840 mg, 0.364 mmol) were sequentially added to a resulting solution, and nitrogen replacement was carried out for 3 times. A reaction mixture was stirred at 25°C for 1 h. Liquid chromatography-mass spectrometry detected that raw materials disappeared. Thin layer chromatography (dichloromethane/methanol=5/1) showed that the raw materials disappeared and a new point was generated. A reaction solution was concentrated under reduced pressure, and a resulting crude product was purified by column chromatography (dichloromethane/methanol= 8/1 to 5/1) to obtain a white solid compound 6 (about 620 mg).
¹H NMR (400 MHz, CD₃OD)
*δ*=7.41-7.43 (m, 2H), 7.23-7.34 (m, 7H), 6.83-6.90 (m, 4H), 5.31-5.35 (m, 4H), 5.01-5.12 (m, 4H), 4.63-4.65 (m, 4H), 4.41-4.45 (m, 2H), 4.31-4.33 (m, 1H), 3.99-4.22 (m, 22H), 3.87-3.97 (m, 6H), 3.58-3.81 (m, 45H), 3.34-3.43 (m, 10H), 2.19-2.40 (m, 10H), 2.14 (s, 12H), 2.02 (s, 12H), 1.92-1.96 (mz, 24H), 1.48-1.63 (m, 4H), 1.28-1.38 (m, 8H). LCMS: m/z=1460.0 (M/2+H)⁺.

### 4. 4. Preparation of a compound E7

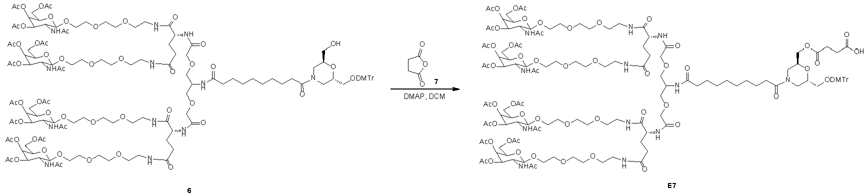

The compound 6 (300 mg, 0.103 mmol) was dissolved in DCM (10.0 mL) at room temperature, DIEA (0.102 mL, 0.618 mmol), a compound 7 (10.3 mg, 0.103 mmol) and DMAP (12.6 mg, 0.103 mmol) were sequentially added to a resulting solution, and nitrogen replacement was carried out for 3 times. A reaction mixture was stirred at 25°C for 2 h. Liquid chromatography-mass spectrometry detected that raw materials disappeared. A reaction solution was concentrated under reduced pressure, and a resulting crude product was subjected to preparative separation by preparative high performance liquid chromatography (preparative-HPLC, column: Waters Xbridge BEH C18 100*30mm*10um; mobile phase: water-ACN; B%: 17%-57%, 5 min) to obtain a white solid compound E7 (53.0 mg, yield: 17.08%, purity: 78.94%).
¹H NMR (400 MHz, CD₃OD)
*δ*=7.41-7.45 (m, 2H), 7.17-7.34 (m, 7H), 6.85-6.89 (m, 4H), 5.32-5.36 (m, 4H), 5.03-5.13 (m, 4H), 4.63-4.67 (m, 4H), 4.38-4.47 (m, 2H), 4.32-4.34 (m, 1H), 4.01-4.26 (m, 22H), 3.88-4.00 (m, 6H), 3.77-3.81 (m, 7H), 3.49-3.76 (m, 45H), 3.33-3.47 (m, 10H), 2.56-2.62 (m, 2H), 2.45-2.55 (m, 3H), 2.21-2.38 (m, 7H), 2.14 (s, 12H), 2.05-2.11 (m, 2H), 2.02 (s, 12H), 1.92-1.96 (m, 24H), 1.47-1.68 (m, 4H), 1.28-1.34 (m, 8H).
MS: m/z=3022.36 (M+H)⁺.

### Example 2 Preparation of a compound E13

### 1. Preparation of an intermediate 2-2

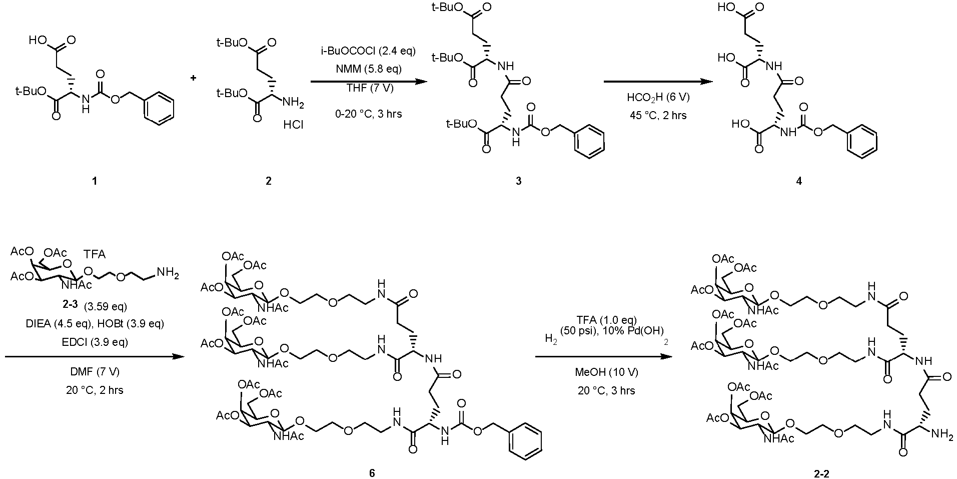

### 1.1. Preparation of a compound 3

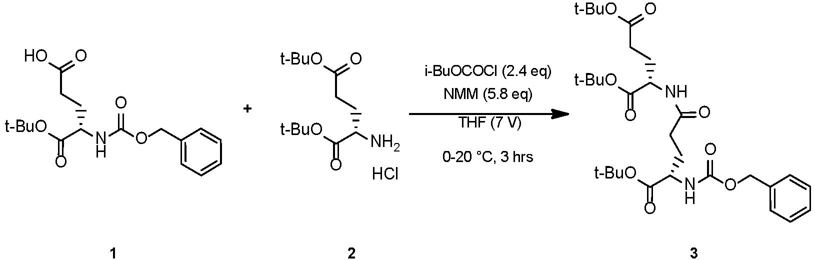

### 4 reactions were carried out in parallel.

4-methylmorpholine (434 g, 4.30 mol, 472 mL) was added to a THF solution (1.75 L) of a compound 1 (250 g, 741 mmol) and then cooled to 0°C. Isobutyl chloroformate (243 g, 1.78 mol, 233 mL) was added to a reaction mixture within 10 min to maintain a reaction temperature less than 4.0°C. After the addition, the mixture was stirred for 40 min or above, and a compound 2 (526 g, 1.78 mol) was added to the reaction mixture successively within 10 min to maintain a reaction temperature less than 4.0°C. After the addition, an ice bath was removed, and then reactants were allowed to be heated to room temperature continuously for 2 h. TLC (petroleum ether/ethyl acetate=1/1, compound 1 R_{f}=0.43) showed that the compound 1 was completely consumed and a new point was formed. The 4 reactions were combined. A reaction solution was poured to a stirred cold (0°C) 0.50 M HCl (aq.) (12.0 L) solution and stirred for about 10 min. Then, EtOAc (4.00 L×3) was added and stirred for a period of time, liquid separation was carried out, and an organic phase was washed with a salt solution (10.0 L), dried with Na₂SO₄ and concentrated under vacuum to produce a thick colorless oily compound. Hexane (1.20 L) was added to the stirred oily compound. White smoke appeared in a solution and then disappeared after further stirring. A seed crystal (1.20 g, 0.10 wt %) was added, and a white crystal was formed slowly at this time. Within 20 min, a suspension was thick enough to hinder stirring. At this time, additional hexane (6.00 L) was added and stirred for 12 h. The suspension was filtered, cleaned with hexane (1.20 L) and dried to obtain a white solid compound 3 (about 1.20 kg).
¹H NMR: (400 MHz DMSO)
*δ*=8.10-8.08 (m, 1H), 7.62-7.60 (m, 1H), 7.38-7.31 (m, 5H), 5.11-4.94 (m, 2H), 4.12-4.09 (m, 1H), 3.92-3.87 (m, 1H), 2.25-2.19 (m, 4H), 1.90-1.88 (m, 2H), 1.73-1.67 (m, 2H), 1.40-1.39 (m, 27H).

### 1.2 Preparation of a compound 4

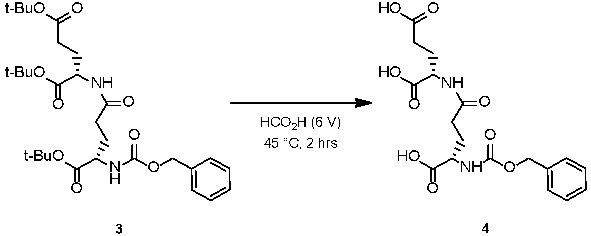

### 5 reactions were carried out in parallel.

An HCOOH solution (2.40 L) of the compound 3 (240 g, 415 mmol) was stirred at 45°C for 2 h. LCMS (compound 4=0.570 min) showed that the compound 3 was completely consumed and a main peak having a required m/z value appeared. The 5 reactions were combined. Toluene and ACN (each 1.50 L) were used for dilution and concentration. Azeotropic treatment was carried out with ACN and toluene (500 mL) at 1:1 and ACN for three times (500 mL each time) to remove formic acid. A compound 4 was subjected to high vacuum drying. Then, a residue was stirred with DCM (500 mL), and an organic layer was poured off. Then, azeotropic drying was carried out for two times with ACN (400 mL), vacuum drying was carried out, and then azeotropic treatment was carried out with toluene (400 mL) for 9 times to obtain a white solid compound 4 (about 800 g).
¹H NMR: (400 MHz DMSO)
*δ*=12.4-12.2 (m, 2H), 8.12-8.10 (m, 1H), 7.62-7.60 (m, 1H), 7.38-7.34 (m, 5H), 5.07-5.02 (m, 2H), 4.22-4.17 (m, 1H), 3.99-3.96 (m, 1H), 2.31-2.21 (m, 4H), 2.00-1.93 (m, 2H), 1.76-1.75 (m, 2H).

### 1.3. Preparation of a compound 6

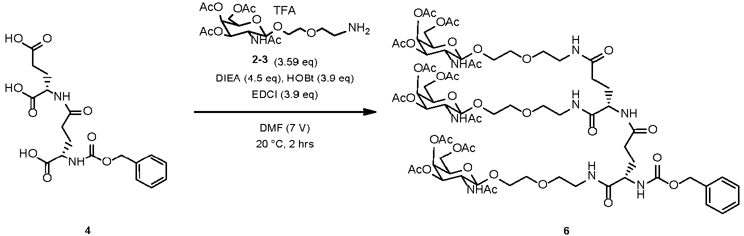

HOBT (103 g, 760 mmol), EDCI (146 g, 760 mmol) and DIEA (113 g, 877 mmol, 153 mL) were sequentially added to a stirred DMF solution (2.00 L) of the compound 4 (80.0 g, 194 mmol) and a compound 2-3 (384 g, 700 mmol, TFA). Reactants were stirred at 20°C for 2 h. TLC (dichloromethane/methanol=5/1, compound 6 R_{f}=0.43) showed that the compound 4 was completely consumed and a new point was formed. A reaction mixture was slowly poured to a stirred cold 0.5 M HCl aqueous solution (230 mL), stirred for 10 min to form a white solid and then filtered, and an aqueous phase was extracted with DCM (1.50 L) for two times. Combined organic phases were cleaned with 5% NaHCO₃ (aq.) (200 mL), then dried (Na₂SO₄) and evaporated and concentrated under increased pressure. A residue was purified by column chromatography (SiO₂, dichloromethane/methanol=5/1, compound 6 R_{f}=0.43) to obtain a yellow solid compound 6 (about 180 g).
¹H NMR: (400 MHz DMSO)
*δ* 7.95-7.91 (m, 3H), 7.82-7.80 (m, 4H), 7.39-7.31 (m, 6H), 5.21-5.01 (m, 3H), 5.00-4.96 (m, 5H), 4.56-4.53 (m, 3H), 4.02 (s, 1H), 3.88 (s, 9H), 3.85 (s, 4H), 3.76 (s, 3H), 3.50-349 (m, 9H), 3.39-3.36 (m, 6H), 3.19-3.15 (m, 6H), 2.15-2.05 (m, 12H), 1.99 (s, 9H), 1.89-1.77 (m, 21H).

### 1.4. Preparation of an intermediate 2-2

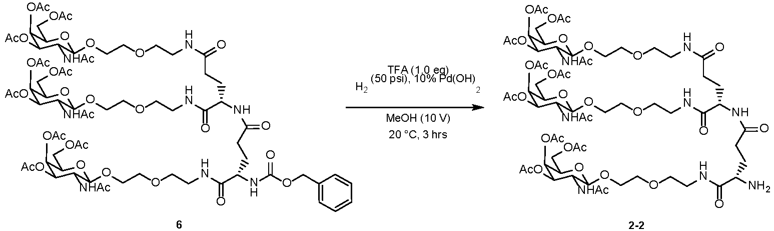

Pd(OH)₂/C (4.94 g, 3.52 mmol, content: 10%) was added to a dry hydrogenation flask in an argon atmosphere, MeOH (350 mL) was added, and the compound 6 (47.4 g, 28.5 mmol) and TFA (3.26 g, 28.5 mmol, 2.11 mL) were sequentially added and stirred in H₂ (50 psi) at 20°C for 3 h. TLC (dichloromethane/methanol=5/1, intermediate 2-2 R_{f}=0.25) monitored a reaction and showed that the compound 6 was consumed. A reaction mixture was filtered, and a filtrate was concentrated under vacuum to obtain a white solid intermediate 2-2 (TFA salt) (46.3 g, yield: 94.6%, purity: 95.7%, TFA).
¹H NMR: (400 MHz DMSO)
δ=8.56-8.50 (m, 1H), 8.14-8.05 (m, 4H), 7.99 (s, 1H), 7.88-7.82 (m, 4H), 5.22-5.21 (m, 3H), 4.99-4.96 (m, 3H), 4.55-4.53 (m, 3H), 4.09 (s, 1H), 4.03 (s, 9H), 3.87-3.79 (m, 6H), 3.59-3.52 (m, 1H), 3.51-347 (m, 9H), 3.39-3.37 (m, 5H), 3.17-3.16 (m, 6H), 2.22-2.10 (m, 12H), 1.99 (s, 9H), 1.93-1.73 (m, 21H).

### 2. 2. Preparation of a compound E13

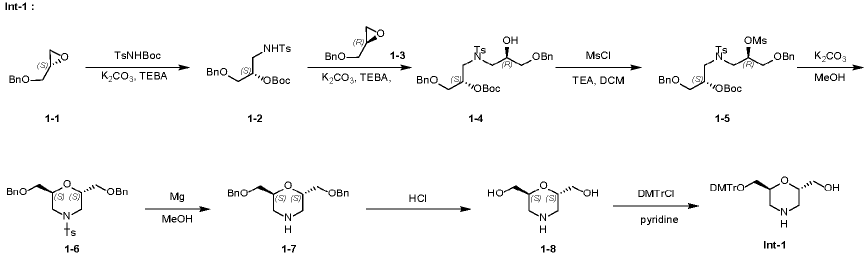

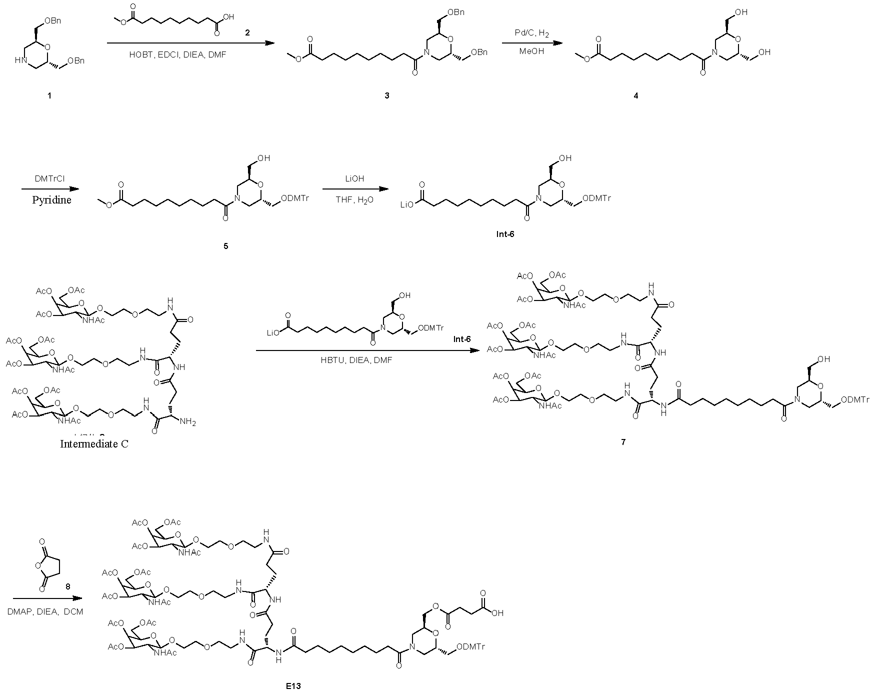

### 2.1 Preparation of a compound Int1

### 2.1.1 Preparation of a compound 1-2

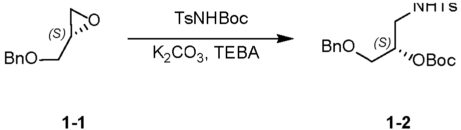

At room temperature, a compound 1-1 (38.0 g, 231 mmol), TsNHBoc (75.4 g, 278 mmol), K₂CO₃ (6.40 g, 46.3 mmol) and TEBA (5.27 g, 23.1 mmol) were added to a three-necked flask. A reaction was carried out at 95°C for 2 h, and then cooling was carried out to 25°C. TLC (PE/EA=2/1, UV 254 nm) showed that the reaction was completed. A reaction solution was diluted with water (500 mL) and then extracted with dichloromethane (200 mL×3). An organic phase was concentrated under reduced pressure, and a crude product was purified by column separation (PE/EA=10/1 to 3/1) to obtain a colorless oily compound 1-2 (about 40.0 g).
¹H NMR (400 MHz, CDCl₃)
δ 7.72 (d, *J =* 8.4 Hz, 2 H), 7.28 - 7.39 (m, 7 H), 4.72 - 4.86 (m, 2 H), 4.42 - 4.56 (m, 2 H), 3.52 - 3.61 m, 2 H), 3.14 - 3.39 (m, 2 H), 2.43 (s, 3 H), 1.47 (s, 9 H).

### 2.1.2 Preparation of a compound 1-4

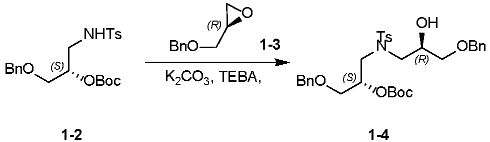

The compound 1-2 (79.0 g, 181 mmol), a compound 1-3 (4.21 g, 30.5 mmol) and TEBA (3.47 g, 15.2 mmol) were enabled to undergo a reaction at 95°C for 3 h. TLC (PE/EA=2/1, UV 254 nm) showed that the reaction was completed. A reaction solution was diluted with water (500 mL) and then extracted with dichloromethane (200 mL×3). An organic phase was concentrated under reduced pressure, and a crude product was purified by column separation (PE/EA=10/1 to 3/1) to obtain a colorless oily compound 1-4 (about 40.0 g).
¹H NMR (400 MHz, CDCl₃)
δ=7.64 - 7.77 (m, 2 H), 7.27 - 7.38 (m, 13 H), 5.05 - 5.16 (m, 1 H), 4.50 - 4.59 (m, 4 H), 4.03 - 4.11 (m, 1 H), 3.62 - 3.76 (m, 2 H), 3.44 - 3.58 (m, 3 H), 3.25 - 3.33 (m, 2 H), 3.15 - 3.20 (m, 1 H), 2.42 (s, 3 H), 1.46 (s, 9 H).

### 2.1.3 Preparation of a compound 1-5

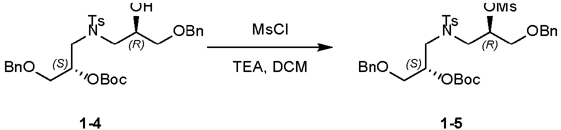

In an ice bath, the compound 1-4 (77.0 g, 128 mmol) and Et₃N (28.6 mL, 205 mmol) were sequentially added to dichloromethane (800 mL), and methanesulfonyl chloride (24.2 g, 212 mmol) was slowly added dropwise. A reaction was carried out at 25°C for 2 h. LCMS showed that the reaction was completed. Water (800 mL) was added to a reaction solution for washing, and an organic phase was concentrated under reduced pressure to obtain a colorless oily compound 1-5 (about 90.0 g).
LCMS (ESI): m/z=700.1 (M+Na)⁺;
¹H NMR (400 MHz, CDCl₃) δ 7.68 (d, *J* = 8.4 Hz, 2 H), 7.28 - 7.39 (m, 12 H), 5.10 - 5.20 (m, 1 H), 4.93 - 5.02 (m, 1 H), 4.52 - 4.61 (m, 4 H), 3.72 - 3.88 (m, 2 H), 3.52 - 3.67 (m, 4 H), 3.27 - 3.36 (m, 1 H), 3.15 - 3.22 (m, 1 H), 3.04 (s, 3 H), 2.43 (s, 3 H), 1.43 (s, 9 H). LCMS: m/z=700.1 (M+Na)⁺.

### 2.1.4 Preparation of a compound 1-6

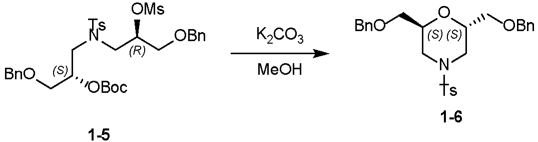

At room temperature, the compound 1-5 ( 90.0 g, 133 mmol) and potassium carbonate (91.8 g, 664 mmol) were added to MeOH (900 mL). A reaction was carried out at 66°C for 2 h. TLC (PE/EA=2/1, UV 254 nm) showed that a new point was generated. An organic phase was concentrated under reduced pressure, water (200 mL) was added to a reaction solution for dilution, and extraction was carried out with dichloromethane (200 mL×3). An organic phase was concentrated under reduced pressure, and a crude product was purified by column separation (PE/EA=30/1 to 2/1) to obtain a white solid compound 1-6 (about 64.0 g).

¹H NMR (400 MHz, CDCl₃) δ 7.62 (d, *J =* 8.4 Hz, 2 H), 7.27 - 7.39 (m, 12 H), 4.48 - 4.62 (m, 4 H), 3.93 - 4.08 (m, 2 H), 3.55 - 3.69 (m, 4 H), 2.84 - 3.10 (m, 4 H), 2.44 (s, 3 H).

### 1.1.5 Preparation of a compound 1-7

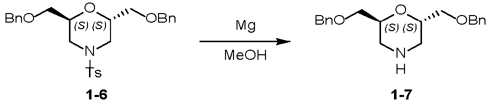

At room temperature, the compound 1-6 ( 69.0 g, 143 mmol) and magnesium chips (54.7 g, 2.28 mol) were added to MeOH (400 mL) to carry out a reaction at 66°C for 1 h. TLC (DCM/MeOH= 10/1, UV 254 nm) showed that raw materials were completely reacted and a new point was generated. A reaction solution was diluted with water (3,000 mL) and a saturated ammonium chloride aqueous solution (3,000 mL), and then extracted with dichloromethane (1,000×3). An organic phase was washed with saturated sodium bicarbonate (300 mL×3), and the organic phase was concentrated under reduced pressure to obtain a colorless oily compound 1-7 (about 32.0 g).

¹H NMR (400 MHz, CDCl₃) δ 7.27 - 7.40 (m, 10 H), 4.57 (s, 4 H), 3.90 - 4.00 (m, 2 H), 3.59 - 3.69 (m, 4 H), 2.77 - 3.04 (m, 4 H).

### 1.1.6 Preparation of a compound 1-8

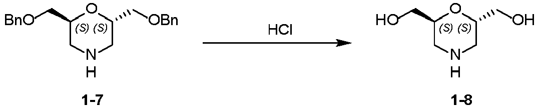

At room temperature, the compound 1-7 (8.00 g, 24.4 mmol) was added to HCl (100 mL, 12 M) to carry out a reaction at 50°C for 2 h. A reaction solution was concentrated under reduced pressure to obtain a colorless oily compound 1-8 (about 3.60 g, HCl salt),

¹H NMR (400 MHz, CDCl₃) δ 4.12 - 4.22 (m, 2 H), 3.78 (d, *J=* 4.4 Hz, 4 H), 3.17 - 3.30 (m, 4 H).

### 1.1.7 Preparation of a compound Int-1

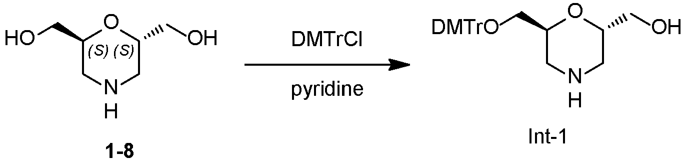

At room temperature, the compound 1-8 (8.29 g, 24.5 mmol) was added to pyridine (50 mL, 618 mmol), and then DMTrCl (4.77 g, 12.3 mmol) was added. The reaction system was enabled to undergo a reaction at 25°C for 18 h. TLC (DCM/MeOH=10/1, UV 254 nm) showed that a product was generated. A reaction solution was concentrated under reduced pressure, diluted with saturated ammonium chloride (200 mL) and extracted with DCM (100 mL×3). An organic phase was concentrated under reduced pressure. A crude product was separated by column chromatography (DCM/MeOH=99/1 to 10/1) to obtain a yellow solid Int-1 (about 2.6 g, 5.78 mmol, 23.6%).

¹H NMR (400 MHz, CDCl₃) δ 7.38 - 7.50 (m, 2 H), 7.25 - 7.36 (m, 6 H), 7.18 - 7.25 (m, 1 H), 6.87 (d, *J =* 8.8 Hz, 4 H), 4.26 - 4.40 (m, 1 H), 3.94 - 4.05 (m, 1 H), 3.85 (d, *J =* 4.4 Hz, 2 H) 3.73 - 3.82 (m, 6 H), 3.34 - 3.41 (m, 1 H), 3.23 - 3.30 (m, 3 H), 3.05 - 3.23 (m, 2 H).

### 2.2 2.2 Preparation of a compound Int6

### 2.2.1 Preparation of a compound 3

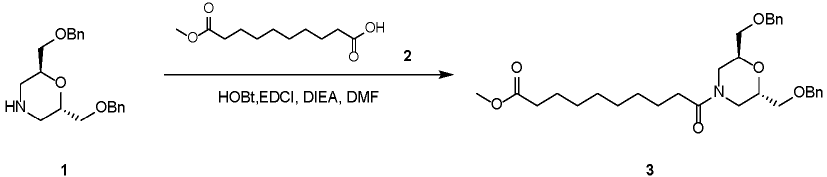

A compound 1 (1.00 g, 3.05 mmol) was dissolved in anhydrous N,N-dimethylformamide (10.0 mL) at 25°C, HOBt (0.54 g, 3.97 mmol), EDCI (0.76 g, 3.97 mmol) and DIEA (1.51 mL, 9.16 mmol) were sequentially added, and then a compound 2 (0.79 g, 3.66 mmol) was added. A mixed solution was stirred at 25°C for 2 h. Liquid chromatography-mass spectrometry showed that raw materials were completely reacted and a product was generated. Thin layer chromatography (dichloromethane:methanol=10:1) showed that the raw materials were completely reacted and a product was generated. Dichloromethane (200 ml) was added to the mixed solution, and the mixed solution was washed with a saturated citric acid solution (20 ml×3), a saturated sodium bicarbonate solution (20 ml×3) and a saturated salt solution (20 ml×3) for three times, respectively. An organic phase was dried with anhydrous sodium sulfate and then concentrated under reduced pressure to obtain a crude product. The crude product was purified by column chromatography (dichloromethane: methanol=10:1) to obtain a yellow oily compound 3 (about 1.00 g).

¹H NMR (400 MHz, CD₃OD) δ 7.21-7.41 (m, 10H), 4.47-4.60 (m, 4H), 3.88-4.01 (m, 2H), 3.75 (dd, *J =* 3.2, 13.2 Hz, 1H), 3.63-3.65 (m, 3H), 3.40-3.63 (m, 7H), 2.27-2.34 (m, 4H), 1.50-1.60 (m, 4H), 1.28-1.34 (m, 8H).

LCMS: m/z=526.8 (M+H)⁺.

### 2.2.2 2.2.2 Preparation of a compound 4

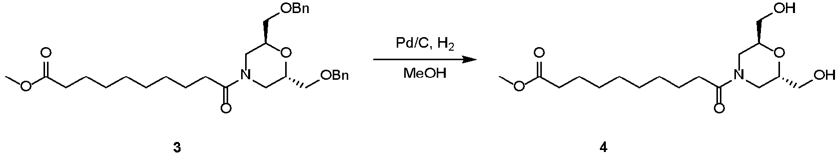

The compound 3 (380 mg, 0.20 mmol) was dissolved in anhydrous methanol (10.0 mL) at 25°C, and wet Pd/C (500 mg, 4.70 mmol) with a mass fraction of 10% was added. A reaction solution was stirred at 50°C for 12 h. Liquid chromatography-mass spectrometry showed that a product was generated. The reaction solution was filtered with diatomite, and a filtrate was concentrated under reduced pressure to obtain a yellow oily compound 4 (about 600 mg).

¹H NMR (400 MHz, CD₃OD) δ 3.79-3.89 (m, 2H), 3.73 (dd, *J =* 3.6, 13.2 Hz, 1H), 3.64-3.68 (m, 5H), 3.57-3.62 (m, 2H), 3.48-3.56 (m, 2H), 3.38-3.47 (m, 1H), 1.58-1.62 (m, 5H), 1.34 (d, *J* = 2.8 Hz, 11H), 1.30-1.37 (m, 1H). LCMS: m/z=346.5 (M+H)⁺.

### 2.2.3 2.2.3 Preparation of a compound 5

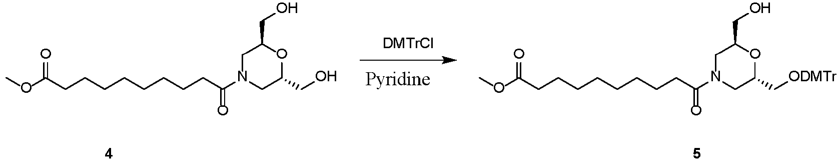

The compound 4 (600 mg, 1.74 mmol) was dissolved in pyridine (3.00 mL) at 25°C, and DMTrCl (883 mg, 2.61 mmol) was added. A reaction solution was stirred at 25°C for 1 h. Liquid chromatography-mass spectrometry showed that a product was generated. Thin layer chromatography (dichloromethane:methanol=10:1) showed that raw materials were completely reacted and a product was generated. Dichloromethane (200 ml) was added to a mixed solution, and the mixed solution was washed with a saturated sodium bicarbonate solution (20 ml×3) and a salt solution (20 ml×3) for three times, respectively. An organic phase was dried with anhydrous sodium sulfate and then concentrated under reduced pressure to obtain a crude product. The crude product was purified by column chromatography (petroleum ether: ethyl acetate=3:1) to obtain a colorless liquid compound 5 (about 475 mg).

¹H NMR (400 MHz, CD₃OD) δ 7.41-7.43 (m, 2H), 7.16-7.34 (m, 7H), 6.83-6.87 (m, 4H), 3.88-4.04 (m, 2H), 3.77-4.09 (m, 7H), 3.61-3.68 (m, 4H), 3.43-3.58 (m, 3H), 3.33-3.38 (m, 1H), 3.14-3.20 (m, 1H), 3.05 (dd, *J* = 8.4, 13.2 Hz, 1H), 2.20-2.42 (m, 4H), 1.51-1.60 (m, 4H), 1.25-1.39 (m, 8H). LCMS: m/z=648.3 (M+H)⁺.

### 2.2.4 2.2.4 Preparation of a compound Int-6

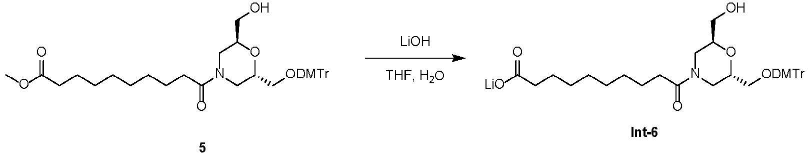

The compound 5 (475 mg, 0.73 mmol) was dissolved in tetrahydrofuran (8.00 mL) and H₂O (2.00 mL) at 25°C, and lithium hydroxide monohydrate (36.9 mg, 0.89 mmol) was added. A reaction solution was stirred at 25°C for 12 h. Thin layer chromatography (dichloromethane:methanol=10:1) showed that raw materials were completely reacted and a product was generated. The reaction solution was concentrated under reduced pressure and then directly freeze-dried on a freeze dryer to obtain a white solid product compound 6 (about 430 mg).

### 2.3 2.3 Preparation of a compound E13

### 2.3.1 Preparation of a compound 7

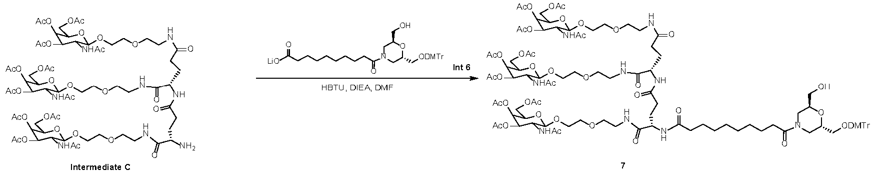

The compound Int-6 (100 mg, 0.156 mmol) was dissolved in anhydrous N,N-dimethylformamide (3.00 mL) at 25°C, and an HBTU reagent (89.0 mg, 0.234 mmol), DIEA (0.078 mL, 0.47 mmol) and an intermediate C (254 mg, 0.156 mmol, prepared by a method of the intermediate 2-2 in Example 2) were sequentially added. A reaction solution was stirred at 25°C for 12 h. Thin layer chromatography (dichloromethane:methanol=10:1) showed that a new point was generated. Dichloromethane (200 ml) was added to a mixed solution, and the mixed solution was washed with a saturated sodium bicarbonate solution (20 ml×3) for three times and washed with a salt solution (20 ml×3) for three times. An organic phase was dried with anhydrous sodium sulfate and then concentrated under reduced pressure to obtain a crude product. The crude product was purified by column chromatography (dichloromethane: methanol=10:1) to obtain a yellow oily product compound 7 (about 140 mg).

¹H NMR (400 MHz, CD₃OD) δ 7.43 (*d, J =* 8.4 Hz, 2H), 7.18-7.34 (m, 7H), 6.81-6.91 (m, 4H), 5.34 (d, *J* = 2.8 Hz, 3H), 5.09-5.11 (m, 3H), 4.62-4.66 (m, 3H), 4.27-4.35 (m, 2H), 4.07-4.17 (m, 9H), 4.00-4.07 (m, 4H), 3.89-3.98 (m, 5H), 3.79 (d, *J* = 2.4 Hz, 6H), 3.69-3.75 (m, 5H), 3.60-3.64 (m, 9H), 3.52-3.56 (m, 10H), 3.43-3.50 (m, 4H), 3.39-3.40 (m, 1H), 2.25-2.40 (m, 8H), 2.13-2.15 (m, 9H), 2.02 (s, 9H), 1.94-1.96 (m, 18H), 1.48-1.66 (m, 4H), 1.21-1.36 (m, 11H).

### 2.3.2 Preparation of E13

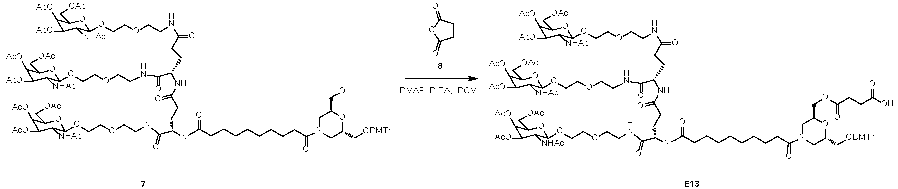

The compound 7 (400 mg, 0.187 mmol) was dissolved in anhydrous dichloromethane (5.00 mL), DIEA (0.18 mL, 1.12 mmol) and DMAP (5.71 mg, 0.047 mmol) were sequentially added, and a compound 8 (112 mg, 1.12 mmol) was added at last. A mixed solution was stirred at 25°C for 3 h. Thin layer chromatography (dichloromethane:methanol=10:1) showed that a new point was generated. Dichloromethane (200 ml) was added to the mixed solution, the mixed solution was washed with a saturated salt solution (15.0 ml×3) for three times, and an organic phase was dried with anhydrous sodium sulfate and then concentrated under reduced pressure to obtain a crude product. The crude product was separated by high performance liquid chromatography (chromatographic column: Welch Xtimate C18 150*25mm*5um; mobile phase: water-ACN; gradient: 27%-57%/11 min; flow rate: 25 ml/min) to obtain a white solid compound E13 (74.8 mg, 0.033 mmol, yield: 17.9%).

LCMS (ESI): m/z=1149.5 (M/2+H)⁺ ; ¹H NMR (400 MHz, CD₃OD) δ 7.42-7.44 (m, 2H), 7.18-7.34 (m, 7H), 6.83-6.90 (m, 4H), 5.33-5.34 (m, 3H), 5.08-5.11 (m, 3H), 4.61-4.68 (m, 3H), 4.27-4.36 (m, 2H), 4.19-4.27 (m, 1H), 4.10-4.19 (m, 7H), 4.08 (s, 2H), 4.00-4.07 (m, 4H), 3.90-3.94 (m, 4H), 3.83-3.89 (m, 1H), 3.79 (d, J=2.4 Hz, 6H), 3.70-3.72 (m, 4H), 3.53-3.54 (m, 15H), 3.44-3.48 (m, 3H), 3.35-3.40 (m, 4H), 3.10-3.23 (m, 2H), 2.53-2.63 (m, 4H), 2.22-2.40 (m, 8H), 2.11-2.16 (m, 9H), 2.05-2.10 (m, 2H), 2.02 (s, 9H), 1.90-1.97 (m, 19H), 1.48-1.65 (m, 4H), 1.29-1.32 (m, 8H).

### Example 3 Preparation of an siRNA

The siRNA of the present invention is prepared by a solid-phase phosphoramidite method well known in the field. The method can specifically refer to, for example, PCT publication No. WO2016081444 and No. WO2019105419, and is briefly described below.

### 1. Preparation of an siRNA without connection of a ligand

### 1.1 Synthesis of a sense strand (SS strand)

Through the solid-phase phosphoramidite synthesis method, with a blank CPG solid support as an initial cycle, nucleoside monomers were connected one by one in a direction from 3' to 5' according to a nucleotide arrangement sequence of a sense strand. Connection of each of the nucleoside monomers includes reactions in four steps including deprotection, coupling, capping, and oxidation or thiolation. Synthesis conditions of an oligonucleotide with a synthesis scale of 5 umol are as follows:
the nucleoside monomers were provided by a 0.05 mol/L acetonitrile solution, and the reaction in each step was carried out under a same condition, namely at a temperature of 25°C; the deprotection was carried out with a 3% trichloroacetic acid-dichloromethane solution, and the deprotection was carried out for 3 times; the coupling reaction was carried out with a 0.25 mol/L ETT-acetonitrile solution as an activator, and the coupling was carried out for 2 times; the capping was carried out with 10% acetic anhydride-acetonitrile and pyridine/N-methylimidazole/acetonitrile (10:14:76, v/v/v), and the capping was carried out for 2 times; the oxidation was carried out with 0.05 mol/L iodine/tetrahydrofuran/pyridine/water (70/20/10, v/v/v), and the oxidation was carried out for 2 times; and the thiolation was carried out with 0.2 mol/L acetonitrile/3-methylpyridine (1/1, v/v) of PADS, and the thiolation was carried out for 2 times.

### 1.2 Synthesis of an antisense strand (AS strand)

Through the solid-phase phosphoramidite synthesis method, with a blank CPG solid support as an initial cycle, nucleoside monomers were connected one by one in a direction from 3' to 5' according to a nucleotide arrangement sequence of an antisense strand. Connection of each of the nucleoside monomers includes reactions in four steps including deprotection, coupling, capping, and oxidation or thiolation. Synthesis conditions of a 5 umol oligonucleotide of the antisense strand are the same as those of the sense strand.

### 1.3 Purification and annealing of an oligonucleotide

### 1.3.1 Ammonolysis

A synthesized solid support (sense strand or antisense strand) was added to a 5 mL centrifuge tube, 3% diethylamine/ammonia water (v/v) was added, and a reaction was carried out in a constant-temperature water bath at 35°C (or 55°C) for 16 h (or 8 h). Filtration was carried out, the solid support was washed with ethanol/water for three times with 1 mL each time, a filtrate was centrifuged and concentrated, and a crude product was purified.

### 1.3.2 Purification

Methods for purification and desalination are well known to persons in the field. For example, a strong anion filler column can be used, a sodium chloride-sodium hydroxide system was used for elution and purification, a product was collected in a tube, a gel filler purification column can be used for desalination, and an elution system was pure water.

### 1.3.3 Annealing

According to Table 6, the sense strand (SS strand) and the antisense strand (AS strand) were mixed at a molar ratio (SS strand/AS strand=1/1.05), heated to 70-95°C in a water bath pot, maintained for 3-5 min, and then naturally cooled to room temperature, and the system was freeze-dried to obtain a product. Finally, double-stranded DR002254 to DR002343 were obtained.

### 2. Preparation of an siRNA connected by a sense strand and a ligand

### 2.1 Connection of a ligand and a CPG carrier

### 2.1.2 Connection of the compound E7 and the CPG carrier

After the compound E7 (53 mg, 0.018 mmol) and HBTU (13.3 mg, 0.035 mmol) were mixed, acetonitrile (5 mL) was added for dissolution by oscillation, and then DIEA (9.0 mg, 0.07 mmol) and DMAP (2.1 mg, 0.018 mmol) were added for dissolution by oscillation until a solution was clear. A blank carrier Resin (550 mg, CPG pore size 1,000 Å) was weighed and added to the reaction solution, the temperature was controlled at 20°C, and shaking was conducted to carry out a reaction overnight. A sample was taken and monitored, TLC was carried out, and results showed that a reaction was completed, where DCM/methanol=4/1 was used as a developing agent, and phosphomolybdic acid was used as a color developing agent. Filtration was carried out by a sand core funnel, a filter cake was washed with anhydrous acetonitrile (20 mL*5), and the filter cake was taken and subjected to suction filtration under reduced pressure by an oil pump for 6 h to obtain 530 mg of a white-like solid.

530 mg of the condensed product was placed in a 50 mL round-bottomed flask, CapC (DMAP/acetonitrile), CapB (N-methylimidazole/pyridine/acetonitrile) and CapA (acetic anhydride/acetonitrile) were sequentially added, and shaking was carried out overnight at room temperature. Filtration was carried out, a filter cake was washed with acetonitrile (20 mL*4), and the filter cake was taken and subjected to suction filtration under reduced pressure by an oil pump for 8 h to obtain 200 mg of a white-like solid for solid-phase synthesis.

### 2.1.2 Connection of the compound E13 and the CPG carrier

The compound E13 and the CPG carrier were connected by a method similar to a connection method of the compound E7 and the CPG carrier.

### 2.2 Synthesis of a sense strand (SS strand)

Through the solid-phase phosphoramidite synthesis method, with a GL6 solid support prepared above as an initial cycle, nucleoside monomers were connected one by one in a direction from 3' to 5' according to a nucleotide arrangement sequence of a sense strand. Connection of each of the nucleoside monomers includes reactions in four steps including deprotection, coupling, capping, and oxidation or thiolation. Synthesis conditions of an oligonucleotide with a synthesis scale of 5 umol are as follows:
the nucleoside monomers were provided by a 0.05 mol/L acetonitrile solution, and the reaction in each step was carried out under a same condition, namely at a temperature of 25°C; the deprotection was carried out with a 3% trichloroacetic acid-dichloromethane solution, and the deprotection was carried out for 3 times; the coupling reaction was carried out with a 0.25 mol/L ETT-acetonitrile solution as an activator, and the coupling was carried out for 2 times; the capping was carried out with 10% acetic anhydride-acetonitrile and pyridine/N-methylimidazole/acetonitrile (10:14:76, v/v/v), and the capping was carried out for 2 times; the oxidation was carried out with 0.05 mol/L iodine/tetrahydrofuran/pyridine/water (70/20/10, v/v/v), and the oxidation was carried out for 2 times; and the thiolation was carried out with 0.2 mol/L acetonitrile/3-methylpyridine (1/1, v/v) of PADS, and the thiolation was carried out for 2 times.

### 2.3 Synthesis of an antisense strand (AS strand)

Through the solid-phase phosphoramidite synthesis method, with a blank CPG solid support as an initial cycle, nucleoside monomers were connected one by one in a direction from 3' to 5' according to a nucleotide arrangement sequence of an antisense strand. Connection of each of the nucleoside monomers includes reactions in four steps including deprotection, coupling, capping, and oxidation or thiolation. Synthesis conditions of a 5 umol oligonucleotide of the antisense strand are the same as those of the sense strand.

### 2.4 Purification and annealing of an oligonucleotide

### 2.4.1 Ammonolysis

A synthesized solid support (sense strand or antisense strand) was added to a 5 mL centrifuge tube, 3% diethylamine/ammonia water (v/v) was added, and a reaction was carried out in a constant-temperature water bath at 35°C (or 55°C) for 16 h (or 8 h). Filtration was carried out, the solid support was washed with ethanol/water for three times with 1 mL each time, a filtrate was centrifuged and concentrated, and a crude product was purified.

### 2.4.2 Purification

Methods for purification and desalination are well known to persons in the field. For example, a strong anion filler column can be used, a sodium chloride-sodium hydroxide system was used for elution and purification, a product was collected in a tube, a gel filler purification column can be used for desalination, and an elution system was pure water.

### 2.4.3 Annealing

According to Table 6, the sense strand (SS strand) and the antisense strand (AS strand) were mixed at a molar ratio (SS strand/AS strand=1/1.05), heated to 70-95°C in a water bath pot, maintained for 3-5 min, and then naturally cooled to room temperature, and the system was freeze-dried to obtain a product.

An siRNA conjugated with L96 was obtained by a similar method.

Finally, the following siRNA(s) were obtained: DR002221, DR002358 to DR002364, DR005642, DR005656, DR005672, DR005674, DR005675, and DR005676.

### Example 4 Activity screening in a cell line

### 1. Activity screening in an HepG2 cell line

### Cell transfection

On the first day, an HepG2 (Nanjing Cobioer, article No. CBP60199) cell line was digested, resuspended, counted and spread on a 96-well plate at 100 µL/well and 1×10⁴ cells/well, and transfection was performed 18 h later.

On the second day, the siRNA prepared in Example 3 (DR002221 or DR002254 to DR002343) was diluted with Opti-MEM. 198 µL of Opti-MEM was taken, added to 2 µL of a compound mother solution and evenly mixed by blowing and sucking for later use. During each experiment, corresponding dilution operation was carried out according to different experimental demands.

On the second day, 0.9 µL of RNAiMAX (Thermo, 13778150) was diluted with 14.1 µL of Opti-MEM and evenly mixed by gentle blowing and sucking, followed by standing at room temperature for 5 min. Then, 15 µL of a prepared RNAi-MAX mixture and 15 µL of the diluted siRNA (with a final concentration of 10 nM) were evenly mixed by gentle blowing and sucking, subjected to standing at room temperature for 10 min, and then added to a 96-well plate at 10 µL/well. An RNA was extracted after culture was performed in an incubator containing 5% of CO₂ at 37°C for 24 h.

### RNA extraction

According to an operation protocol of a high-throughput cell RNA extraction kit (FireGen, FG0412), cell RNA extraction was performed by a nucleic acid extraction apparatus (Hangzhou Allsheng, Auto-pure96).

### RNA reverse transcription

A denaturation reaction mixture was prepared with reference to a PrimeScript^{™} II 1st Strand cDNA Synthesis Kit (Takara, 6210B). Each well contained 1 µL of an Oligo dT Primer, 1 µL of a dNTP Mixture, and 12.5 µL of a template RNA. Incubation was performed to carry out a denaturation reaction in a conventional PCR apparatus at 65°C for 5 min. The mixture was placed on ice and rapidly cooled for 2 min.

A reverse transcription reaction solution was prepared with reference to a PrimeScript^{™} II 1st Strand cDNA Synthesis Kit (Takara, 6210B). Each well contained 4 µL of a 5×Prime Script II Buffer, 0.5 µL of an RNase Inhibitor, and 1 µL of PrimeScript II RTase.

14.5 µL of a denatured reaction solution and the reverse transcription reaction solution were slowly mixed evenly, incubated in a conventional PCR apparatus for reverse transcription at 42°C for 45 min, and incubated for inactivation at 95°C for 5 min. A reverse transcription product (cDNA) was cooled at 4°C.

After the reverse transcription was completed, 30 µL of distilled water without a DNA enzyme and an RNA enzyme was added to a cDNA sample in each well.

### Fluorescent quantitative PCR

With reference to an operation process of a TaqMan^{™} Fast Advanced Master Mix (ABI, 4444965), 20 µL of a system was subjected to a fluorescent quantitative PCR reaction (ABI, QuantStudio3). A reaction procedure was as follows: (50°C, 2 min)×1 Cycle; (95°C, 20 s)× 1 Cycle; (95°C, 1 s; 60°C, 24 s)×40 Cycles.

**Table 7 Primer information**

| Primer name | Sequence information (5'-3') | Fluorescent group |
|---|---|---|
| hACTB-Primer-Forward | ACGTGGACATCCGCAAAGAC (SEQ ID NO: 417) | / |
| hACTB-Primer-Reverse | TCTTCATTGTGCTGGGTGCC (SEQ ID NO: 418) | / |
| hACTB-Probe | AACACAGTGCTGTCTGGCGGCACCA (SEQ ID NO: 419) | 5'TET, 3'BHQ2 |
| hPCSK9-Primer-Forward | AGAAACGCAGATTGGGCTGG (SEQ ID NO: 420) | / |
| hPCSK9-Probe-Reverse | GCCATCACTCACCGAGCTTC (SEQ ID NO: 421) | / |
| hPCSK9-Probe | TGAGGCTGGGAAGGGGAACACAGACCA (SEQ ID NO: 422) | 5'6-FAM, 3'BHQ1 |

### Data statistics

A 2^{-△△^{Ct}} value was calculated and converted into a percentage to obtain a residual inhibitory rate. ΔΔCt=[(Ct value of a target gene in an experimental group - Ct value of an internal reference in the experimental group) - (Ct value of a target gene in a control group - Ct value of an internal reference in the control group)].

The target gene is hPCSK9, and the internal reference is hACTB..

### 2. Activity screening for an Huh7 cell line

Similar to the activity screening in the HepG2 cell line, an Huh7 (Nanjing Cobioer, article No. CBP60202) cell line was used for activity screening. With a final concentration of the siRNA as 10 nM and 0.1 nM, activity screening of the siRNA compound was performed.

Results of the activity screening in the HepG2 cell line and the activity screening in the Huh7 cell line are shown in Table 8.

**Table 8 Experimental results of high-throughput activity screening in HepG2 and Huh7 cell lines**

| | HepG2-10nM | | Huh7-10nM | | Huh7-0.1nM | |
|---|---|---|---|---|---|---|
| siRNA number | Residual inhibitory rate | SD | Residual inhibitory rate | SD | Residual inhibitory rate | SD |
| Mock | 100.6% | 15.5% | 100.05% | 4.41% | 100.04% | 3.92% |
| DR002221 | 48.7% | 12.9% | 14.10% | 0.42% | 69.60% | 7.44% |
| DR002291 | 39.7% | 1.0% | 22.68% | 2.44% | 74.45% | 11.99% |
| DR002292 | 25.3% | 4.2% | 18.01% | 1.41% | 64.06% | 5.33% |
| DR002293 | 46.3% | 4.9% | 24.37% | 2.27% | 68.66% | 5.71% |
| DR002296 | 53.1% | 6.0% | 25.26% | 0.00% | 64.42% | 11.00% |
| DR002297 | 50.7% | 17.9% | 29.32% | 0.14% | 64.72% | 5.07% |
| DR002298 | 53.3% | 13.9% | 31.55% | 1.24% | 78.87% | 6.56% |
| DR002299 | 26.5% | 2.0% | 17.76% | 1.22% | 65.78% | 2.90% |

### Example 5 Activity screening (transfection) in primary mouse hepatocytes (PMH) of Tg mice

### Cell transfection

Primary mouse hepatocytes of Tg mice were isolated (Tg mice were derived from Gem Pharmatech, T053388), counted and spread on a 24-well plate at 900 µL/well and 8×10⁴ cells/well.

### Transfection

10 µL of the diluted siRNA (DR002221 or DR002254 to DR002343) prepared in Example 3 (with a final concentration of 10 nM) was added to 40 µL of Opti-MEM for even mixing. 3 µL of RNAiMAX was added to 47 µL of Opti-MEM for even mixing, incubated for 5 min, evenly mixed with the diluted siRNA, subjected to standing at room temperature for 10 min, and then added to corresponding wells. Culture was carried out in an incubator containing 5% of CO₂ at 37°C for 24 h.

### Fluorescent quantitative PCR

With reference to an operation process of a TaqMan^{™} Fast Advanced Master Mix (ABI, 4444965), 20 µL of a system was subjected to a fluorescent quantitative PCR reaction (ABI, QuantStudio3). A reaction procedure was as follows: (50°C, 2 min)×1 Cycle; (95°C, 20 s)× 1 Cycle; (95°C, 1 s; 60°C, 24 s)×40 Cycles.

### RNA extraction

A total RNA was extracted by a magnetic bead method using a high-throughput nucleic acid extraction apparatus, and then fluorescent quantitative PCR detection was performed after reverse transcription.

**Table 9 Primer information**

| Primer name | Sequence information (5'-3') | Fluorescent group |
|---|---|---|
| mGAPDH-Primer-Forward2 | CGGCAAATTCAACGGCACAG (SEQ ID NO: 423) | / |
| mGAPDH-primer-Reverse2 | CCACGACATACTCAGCACCG (SEQ ID NO: 424) | / |
| mGAPDH-Probe2 | ACCATCTTCCAGGAGCGAGACCCCACT | 5'TET, 3'BHQ2 |
| hPCSK9-Primer-Forward | AGAAACGCAGATTGGGCTGG (SEQ ID NO: 425) | / |
| hPCSK9-Primer-Reverse | GCCATCACTCACCGAGCTTC (SEQ ID NO: 426) | / |
| hPCSK9-Primer | TGAGGCTGGGAAGGGGAACACAGACCA (SEQ ID NO: 427) | 5'6-FAM, 3'BHQ1 |

### Data statistics

A 2⁻△△^{Ct} value was calculated and converted into a percentage to obtain a residual inhibitory rate. ΔΔCt=[(Ct value of a target gene in an experimental group - Ct value of an internal reference in the experimental group) - (Ct value of a target gene in a control group - Ct value of an internal reference in the control group)].

Experimental results of activity screening of the siRNA in PMH are shown in Table 10.

**Table 10 Experimental results (transfection) of activity screening in primary mouse hepatocytes of Tg mice**

| siRNA | Residual inhibitory rate | STD |
|---|---|---|
| DR002291 | 2.0% | 0.2% |
| DR002292 | 1.4% | 0.0% |
| DR002293 | 2.2% | 0.0% |
| DR002296 | 1.7% | 0.2% |
| DR002297 | 2.0% | 0.4% |
| DR002299 | 1.9% | 0.2% |
| DR002221 | 2.9% | 0.6% |

### Example 6 IC50 activity screening in primary human hepatocytes (PHH) using an siRNA at multiple concentrations

### A. Activity screening in primary human hepatocytes (PHH cells)

### Cell transfection

1.4 mL of a rat tail collagen solution (Sigma, C3867) was added to 40.6 mL of distilled water without a DNA enzyme and an RNA enzyme for even mixing. 40 µL of a mixture was added to each well of a 96-well culture plate and coated overnight at 4°C, and a coating solution was removed on the second day.

On the second day, before use, the coated cell plate was moistened with DPBS, and then the DPBS was sucked away. PHH cells were added to resuscitation culture medium for resuscitation at 37°C, then centrifuged, suspended and counted. The PHH cells were spread on a 96-well plate at 90 µL/well and 2×10⁴ cells/well. A complete culture medium was replaced 4 h later, and transfection was performed 18 h later.

On the third day, 198 µL of Opti-MEM was added to 2 µL of a 20 µM siRNA (DR002221 or DR002254 to DR002343), evenly mixed by blowing and sucking, and as a first concentration point, diluted at a corresponding gradient according to actual experimental needs.

On the third day, 0.9 µL of RNAiMAX (Thermo, 13778150) was diluted with 14.1 µL of Opti-MEM and evenly mixed by gentle blowing and sucking, followed by standing at room temperature for 5 min. Then, 15 µL of a prepared RNAi-MAX mixture and 15 µL of a diluted compound were evenly mixed by gentle blowing and sucking. A final concentration of the siRNA was 10 nM, 1 nM, 0.1 nM, 0.01 nM, or 0.001 nM. Standing was performed at room temperature for 10 min, and the mixture was added to a 96-well plate at 10 µL/well. An RNA was extracted after culture was performed in an incubator containing 5% of CO₂ at 37°C for 24 h.

### RNA extraction

Similar to Example 4, according to an operation protocol of a high-throughput cell RNA extraction kit (FireGen, FG0412), cell RNA extraction was performed by a nucleic acid extraction apparatus (Hangzhou Allsheng, Auto-pure96).

### RNA reverse transcription

A denaturation reaction mixture was prepared with reference to a PrimeScript^{™} II 1st Strand cDNA Synthesis Kit (Takara 6210B). Each well contained 1 µL of an Oligo dT Primer, 1 µL of a dNTP Mixture, and 12.5 µL of a template RNA. Incubation was performed to carry out a denaturation reaction in a conventional PCR apparatus at 65°C for 5 min. The mixture was placed on ice and rapidly cooled for 2 min.

A reverse transcription reaction solution was prepared with reference to a PrimeScript^{™} II 1st Strand cDNA Synthesis Kit (Takara, 6210B). Each well contained 4 µL of a 5×Prime Script II Buffer, 0.5 µL of an RNase Inhibitor, and 1 µL of PrimeScript II RTase.

14.5 µL of a denatured reaction solution and 5.5 µL of the reverse transcription reaction solution were slowly mixed evenly. Incubation was performed in a conventional PCR apparatus for reverse transcription at 42°C for 45 min, and incubatioin was performed for inactivation at 95°C for 5 min. A reverse transcription product (cDNA) was cooled at 4°C.

After the reverse transcription was completed, 30 µL of distilled water without a DNA enzyme and an RNA enzyme was added to a cDNA sample in each well.

### Fluorescent quantitative PCR

With reference to an operation process of a TaqMan^{™} Fast Advanced Master Mix (ABI, 4444965), 20 µL of a system was subjected to a fluorescent quantitative PCR reaction (ABI, QuantStudio3). A reaction procedure was as follows: (50°C, 2 min)×1 Cycle; (95°C, 20 s)× 1 Cycle; (95°C, 1 s; 60°C, 24 s)×40 Cycles.

**Table 11 Primer information**

| Primer name | Sequence information (5'-3') | Fluorescent group |
|---|---|---|
| hACTB-Primer-Forward | ACGTGGACATCCGCAAAGAC (SEQ ID NO: 428) | / |
| hACTB-Primer-Reverse | TCTTCATTGTGCTGGGTGCC (SEQ ID NO: 429) | / |
| hACTB-Probe | AACACAGTGCTGTCTGGCGGCACCA (SEQ ID NO: 430) | 5'TET, 3'BHQ2 |
| hPCSK9-Primer-Forward | AGAAACGCAGATTGGGCTGG (SEQ ID NO: 431) | / |
| hPCSK9-Primer-Reverse | GCCATCACTCACCGAGCTTC (SEQ ID NO: 432) | / |
| hPCSK9-Probe | TGAGGCTGGGAAGGGGAACACAGACCA (SEQ ID NO: 433) | 5'6-FAM, 3'BHQ1 |

### Data statistics

A 2⁻△△^{Ct} value was calculated and converted into a percentage to obtain a residual inhibitory rate. ΔΔCt=[(Ct value of a target gene in an experimental group - Ct value of an internal reference in the experimental group) - (Ct value of a target gene in a control group - Ct value of an internal reference in the control group)].

Experimental results are shown in Table 12.

**Table 12 Experimental results of IC50 activity screening using a PHH cell line**

| siRNA number | 10nM | 1nM | 0.1nM | 0.01nM | 0.001nM | IC50 (nM) |
|---|---|---|---|---|---|---|
| DR002221- | 30.6% | 45.1% | 71.1% | 98.5% | 93.3% | 0.5291 |
| DR002291 | 27.3% | 34.8% | 53.9% | 80.7% | 103.5% | 0.1416 |
| DR002292 | 33.8% | 36.6% | 54.9% | 77.8% | 100.6% | 0.1501 |
| DR002293 | 36.5% | 42.7% | 56.8% | 87.8% | 96.3% | 0.2943 |
| DR002296 | 22.8% | 43.5% | 52.9% | 86.3% | 102.8% | 0.2422 |
| DR002297 | 31.5% | 39.0% | 62.4% | 81.1% | 95.1% | 0.2996 |
| DR002298 | 32.1% | 47.3% | 61.1% | 82.8% | 85.7% | 0.5343 |
| DR002299 | 31.8% | 41.5% | 60.9% | 69.2% | 74.5% | 0.3567 |

### Example 7 Detection of off-target activity of psi-CHECK2

### Preparation of a plasmid

A corresponding off-target plasmid of an antisense strand was designed according to an siRNA sequence, a psiCHECK2 GSSM-5Hits recombinant plasmid was prepared by Sangon Biotech (Shanghai) Co., Ltd., and the recombinant plasmid was diluted to 1,000 ng/µL for later use.

### Cell transfection

100 µL of an HEK293A cell re-suspending solution was spread to each well of a 96-well plate at 8×10³ cells/well.

On the second day, a complete culture medium in wells was first sucked away and then changed into an Opti-MEM culture medium at 80 µL/well, and starvation treatment was performed for about 1.5 h.

siRNA preparation: An siRNA (DR002221 or DR002254 to DR002343) was diluted for 3 times from a final concentration of 40 nM in a total of 11 concentration points.

Plasmid mixture: A plasmid mixture in each well contained 0.01 µL of a plasmid and 8.99 µL of Opti-MEM.

Lipo mixture: Lipo 2000 (Lipofectamine^{™} 2000 transfection reagent, Thermo, 11668019) was diluted with Opti-MEM and then subjected to standing at room temperature for 5 min. A LIpo mixture in each well contained 0.2 µL of Lipo and 9.8 µL of Opti-MEM.

22 µL of the prepared Lipo mixture, 22 µL of the siRNA and 19.8 µL of the plasmid mixture were separately loaded to a same corresponding well, named Well A, evenly mixed by blowing and beating, incubated at room temperature for 20 min and then subjected to co-transfection. Finally, a Well A mixture was added to cells in each well at 20 µL/well. A final volume of each well was 100 µL, including 80 µL of the original Opti-MEM and 20 µL of the well A mixture. A final concentration of the siRNA was 10 nM, 3.33 nM, 1.11 nM, 0.37 nM, 0.12 nM, 0.041 nM, 0.013 nM, 0.0045 nM, 0.0015 nM, 0.0005 nM, or 0.00016 nM. After culture was performed in an incubator containing 5% of CO₂ at 37°C for 4 h, 100 µL of a DMEM culture medium containing 20% of fetal bovine serum was added to each well. After culture was carried out in the incubator containing 5% of CO₂ at 37°C for 24 h, detection was carried out.

### Detection of results

Before an experiment, well mixed Dual-Glo^{®}Luciferase (Dual-Glo^{®}Luciferase Assay System, Promega, E2940) was remelted, equalized to room temperature and then added with DMEM at a ratio of 1:1 in each tube to prepare a substrate I, which was prepared for immediate use. Dual-Glo^{®} Stop & Glo^{®} Buffer was remelted, equalized to room temperature and then mixed with Dual-Glo^{®} Stop & Glo^{®}Substrate at a ratio of 100:1 to prepare a substrate II, which was prepared for immediate use.

An original culture medium in a 96-well culture plate was sucked away by a vacuum pump.

150 µL of the substrate I was added to each well and incubated on a shaker at room temperature for 10 min.

120 µL of the substrate I was transferred to a 96-well ELISA plate, and a Firefly (firefly luciferase) chemiluminescence value on a microplate reader (Tecan, Infinite 200) was read.

Then, 60 µL of the substrate II was added to each well and incubated on a shaker at room temperature for 10 min. A Renilla (renilla luciferase) chemiluminescence value on the microplate reader was read.

### Analysis and processing of data

The fluorescence activity is determined by the microplate reader, and collected Renilla signals are normalized in accordance with a Firefly signal standard. An inhibitory effect of the siRNA is obtained by comparing unprocessed results (residual inhibitory activity), and a calculation process is shown as follows:
normalized Ren/Fir ratio: Ratio=Renilla/Firefly.
Residual inhibitory rate=(Ratio_{siRNA}/Ratio_{control})* 100%, taking an average value of results of two wells, where Ratio_{control} is a Ratio value of a control well (without the siRNA) (taking an average value of results of two wells).
Mapping: Mapping is carried out by using Graphpad Prism.
Half maximal inhibitory concentration (IC50): In this experiment, mapping is carried out based on Top and Bottom, and an IC50 value is obtained according to a formula Y=Bottom+(Top-Bottom)/(1+10^((LogIC50-X)*HillSlope)), where Y=50, and X=log (concentration).

Experimental results are shown in Table 13.

**Table 13 Experimental results of detection of off-target activity of psi-CHECK2**

| Compound number | 10nM | 3.33n M | 1.11n M | 0.37n M | 0.12n M | 0.041n M | 0.013n M | 0.0045n M | 0.0015n M | 0.0005n M | 0.00016n M | GSSM -5Hits fit IC50 (nM) |
|---|---|---|---|---|---|---|---|---|---|---|---|---|
| DR002221 | 106.1% | 103.7 % | 94.9% | 101.0 % | 96.9% | 93.4% | 96.2% | 96.0% | 106.2% | 90.2% | 86.6% | > 10 |
| DR002291 | 43.7% | 54.0% | 72.2% | 104.9 % | 101.3 % | 100.6% | 101.8% | 102.3% | 112.6% | 108.2% | 94.8% | 2.8961 |
| DR002292 | 97.3% | 89.8% | 105.9 % | 120.4 % | 93.6% | 85.6% | 99.8% | 99.4% | 172.9% | 90.5% | 96.0% | > 10 |
| DR002293 | 53.4% | 69.4% | 90.3% | 89.8% | 89.7% | 101.0% | 90.6% | 102.5% | 95.5% | 97.9% | 93.9% | > 10 |
| DR002296 | 74.4% | 90.5% | 109.8 % | 112.4 % | 103.1 % | 113.9% | 109.7% | 106.5% | 100.0% | 103.9% | 89.7% | > 10 |
| DR002297 | 14.3% | 18.7% | 39.9% | 76.3% | 99.1% | 103.4% | 107.0% | 102.4% | 104.9% | 104.0% | 87.6% | 0.8063 |
| DR002298 | 7.0% | 7.3% | 11.4% | 34.8% | 82.9% | 98.9% | 112.6% | 107.5% | 110.0% | 104.6% | 99.9% | 0.2604 |
| DR002299 | 43.0% | 43.8% | 72.3% | 102.6 % | 100.2 % | 101.7% | 116.4% | 100.9% | 96.4% | 96.3% | 86.6% | 1.2817 |

### Example 8 IC50 activity screening (free uptake) using primary mouse hepatocytes (PMH) of Tg mice

### Cell transfection

Primary mouse hepatocytes of Tg mice were isolated (Tg mice were derived from Gem Pharmatech, T053388), counted and spread on a 24-well plate at 900 µL/well and 8×104 cells/well.

10 µL of a diluted siRNA (DR002221 or DR002358 to DR002364) was added to 90 µL of Opti-MEM for even mixing, added to corresponding wells, and cultured in an incubator containing 5% of CO₂ at 37°C for 24 h to carry out free uptake activity screening. The siRNA with an initial concentration of 20 nM was diluted for 5 times to obtain 5 concentration points (20 nM, 4 nM, 0.8 nM, 0.16 nM, or 0.032 nM).

### Fluorescent quantitative PCR

A total RNA was extracted by a magnetic bead method using a high-throughput nucleic acid extraction apparatus, and then fluorescent quantitative PCR detection was performed after reverse transcription.

**Table 14 Primer information**

| Primer name | Sequence information (5'-3') | Fluorescent group |
|---|---|---|
| mGAPDH-Primer-Forward2 | CGGCAAATTCAACGGCACAG (SEQ ID NO: 434) | / |
| mGAPDH-Primer-Reverse2 | CCACGACATACTCAGCACCG (SEQ ID NO: 435) | / |
| mGAPDH-Probe2 | ACCATCTTCCAGGAGCGAGACCCCACT (SEQ ID NO: 436) | 5'TET, 3'BHQ2 |
| hPCSK9-Primer-Forward | AGAAACGCAGATTGGGCTGG (SEQ ID NO: 437) | / |
| hPCSK9-Primer-Reverse | GCCATCACTCACCGAGCTTC (SEQ ID NO: 438) | / |
| hPCSK9-Probe | TGAGGCTGGGAAGGGGAACACAGACCA (SEQ ID NO: 439) | 5'6-FAM, 3'BHQ1 |

### Data statistics

A 2⁻△△^{Ct} value was calculated and converted into a percentage to obtain a residual inhibitory rate. ΔΔCt=[(Ct value of a target gene in an experimental group - Ct value of an internal reference in the experimental group) - (Ct value of a target gene in a control group - Ct value of an internal reference in the control group)].

Experimental results are shown in Table 15.

**Table 15 Experimental results (free uptake) of IC50 activity screening in primary mouse hepatocytes of Tg mice using an siRNA at 5 concentrations**

| Compound | 20nM | 4nM | 0.8nM | 0.16nM | 0.032nM | IC50 (nM) |
|---|---|---|---|---|---|---|
| DR002358 | 3.4% | 9.2% | 31.2% | 74.6% | 98.5% | 0.3240 |
| DR002359 | 3.9% | 7.4% | 22.6% | 46.9% | 67.5% | 0.2943 |
| DR002360 | 19.6% | 38.4% | 80.0% | 118.3% | 109.2% | 1.7500 |
| DR002361 | 6.0% | 11.9% | 35.3% | 56.9% | 80.0% | 0.7026 |
| DR002362 | 11.6% | 19.0% | 48.5% | 71.2% | 82.1% | 0.6556 |
| DR002363 | 10.4% | 13.9% | 34.8% | 44.1% | 70.3% | 0.8480 |
| DR002364 | 6.0% | 13.3% | 39.7% | 75.1% | 81.9% | 0.6101 |
| DR002221 | 11.8% | 24.4% | 42.9% | 55.0% | 62.5% | 1.2410 |

### Example 9 In vivo activity screening

Experimental animals: 40 humanized PCSK9 Tg mice that were male and 11- to 12-week-old were selected.

A baseline level of a PCSK9 protein was determined by ELISA on D-12 (namely, day 12 before administration, same below) and D-5 before administration of the animals, and the baseline level was defined as 100%.

On D0, a test compound was administered subcutaneously at 3 mg/kg by single administration, as shown in Table 16 for detail.

**Table 16 Administration details for in vivo activity screening**

| Group information | siRNA | Animal number | Administration dose | Administration volume | Administration route and frequency |
|---|---|---|---|---|---|
| Vehicle | PBS | 5 | 3 mg/kg | 5 mL/kg | Subcutaneous, single time |
| TA01 | DR002221 | 5 | 3 mg/kg | 5 mL/kg | Subcutaneous, single time |
| TA02 | DR005642 | 5 | 3 mg/kg | 5 mL/kg | Subcutaneous, single time |
| TA03 | DR005656 | 5 | 3 mg/kg | 5 mL/kg | Subcutaneous, single time |
| TA04 | DR002359 | 5 | 3 mg/kg | 5 mL/kg | Subcutaneous, single time |
| TA05 | DR005672 | 5 | 3 mg/kg | 5 mL/kg | Subcutaneous, single time |
| TA06 | DR005675 | 5 | 3 mg/kg | 5 mL/kg | Subcutaneous, single time |
| TA07 | DR005674 | 5 | 3 mg/kg | 5 mL/kg | Subcutaneous, single time |

On D7, D14, D21 and D28 after the administration, whole blood was taken, respectively. After standing was carried out at room temperature, serum was obtained by centrifugation, and a PCSK9 protein level was determined by ELISA. Results are shown in Table 17.

**Table 17 In vivo activity screening results**

| Compound | D7 | | D14 | | D21 | | D28 | |
|---|---|---|---|---|---|---|---|---|
| | Average | SD | Average | SD | Average | SD | Average | SD |
| PBS | 122.0% | 39.4% | 110.5% | 36.8% | 126.5% | 31.5% | 120.4% | 36.1% |
| DR002221 | 13.3% | 2.9% | 12.9% | 2.1% | 21.7% | 3.0% | 29.6% | 4.0% |
| DR005642 | 13.8% | 2.2% | 13.5% | 3.5% | 19.9% | 5.8% | 22.4% | 5.5% |
| DR005656 | 15.8% | 4.4% | 15.0% | 5.8% | 26.3% | 12.3% | 27.6% | 11.9% |
| DR002359 | 11.6% | 3.3% | 17.7% | 7.0% | 24.4% | 20.7% | 39.7% | 5.1% |
| DR005672 | 11.3% | 2.3% | 9.2% | 2.0% | 16.1% | 3.7% | 25.9% | 8.3% |
| DR005675 | 12.4% | 1.4% | 16.2% | 4.0% | 25.4% | 4.8% | 49.0% | 13.5% |
| DR005674 | 7.2% | 1.9% | 8.4% | 1.8% | 8.5% | 3.3% | 11.1% | 3.2% |

## Claims

1. A small interfering RNA (siRNA) for inhibiting expression of proprotein convertase subtilisin/kexin type 9 (PCSK9) in a cell, the siRNA comprising a sense strand and an antisense strand that form a double-stranded region, wherein lengths of the sense strand and the antisense strand are each independently 15-30 nucleotides, and the antisense strand comprises a nucleotide sequence of at least 15 consecutive nucleotides of a nucleotide sequence shown in any one of SEQ ID NO: 105-208.

2. The siRNA according to claim 1, wherein the sense strand comprises a nucleotide sequence of at least 15 consecutive nucleotides of a nucleotide sequence shown in any one of SEQ ID NO: 1-104.

3. The siRNA according to claim 1 or 2, wherein a length of the double-stranded region is 15-25 base pairs, preferably 17-21 base pairs, more preferably 19-21 base pairs.

4. The siRNA according to any one of claims 1-3, wherein the siRNA comprises a sense strand sequence and an antisense strand sequence that are paired as shown in Table 3.

5. The siRNA according to any one of claims 1-3, wherein the antisense strand comprises a nucleotide sequence of at least 15 consecutive nucleotides, a nucleotide sequence of at least 16 consecutive nucleotides, a nucleotide sequence of at least 17 consecutive nucleotides, a nucleotide sequence of at least 18 consecutive nucleotides, a nucleotide sequence of at least 19 consecutive nucleotides, or a nucleotide sequence of at least 20 consecutive nucleotides of a nucleotide sequence shown in any one of SEQ ID NO: 203, 204, 142, 207, 143, 197, 205, 206, 144, 147, 148, 149, and 150, and preferably, the antisense strand comprises the nucleotide sequence shown in any one of SEQ ID NO: 203, 204, 142, 207, 143, 197, 205, 206, 144, 147, 148, 149, and 150.

6. The siRNA according to any one of claims 1-3 and 5, wherein the sense strand comprises a nucleotide sequence of at least 15 consecutive nucleotides, a nucleotide sequence of at least 16 consecutive nucleotides, a nucleotide sequence of at least 17 consecutive nucleotides, a nucleotide sequence of at least 18 consecutive nucleotides, a nucleotide sequence of at least 19 consecutive nucleotides, or a nucleotide sequence of at least 20 consecutive nucleotides of a nucleotide sequence shown in any one of SEQ ID NO: 99, 100, 38, 103, 39, 93, 101, 102, 40, 43, 44, 45, and 46, and preferably, the sense strand comprises the nucleotide sequence shown in any one of SEQ ID NO: 99, 100, 38, 103, 39, 93, 101, 102, 40, 43, 44, 45, and 46.

7. The siRNA according to claim 6, wherein:
(a) the antisense strand comprises a nucleotide sequence shown in SEQ ID NO: 203, and the sense strand comprises a nucleotide sequence shown in SEQ ID NO: 99;
(b) the antisense strand comprises a nucleotide sequence shown in SEQ ID NO: 204, and the sense strand comprises a nucleotide sequence shown in SEQ ID NO: 100;
(c) the antisense strand comprises a nucleotide sequence shown in SEQ ID NO: 142, and the sense strand comprises a nucleotide sequence shown in SEQ ID NO: 38;
(d) the antisense strand comprises a nucleotide sequence shown in SEQ ID NO: 207, and the sense strand comprises a nucleotide sequence shown in SEQ ID NO: 103;
(e) the antisense strand comprises a nucleotide sequence shown in SEQ ID NO: 143, and the sense strand comprises a nucleotide sequence shown in SEQ ID NO: 39;
(f) the antisense strand comprises a nucleotide sequence shown in SEQ ID NO: 197, and the sense strand comprises a nucleotide sequence shown in SEQ ID NO: 93;
(g) the antisense strand comprises a nucleotide sequence shown in SEQ ID NO: 205, and the sense strand comprises a nucleotide sequence shown in SEQ ID NO: 101;
(h) the antisense strand comprises a nucleotide sequence shown in SEQ ID NO: 206, and the sense strand comprises a nucleotide sequence shown in SEQ ID NO: 102;
(i) the antisense strand comprises a nucleotide sequence shown in SEQ ID NO: 144, and the sense strand comprises a nucleotide sequence shown in SEQ ID NO: 40;
(j) the antisense strand comprises a nucleotide sequence shown in SEQ ID NO: 147, and the sense strand comprises a nucleotide sequence shown in SEQ ID NO: 43;
(k) the antisense strand comprises a nucleotide sequence shown in SEQ ID NO: 148, and the sense strand comprises a nucleotide sequence shown in SEQ ID NO: 44;
(l) the antisense strand comprises a nucleotide sequence shown in SEQ ID NO: 149, and the sense strand comprises a nucleotide sequence shown in SEQ ID NO: 45; or
(m) the antisense strand comprises a nucleotide sequence shown in SEQ ID NO: 150, and the sense strand comprises a nucleotide sequence shown in SEQ ID NO: 46.

8. The siRNA according to any one of claims 1-7, wherein substantially all nucleotides of the sense strand and substantially all nucleotides of the antisense strand are modified nucleotides, or all the nucleotides of the sense strand and all the nucleotides of the antisense strand are modified nucleotides.

9. The siRNA according to claim 8, wherein the sense strand and the antisense strand each independently comprise one or more nucleotide modifications selected from the following group: 2'-O-methyl modified nucleotides, 2'-fluoro modified nucleotides, 2'-deoxy- modified nucleotides, inosine ribonucleotides, abasic nucleotides, reverse abasic deoxyribonucleotides, nucleotides containing a thiophosphate group, vinyl phosphate modified nucleotides, locked nucleotides, 2'-amino- modified nucleotides, 2'-alkyl- modified nucleotides, morpholino nucleotides, aminophosphates, unnatural bases containing nucleotides, and terminal nucleotides or deoxyribonucleotides linked to cholesterol-based derivatives or a sebacamide dodecanoate group.

10. The siRNA according to claim 8, wherein the sense strand and the antisense strand each independently comprise one or more nucleotide modifications selected from the following group: 2'-O-methyl modified nucleotides, 2'-fluoro modified nucleotides, deoxyribonucleotides, or nucleotides containing a thiophosphate group.

11. The siRNA according to claim 8, wherein the antisense strand comprises a modified nucleotide sequence shown in Table 5 of the specification, and/or the sense strand comprises a modified nucleotide sequence shown in Table 4 of the specification.

12. The siRNA according to claim 8, wherein the siRNA comprises a modified sense strand sequence and a modified antisense strand sequence that are paired as shown in Table 6 of the specification.

13. The siRNA according to claim 8, wherein:
(a) the sense strand comprises CmsUmsAmGmAmCmCfUmGfUmdTUmUmGmCmUmUmUmUmGmsUm, and the antisense strand comprises AmsCfsAmAfAfAfGmCfAmAfAmAfCmAfGmGfUmCfUmAmGmsAmsAm;
(b) the sense strand comprises UmsGmsUmUmUmUmGfCfUfUmUmUmGmUmAmAmCmsUmsUm, and the antisense strand comprises AmsAfsGmUfUmAfCmAfAmAfAmGfCmAfAmAfAmCfAmsGfsGm;
(c) the sense strand comprises UmsGmsUmUmUmUmGfCfUfUmUmUmGmUmAmAmCmsUmsUm, and the antisense strand comprises AmsAfsGmUfUmAfCmAfAmAfAmGfCmAfAmAfAmCfAmsGfsGm;
(d) the sense strand comprises GmsUmsUmUmUmGmCfUfUfUmUmGmUmAmAmCmUmsUmsAm, and the antisense strand comprises UmsAfsAmGfUmUfAmCfAmAfAmAfGmCfAmAfAmAfCmsAfsGm;
(e) the sense strand comprises GmsUmsUmUmUmGmCfUfUfUmUmGmUmAmAmCmUmUmsAm, and the antisense strand comprises UmsAfsAmGfUmUfAmCfAmAfAmAfGmCfAmAfAmAfCmsAfsGm;
(f) the sense strand comprises GmsUmsUmUmUmGmCfUfUfUmUmGmUmAmAmCmUmUmAm, and the antisense strand comprises UmsAfsAmGfUmUfAmCfAmAfAmAfGmCfAmAfAmAfCmsAfsGm;
(g) the sense strand comprises UmsUmsUmUmGmCmUfUfUfUmGmUmAmAmCmUmUmsGmsAm, and the antisense strand comprises UmsCfsAmAfGmUfUmAfCmAfAmAfAmGfCmAfAmAfAmsCfsAm;
(h) the sense strand comprises UmsGmsCmUmUmUmUfGfUfAmAmCmUmUmGmAmAmsGmsAm, and the antisense strand comprises UmsCfsUmUfCmAfAmGfUmUfAmCfAmAfAmAfGmCfAmsAfsAm;
(i) the sense strand comprises GmsCmsUmUmUmUmGfUfAfAmCmUmUmGmAmAmGmsAmsUm, and the antisense strand comprises AmsUfsCmUfUmCfAmAfGmUfUmAfCmAfAmAfAmGfCmsAfsAm;
(j) the sense strand comprises CmsUmsUmUmUmGmUfAfAfCmUmUmGmAmAmGmAmsUmsAm, and the antisense strand comprises UmsAfsUmCfUmUfCmAfAmGfUmUfAmCfAmAfAmAfGmsCfsAm; or
(k) the sense strand comprises UmsUmsUmUmGmUmAfAfCfUmUmGmAmAmGmAmUmsAmsUm, and the antisense strand comprises AmsUfsAmUfCmUfUmCfAmAfGmUfUmAfCmAfAmAfAmsGfsCm.

14. The siRNA according to any one of claims 1-13, wherein the siRNA is further conjugated with a ligand moiety containing N-acetylgalactosamine through a phosphate group or a thiophosphate group, and preferably, the sense strand of the siRNA is conjugated with the ligand moiety through the phosphate group or the thiophosphate group.

15. The siRNA according to claim 14, wherein a 3' end of the sense strand is conjugated with the ligand moiety through the phosphate group or the thiophosphate group.

16. The siRNA according to claim 14 or 15, wherein the ligand moiety comprises a conjugated group shown in Formula (X'): 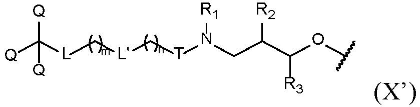 wherein, 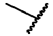 represents a position connected with a biomolecule;
Q is independently H, 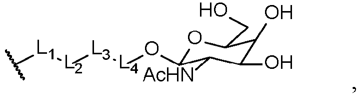 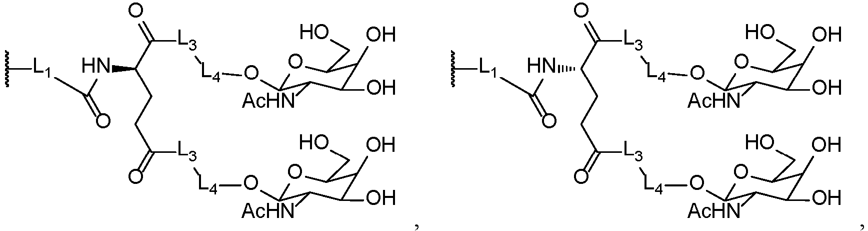 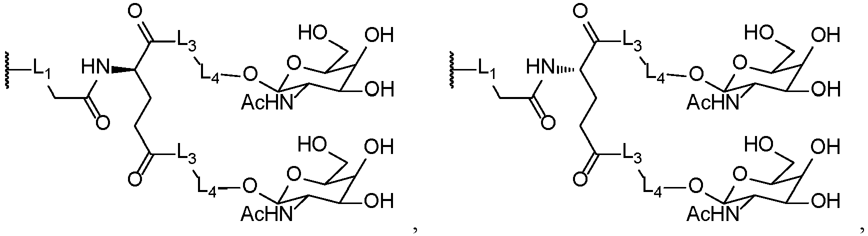 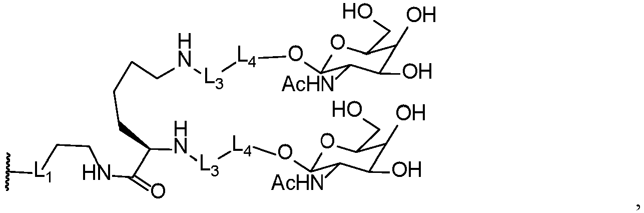 or 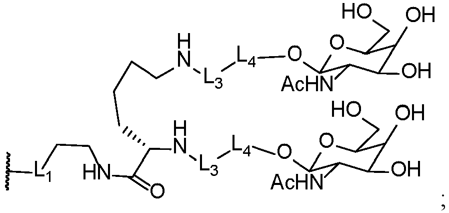
wherein L₁ is a bond, -CH₂-, -CH₂CH₂-, -C(O)-, -CH₂O-, -CH₂O-CH₂CH₂O-, or -NHC(O)-(CH₂NHC(O))ₐ-;
L₂ is a bond or -CH₂CH₂C(O)-;
L₃ is a bond, -(NHCH₂CH₂)_{b}-, -(NHCH₂CH₂CH₂)_{b}-, or -C(O)CH₂-;
L₄ is -(OCH₂CH₂)_{c}-, -(OCH₂CH₂CH₂)_{c}-, -(OCH₂CH₂CH₂CH₂)_{c}-, - (OCH₂CH₂CH₂CH₂CH₂)_{c}-, or -NHC(O)-(CH₂)_{d}-;
wherein a is 0, 1, 2, or 3;
b is 1, 2, 3, 4, or 5;
c is 1, 2, 3, 4, or 5;
d is 1, 2, 3, 4, 5, 6, 7, or 8;
L is a bond, -CH₂O-, or -NHC(O)-;
L' is a bond, -C(O)NH-, -NHC(O)-, or -O(CH₂CH₂O)ₑ-;
wherein e is 1, 2, 3, 4, or 5;
T is a bond, -CH₂-, -C(O)-, -M-, -CH₂-M-, or -C(O)-M-;
wherein M is 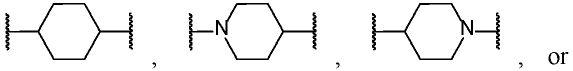 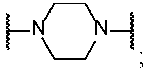
R₁ and R₂ together form -CH₂CH₂O- or -CH₂CH(R)-O-, and R₃ is H;
or the R₁ and the R₃ together form -C₁₋₂ alkylene-, and the R₂ is H;
wherein R is -OR', -CH₂OR', or -CH₂CH₂OR', wherein R' is H, a hydroxyl protective group, or a solid support, and the hydroxyl protective group is preferably -C(O)CH₂CH₂C(O)OH or 4,4'-dimethoxytriphenylmethyl;
m is 0, 1, 2, 3, 4, 5, 6, 7, 8, 9, or 10; and
n is 0, 1, 2, 3, 4, 5, 6, 7, 8, 9, or 10.

17. The siRNA according to claim 16, wherein the conjugated group is shown in Formula (I'): 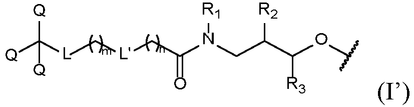 wherein, 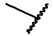 represents a position connected with a biomolecule;
Q is independently H, 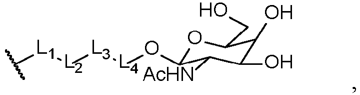 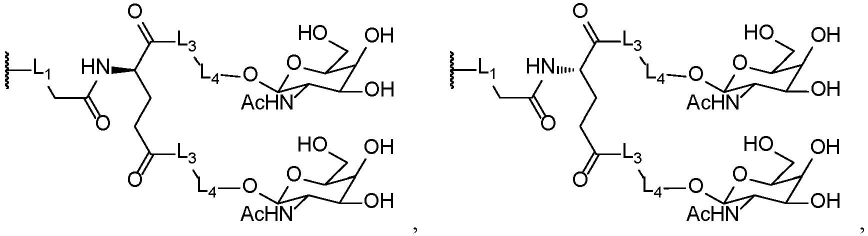 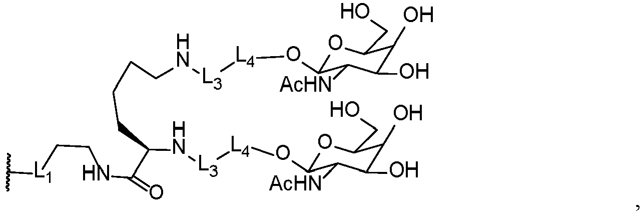 or 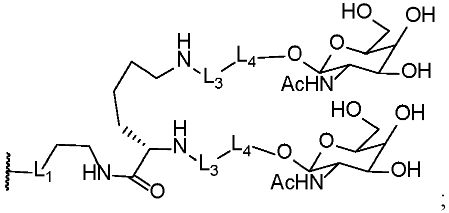
wherein L₁ is a bond, -CH₂-, -CH₂CH₂-, -C(O)-, -CH₂O-, -CH₂O-CH₂CH₂O-, or -NHC(O)-(CH₂NHC(O))ₐ-;
L₂ is a bond or -CH₂CH₂C(O)-;
L₃ is a bond, -(NHCH₂CH₂)_{b}-, -(NHCH₂CH₂CH₂)_{b}-, or -C(O)CH₂-;
L₄ is -(OCH₂CH₂)_{c}-, -(OCH₂CH₂CH₂)_{c}-, -(OCH₂CH₂CH₂CH₂)_{c}-, - (OCH₂CH₂CH₂CH₂CH₂)_{c}-, or -NHC(O)-(CH₂)_{d}-;
wherein a is 0, 1, 2, or 3;
b is 1, 2, 3, 4, or 5;
c is 1, 2, 3, 4, or 5;
d is 1, 2, 3, 4, 5, 6, 7, or 8;
L is -CH₂O- or -NHC(O)-;
L' is a bond, -C(O)NH-, or -NHC(O)-;
R₁ and R₂ together form -CH₂CH₂O- or -CH₂CH(R)-O-, and R₃ is H;
or the R₁ and the R₃ together form -C₁₋₂ alkylene-, and the R₂ is H;
wherein R is -OR', -CH₂OR', or -CH₂CH₂OR', wherein R' is H, a hydroxyl protective group, or a solid support, and the hydroxyl protective group is preferably -C(O)CH₂CH₂C(O)OH or 4,4'-dimethoxytriphenylmethyl;
m is 0, 1, 2, 3, 4, 5, 6, 7, 8, 9, or 10; and
n is 0, 1, 2, 3, 4, 5, 6, 7, 8, 9, or 10.

18. The siRNA according to claim 17, wherein,
the Q is independently H or 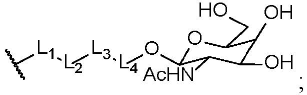
wherein the L₁ is -CH₂O- or -NHC(O)-(CH₂NHC(O))ₐ-;
the L₂ is -CH₂CH₂C(O)-;
the L₃ is -(NHCH₂CH₂)_{b}- or -(NHCH₂CH₂CH₂)_{b}-;
the L₄ is -(OCH₂CH₂)_{c}- or -NHC(O)-(CH₂)_{d}-;
wherein the a is 0, 1, 2, or 3;
the b is 1, 2, 3, 4, or 5;
the c is 1, 2, 3, 4, or 5;
the d is 1, 2, 3, 4, 5, 6, 7, or 8;
the L is -CH₂O-;
the L' is a bond;
the R₁ and the R₂ together form -CH₂CH₂O- or -CH₂CH(R)-O-, and the R₃ is H;
or the R₁ and the R₃ together form -C₁₋₂ alkylene-, and the R₂ is H;
wherein the R is -OR', -CH₂OR', or -CH₂CH₂OR', wherein the R' is H, a hydroxyl protective group, or a solid support, and the hydroxyl protective group is preferably -C(O)CH₂CH₂C(O)OH or 4,4'-dimethoxytriphenylmethyl;
the m is 0, 1, 2, 3, 4, 5, 6, 7, 8, 9, or 10; and
the n is 0, 1, 2, 3, 4, 5, 6, 7, 8, 9, or 10.

19. The siRNA according to claim 18, wherein the conjugated group is shown in Formula (I'-1), Formula (I'-2), or Formula (I'-3): 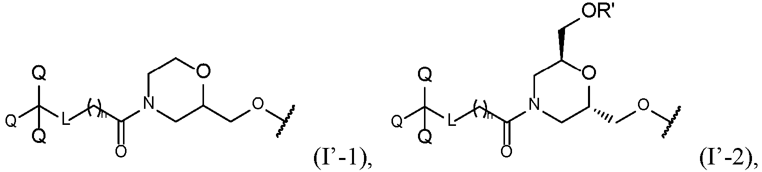 or 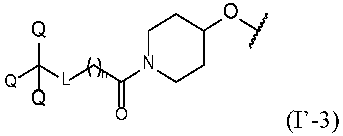 wherein, 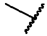 represents a position connected with a biomolecule;
Q is 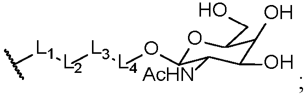
wherein L₁ is -CH₂O- or -NHC(O)-;
L₂ is -CH₂CH₂C(O)-;
L₃ is -(NHCH₂CH₂)_{b}- or -(NHCH₂CH₂CH₂)_{b}-;
L₄ is -(OCH₂CH₂)_{c}- or -NHC(O)-(CH₂)_{d}-;
wherein b is 1, 2, 3, 4, or 5;
c is 1, 2, 3, 4, or 5;
d is 1, 2, 3, 4, 5, 6, 7, or 8;
L is -CH₂O-;
R' is H, a hydroxyl protective group, or a solid support, and the hydroxyl protective group is preferably -C(O)CH₂CH₂C(O)OH or 4,4'-dimethoxytriphenylmethyl; and
n is 0, 1, 2, 3, 4, 5, 6, 7, 8, 9, or 10.

20. The siRNA according to claim 17, wherein,
the Q is independently H, 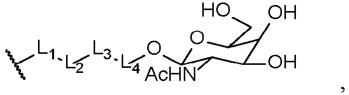 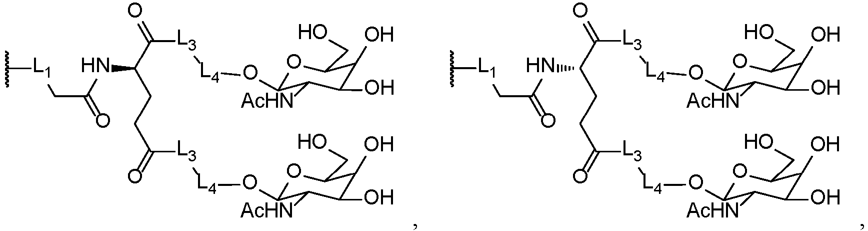 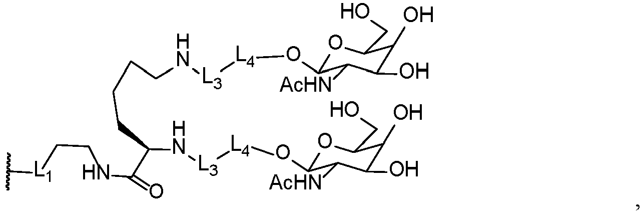 or 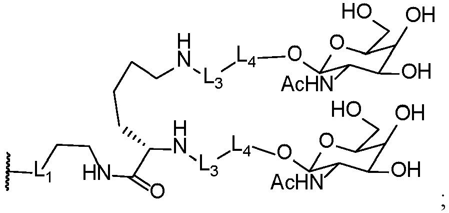
wherein the L₁ is -CH₂O-, -CH₂O-CH₂CH₂O-, or -NHC(O)-(CH₂NHC(O))ₐ-;
the L₂ is -CH₂CH₂C(O)-;
the L₃ is -(NHCH₂CH₂)_{b}-, -(NHCH₂CH₂CH₂)_{b}-, or -C(O)CH₂-;
the L₄ is -(OCH₂CH₂)_{c}- or -NHC(O)-(CH₂)_{d}-;
wherein the a is 0, 1, 2, or 3;
the b is 1, 2, 3, 4, or 5;
the c is 1, 2, 3, 4, or 5;
the d is 1, 2, 3, 4, 5, 6, 7, or 8;
the L is -CH₂O- or -NHC(O)-;
the L' is a bond or -C(O)NH-;
the R₁ and the R₂ together form -CH₂CH₂O- or -CH₂CH(R)-O-, and the R₃ is H;
or the R₁ and the R₃ together form -C₁₋₂ alkylene-, and the R₂ is H;
wherein the R is -OR', -CH₂OR', or -CH₂CH₂OR', wherein the R' is H, a hydroxyl protective group, or a solid support, and the hydroxyl protective group is preferably -C(O)CH₂CH₂C(O)OH or 4,4'-dimethoxytriphenylmethyl;
the m is 0, 1, 2, 3, 4, 5, 6, 7, 8, 9, or 10; and
the n is 0, 1, 2, 3, 4, 5, 6, 7, 8, 9, or 10.

21. The siRNA according to claim 20, wherein the conjugated group is shown in Formula (II'-1) or Formula (II'-2): 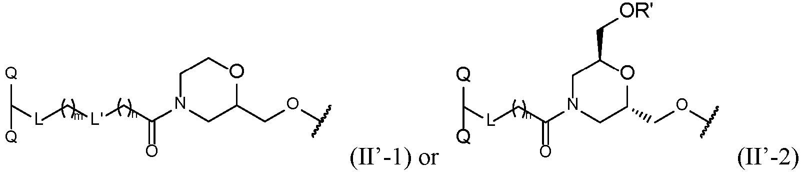 wherein, 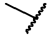 represents a position connected with a biomolecule;
Q is independently 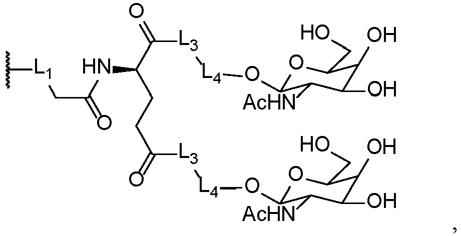 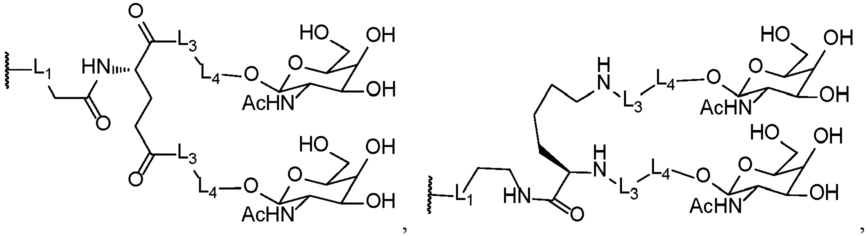 or 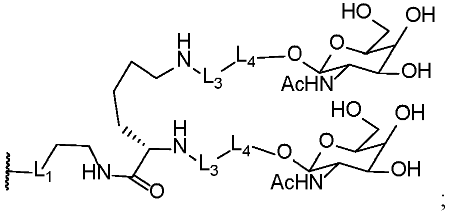
wherein L₁ is -CH₂O- or -CH₂O-CH₂CH₂O-;
L₃ is -(NHCH₂CH₂)_{b}-, -(NHCH₂CH₂CH₂)_{b}-, or -C(O)CH₂-;
L₄ is -(OCH₂CH₂)_{c}- or -NHC(O)-(CH₂)_{d}-;
wherein b is 1, 2, 3, 4, or 5;
c is 1, 2, 3, 4, or 5;
d is 1, 2, 3, 4, 5, 6, 7, or 8;
L is -NHC(O)-;
L' is a bond or -C(O)NH-;
R' is H, a hydroxyl protective group, or a solid support, and the hydroxyl protective group is preferably -C(O)CH₂CH₂C(O)OH or 4,4'-dimethoxytriphenylmethyl;
m is 0, 1, 2, 3, 4, 5, 6, 7, 8, 9, or 10; and
n is 0, 1, 2, 3, 4, 5, 6, 7, 8, 9, or 10.

22. The siRNA according to claim 17, wherein,
the Q is independently H, 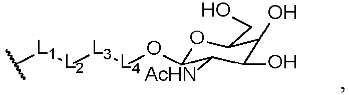 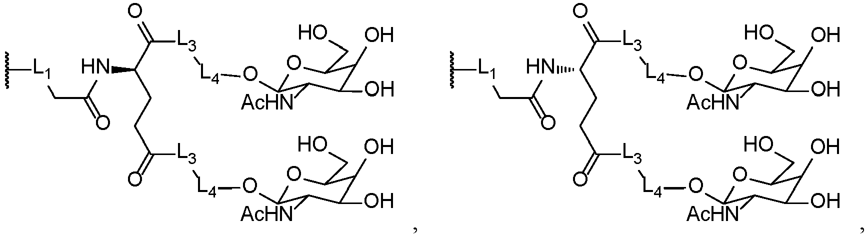 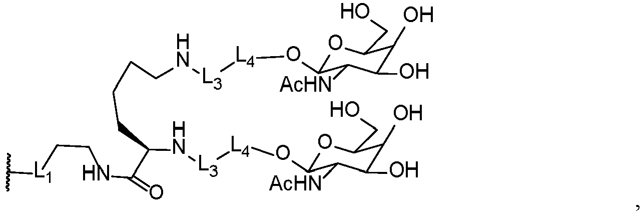 or 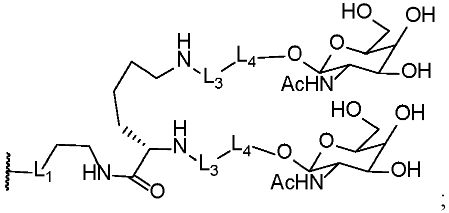
wherein the L₁ is -CH₂-, -C(O)-, -CH₂O-, -CH₂O-CH₂CH₂O-, or -NHC(O)-(CH₂NHC(O))ₐ-;
the L₂ is a bond;
the L₃ is -(NHCH₂CH₂)_{b}-, -(NHCH₂CH₂CH₂)_{b}-, or -C(O)CH₂-;
the L₄ is -(OCH₂CH₂)_{c}- or -NHC(O)-(CH₂)_{d}-;
wherein the a is 0, 1, 2, or 3;
the b is 1, 2, 3, 4, or 5;
the c is 1, 2, 3, 4, or 5;
the d is 1, 2, 3, 4, 5, 6, 7, or 8;
the L is -CH₂O- or -NHC(O)-;
the L' is a bond or -C(O)NH-;
the R₁ and the R₂ together form -CH₂CH₂O- or -CH₂CH(R)-O-, and the R₃ is H;
or the R₁ and the R₃ together form -C₁₋₂ alkylene-, and the R₂ is H;
wherein the R is -OR', -CH₂OR', or -CH₂CH₂OR', wherein the R' is H, a hydroxyl protective group, or a solid support, and the hydroxyl protective group is preferably -C(O)CH₂CH₂C(O)OH or 4,4'-dimethoxytriphenylmethyl;
the m is 0, 1, 2, 3, 4, 5, 6, 7, 8, 9, or 10; and
the n is 0, 1, 2, 3, 4, 5, 6, 7, 8, 9, or 10.

23. The siRNA according to claim 22, wherein the conjugated group is shown in Formula (II'-2): 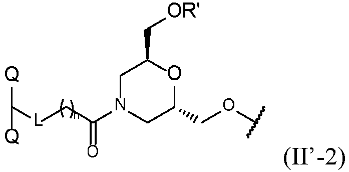 wherein, 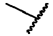 represents a position connected with a biomolecule;
Q is independently 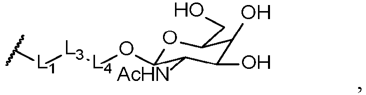 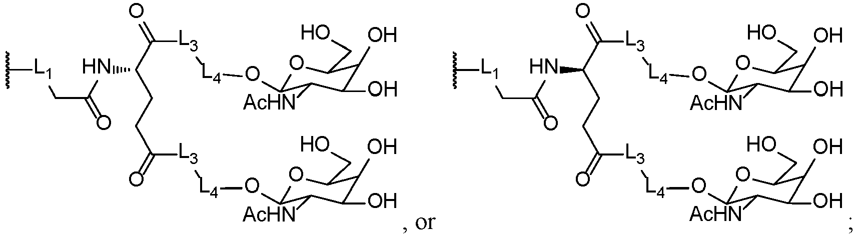
wherein L₁ is -CH₂- or -C(O)-;
L₃ is -(NHCH₂CH₂)_{b}-;
L₄ is -(OCH₂CH₂)_{c}-;
wherein b is 1, 2, 3, 4, or 5;
c is 1, 2, 3, 4, or 5;
L is -CH₂O- or -NHC(O)-;
R' is H, a hydroxyl protective group, or a solid support, and the hydroxyl protective group is preferably -C(O)CH₂CH₂C(O)OH or 4,4'-dimethoxytriphenylmethyl; and
n is 0, 1, 2, 3, 4, 5, 6, 7, 8, 9, or 10.

24. The siRNA according to claim 16, wherein:
the Q is independently H, 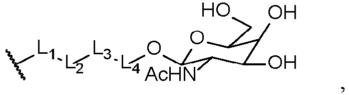 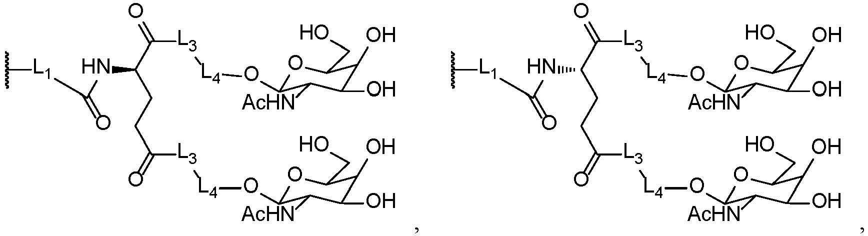 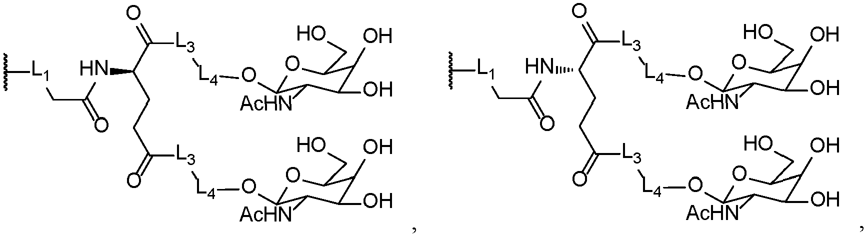 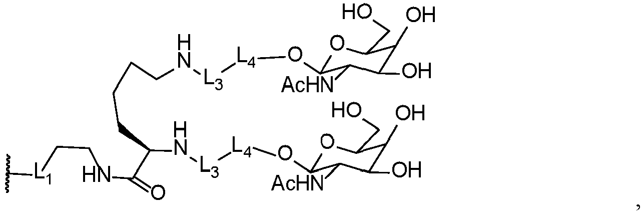 or 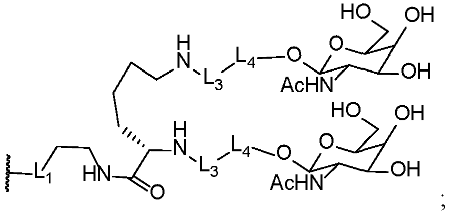
wherein the L1 is a bond, -CH₂-, -CH₂CH₂-, -C(O)-, -CH₂O-, -CH₂O-CH₂CH₂O-, or -NHC(O)-(CH₂NHC(O))ₐ-;
the L₂ is a bond or -CH₂CH₂C(O)-;
the L₃ is a bond, -(NHCH₂CH₂)_{b}-, -(NHCH₂CH₂CH₂)_{b}-, or -C(O)CH₂-;
the L₄ is -(OCH₂CH₂)_{c}-, -(OCH₂CH₂CH₂)_{c}-, -(OCH₂CH₂CH₂CH₂)_{c}-, - (OCH₂CH₂CH₂CH₂CH₂)_{c}-, or -NHC(O)-(CH₂)_{d}-;
wherein the a is 0, 1, 2, or 3;
the b is 1, 2, 3, 4, or 5;
the c is 1, 2, 3, 4, or 5;
the d is 1, 2, 3, 4, 5, 6, 7, or 8;
the L is a bond, -CH₂O-, or -NHC(O)-;
the L' is a bond, -C(O)NH-, -NHC(O)-, or -O(CH₂CH₂O)ₑ-;
wherein the e is 1, 2, 3, 4, or 5;
the T is a bond, -CH₂-, -M-, -CH₂-M-, or -C(O)-M-;
wherein the M is 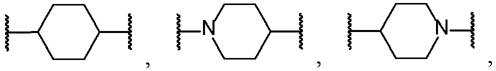 or 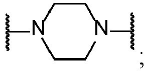
the R₁ and the R₂ together form -CH₂CH₂O- or -CH₂CH(R)-O-, and the R₃ is H;
or the R₁ and the R₃ together form -C₁₋₂ alkylene-, and the R₂ is H;
wherein the R is -OR', -CH₂OR', or -CH₂CH₂OR', wherein the R' is H, a hydroxyl protective group, or a solid support, and the hydroxyl protective group is preferably -C(O)CH₂CH₂C(O)OH or 4,4'-dimethoxytriphenylmethyl;
the m is 0, 1, 2, 3, 4, 5, 6, 7, 8, 9, or 10; and
the n is 0, 1, 2, 3, 4, 5, 6, 7, 8, 9, or 10.

25. The siRNA according to claim 24, wherein,
the T is -M-, -CH₂-M-, or -C(O)-M-, wherein the M is

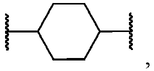

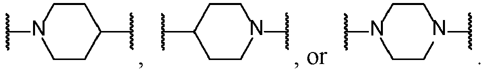

26. The siRNA according to claim 24 or 25, wherein,
the Q is independently H or 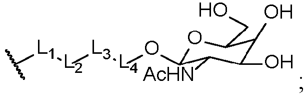
wherein the L₁ is -CH₂O- or -NHC(O)-(CH₂NHC(O))ₐ-;
the L₂ is -CH₂CH₂C(O)-;
the L₃ is -(NHCH₂CH₂)_{b}- or -(NHCH₂CH₂CH₂)_{b}-;
the L₄ is -(OCH₂CH₂)_{c}- or -NHC(O)-(CH₂)_{d}-;
wherein the a is 0, 1, 2, or 3;
the b is 1, 2, 3, 4, or 5;
the c is 1, 2, 3, 4, or 5;
the d is 1, 2, 3, 4, 5, 6, 7, or 8;
the L is a bond or -CH₂O-;
the L' is a bond or -O(CH₂CH₂O)ₑ-;
wherein the e is 1, 2, 3, 4, or 5;
the R₁ and the R₂ together form -CH₂CH₂O- or -CH₂CH(R)-O-, and the R₃ is H;
or the R₁ and the R₃ together form -C₁₋₂ alkylene-, and the R₂ is H;
wherein the R is -OR', -CH₂OR', or -CH₂CH₂OR', wherein the R' is H, a hydroxyl protective group, or a solid support, and the hydroxyl protective group is preferably -C(O)CH₂CH₂C(O)OH or 4,4'-dimethoxytriphenylmethyl;
the m is 0, 1, 2, 3, 4, 5, 6, 7, 8, 9, or 10;
the n is 0, 1, 2, 3, 4, 5, 6, 7, 8, 9, or 10; and
the T is as defined according to claim 24 or 25.

27. The siRNA according to claim 26, wherein the conjugated group is shown in Formula (III'-1), Formula (III'-2), or Formula (III'-3): 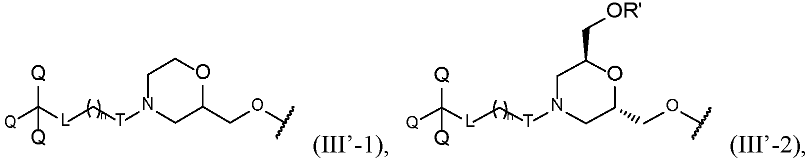 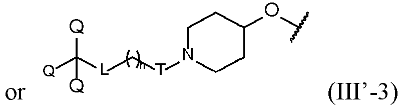 wherein,
Q is 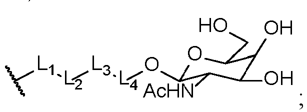
wherein L₁ is -CH₂O- or -NHC(O)-;
L₂ is -CH₂CH₂C(O)-;
L₃ is -(NHCH₂CH₂)_{b}- or -(NHCH₂CH₂CH₂)_{b}-;
L₄ is -(OCH₂CH₂)_{c}- or -NHC(O)-(CH₂)_{d}-;
wherein b is 1, 2, 3, 4, or 5;
c is 1, 2, 3, 4, or 5;
d is 1, 2, 3, 4, 5, 6, 7, or 8;
L is a bond or -CH₂O-;
wherein R' is H, a hydroxyl protective group, or a solid support, and the hydroxyl protective group is preferably -C(O)CH₂CH₂C(O)OH or 4,4'-dimethoxytriphenylmethyl;
n is 0, 1, 2, 3, 4, 5, 6, 7, 8, 9, or 10; and
T is as defined according to claim 24 or 25.

28. The siRNA according to claim 24 or 25, wherein,
the Q is independently H, 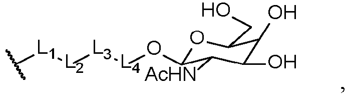 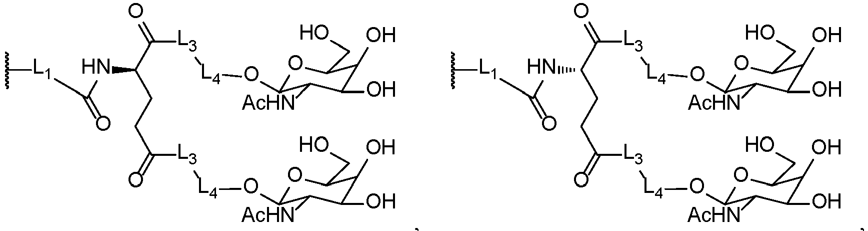 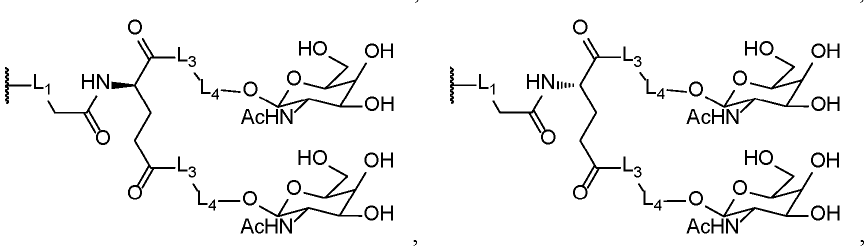 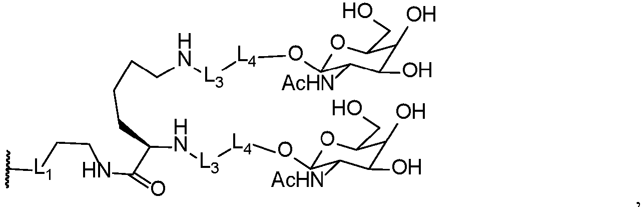 or 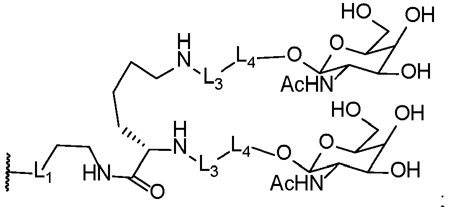
wherein the L₁ is -CH₂-, -CH₂O-, or -C(O)-;
the L₂ is a bond;
the L₃ is -(NHCH₂CH₂)_{b}-, -(NHCH₂CH₂CH₂)_{b}-, or -C(O)CH₂-;
the L₄ is -(OCH₂CH₂)_{c}- or -NHC(O)-(CH₂)_{d}-;
wherein the b is 1, 2, 3, 4, or 5;
the c is 1, 2, 3, 4, or 5;
the d is 1, 2, 3, 4, 5, 6, 7, or 8;
the L is a bond or -NHC(O)-;
the L' is a bond;
the R₁ and the R₂ together form -CH₂CH₂O- or -CH₂CH(R)-O-, and the R₃ is H;
or the R₁ and the R₃ together form -C₁₋₂ alkylene-, and the R₂ is H;
wherein the R is -OR', -CH₂OR', or -CH₂CH₂OR', wherein the R' is H, a hydroxyl protective group, or a solid support, and the hydroxyl protective group is preferably -C(O)CH₂CH₂C(O)OH or 4,4'-dimethoxytriphenylmethyl;
the m is 0, 1, 2, 3, 4, 5, 6, 7, 8, 9, or 10;
the n is 0, 1, 2, 3, 4, 5, 6, 7, 8, 9, or 10; and
the T is as defined according to claim 24 or 25.

29. The siRNA according to claim 24 or 25, wherein the conjugated group is shown in Formula (IV-1) or Formula (IV-2): 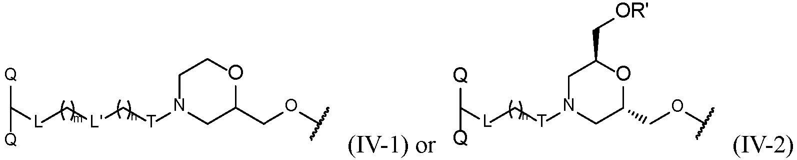 wherein,
Q is independently 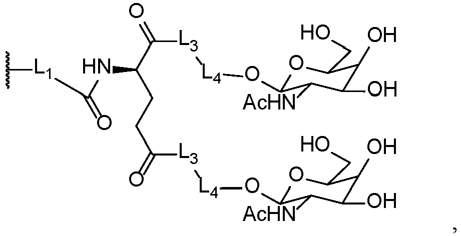 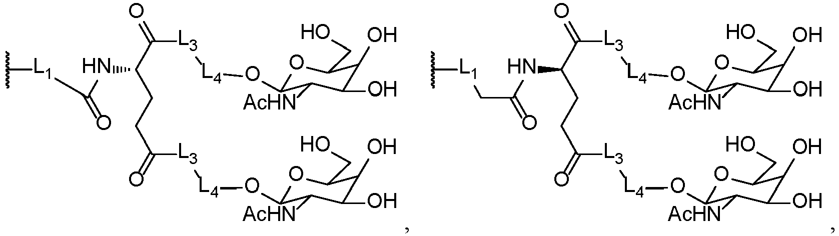 or 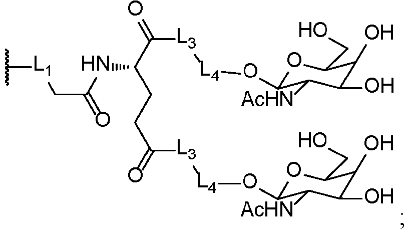
wherein L₁ is -CH₂-, -CH₂O-, or -C(O)-;
L₃ is -(NHCH₂CH₂)_{b}-, -(NHCH₂CH₂CH₂)_{b}-, or -C(O)CH₂-;
L₄ is -(OCH₂CH₂)_{c}- or -NHC(O)-(CH₂)_{d}-;
wherein b is 1, 2, 3, 4, or 5;
c is 1, 2, 3, 4, or 5;
d is 1, 2, 3, 4, 5, 6, 7, or 8;
L is a bond or -NHC(O)-;
L' is a bond;
wherein R' is H, a hydroxyl protective group, or a solid support, and the hydroxyl protective group is preferably -C(O)CH₂CH₂C(O)OH or 4,4'-dimethoxytriphenylmethyl;
m is 0, 1, 2, 3, 4, 5, 6, 7, 8, 9, or 10;
n is 0, 1, 2, 3, 4, 5, 6, 7, 8, 9, or 10; and
T is as defined according to claim 24 or 25.

30. The siRNA according to claim 16, wherein:
the Q is independently H, 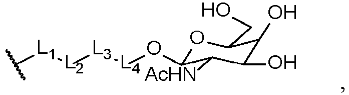 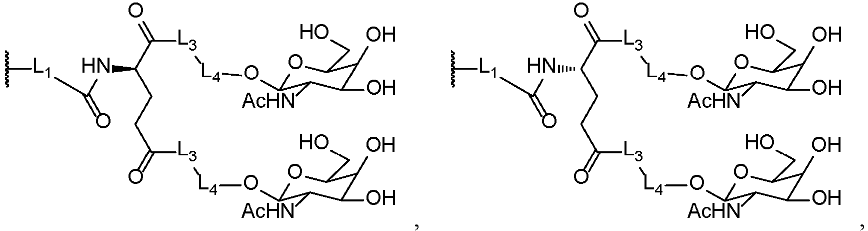 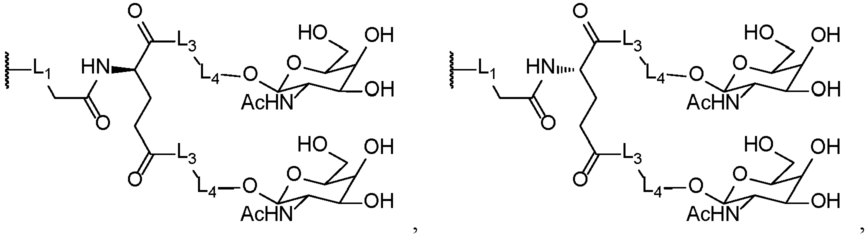 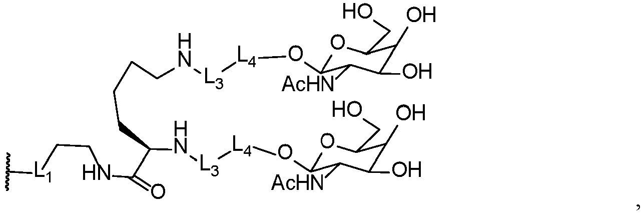
or 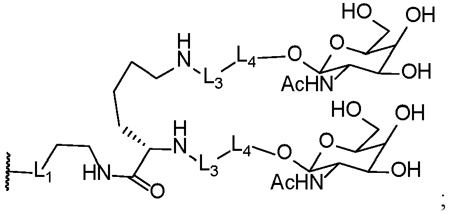
wherein the L₁ is a bond, -CH₂-, -CH₂CH₂-, -C(O)-, -CH₂O-, -CH₂O-CH₂CH₂O-, or -NHC(O)-(CH₂NHC(O))ₐ-;
the L₂ is a bond or -CH₂CH₂C(O)-;
the L₃ is a bond, -(NHCH₂CH₂)_{b}-, -(NHCH₂CH₂CH₂)_{b}-, or -C(O)CH₂-;
the L₄ is -(OCH₂CH₂)_{c}-, -(OCH₂CH₂CH₂)_{c}-, -(OCH₂CH₂CH₂CH₂)_{c}-, - (OCH₂CH₂CH₂CH₂CH₂)_{c}-, or -NHC(O)-(CH₂)_{d}-;
wherein the a is 0, 1, 2, or 3;
the b is 1, 2, 3, 4, or 5;
the c is 1, 2, 3, 4, or 5;
the d is 1, 2, 3, 4, 5, 6, 7, or 8;
the L is a bond, -CH₂O-, or -NHC(O)-;
the L' is -O(CH₂CH₂O)ₑ-;
wherein the e is 1, 2, 3, 4, or 5;
the T is a bond, -CH₂-, -C(O)-, -M-, -CH₂-M-, or -C(O)-M-;
wherein the M is 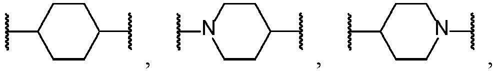 or 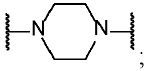
the R₁ and the R₂ together form -CH₂CH₂O- or -CH₂CH(R)-O-, and the R₃ is H;
or the R₁ and the R₃ together form -C₁₋₂ alkylene-, and the R₂ is H;
wherein the R is -OR', -CH₂OR', or -CH₂CH₂OR', wherein the R' is H, a hydroxyl protective group, or a solid support, and the hydroxyl protective group is preferably -C(O)CH₂CH₂C(O)OH or 4,4'-dimethoxytriphenylmethyl;
the m is 0, 1, 2, 3, 4, 5, 6, 7, 8, 9, or 10; and
the n is 0, 1, 2, 3, 4, 5, 6, 7, 8, 9, or 10.

31. The siRNA according to claim 16, wherein the conjugated group is selected from the following:
| Nu mbe r | Structure |
|---|---|
| 1 | 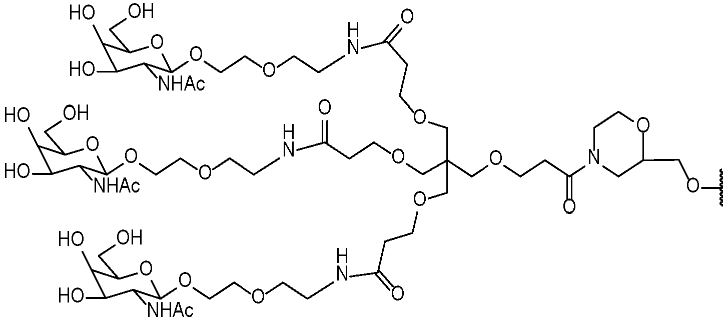 |
| 2 | 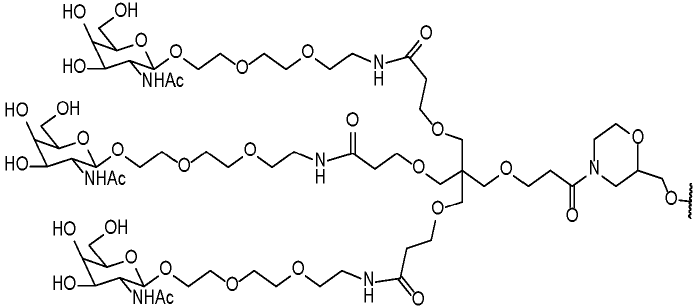 |
| 3 | 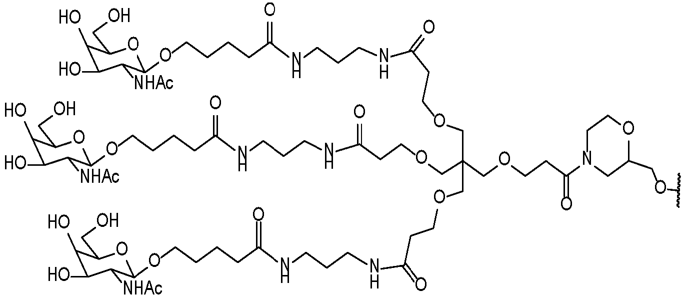 |
| 4 | 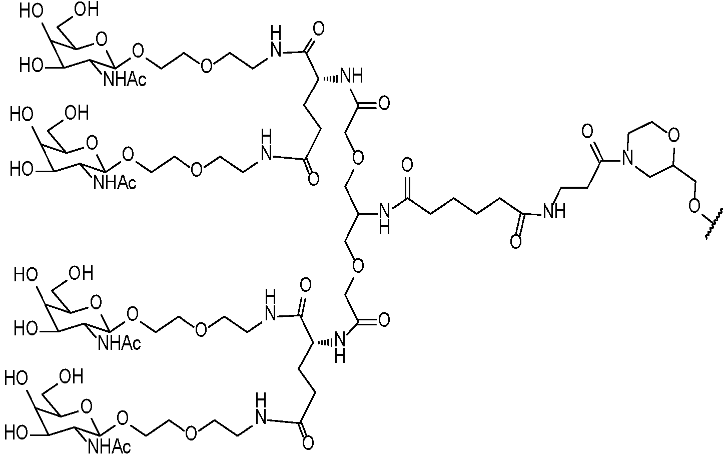 |
| 5 | 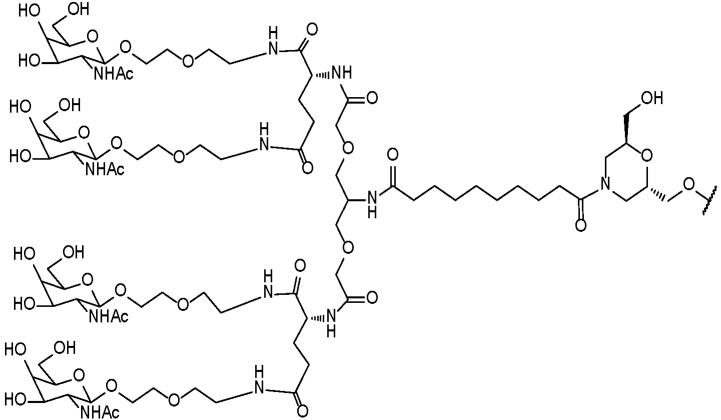 |
| 6 | 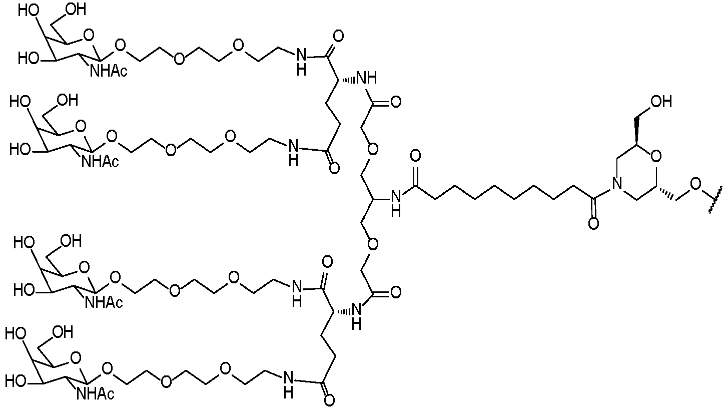 |
| 7 | 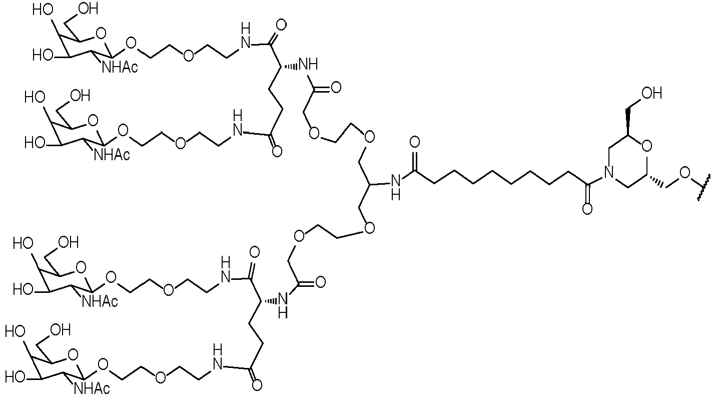 |
| 8 | 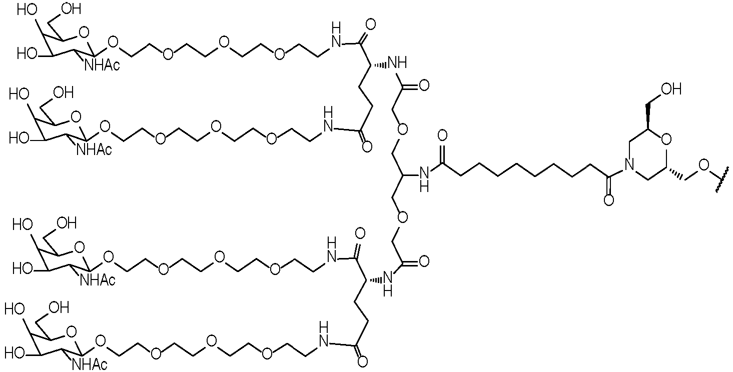 |
| 9 | 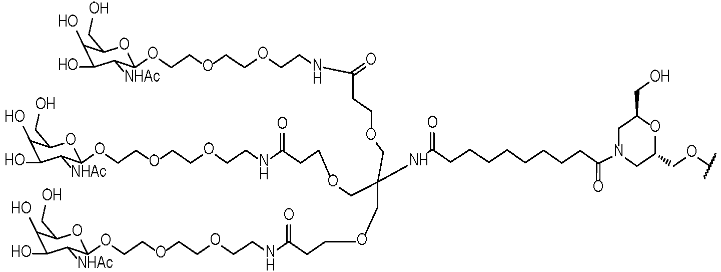 |
| 10 | 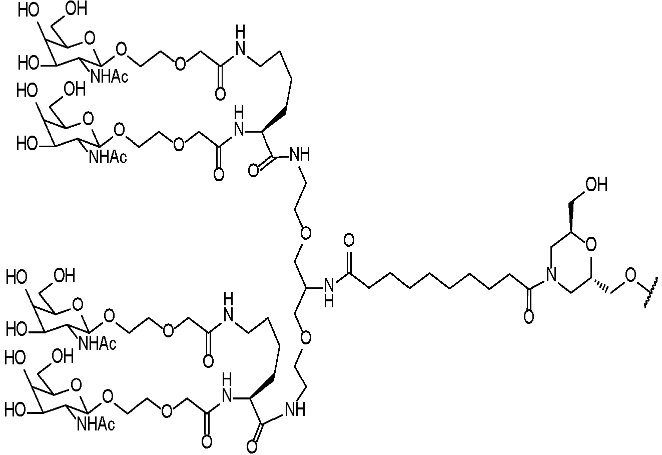 |
| 11 | 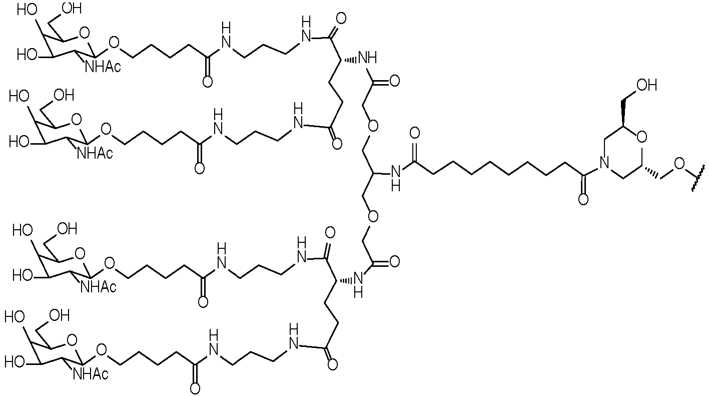 |
| 12 | 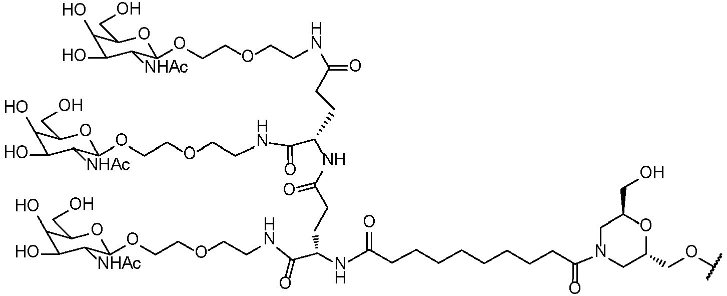 |
| 13 | 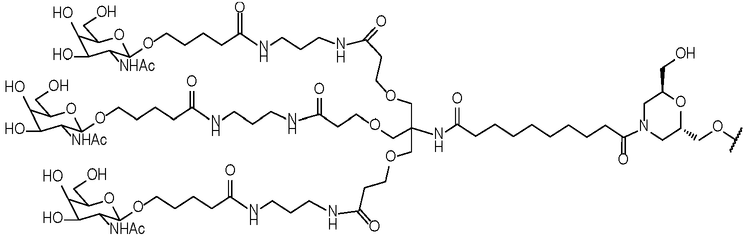 |
| 14 | 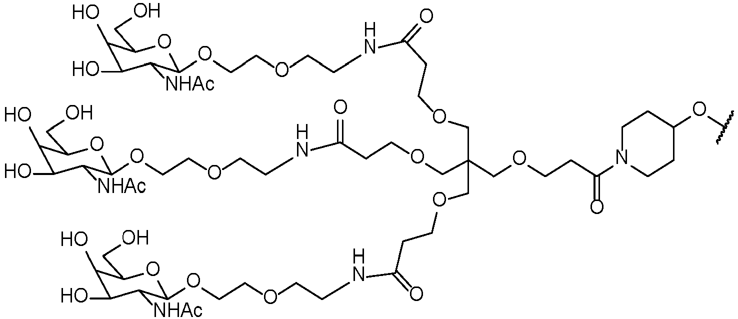 |
| 15 | 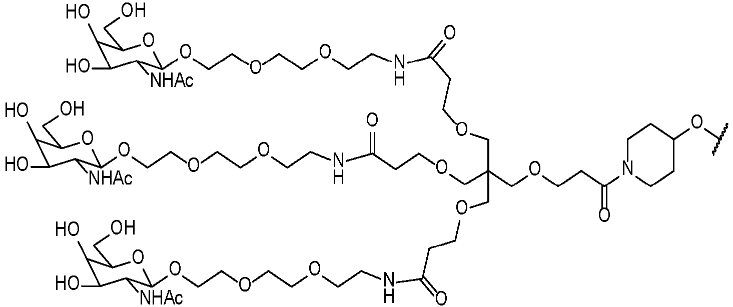 |
| 16 | 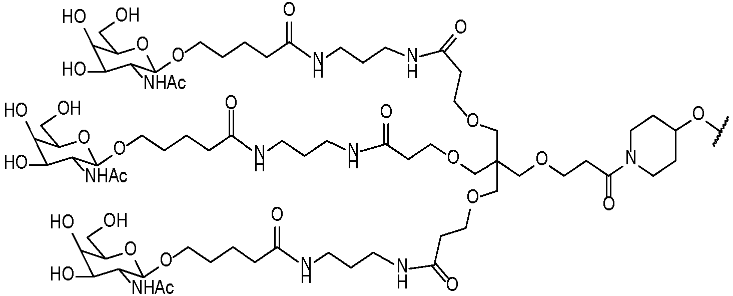 ∘ |

32. The siRNA according to claim 16, wherein the conjugated group is selected from the following:
| Nu mbe r | Structure |
|---|---|
| 17 | 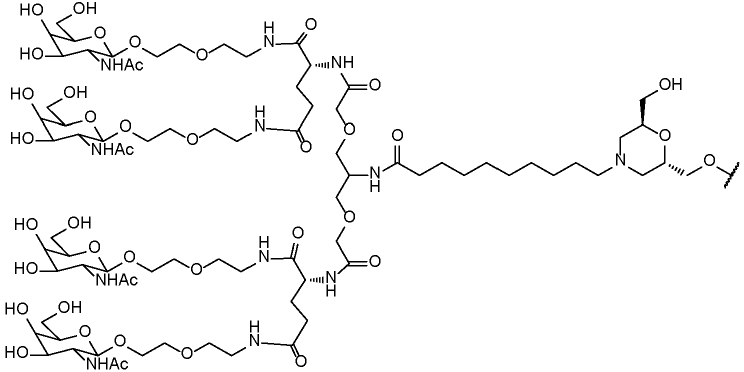 |
| 18 | 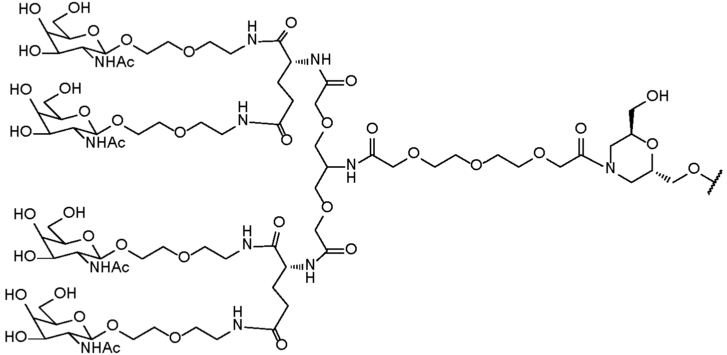 |
| 19 | 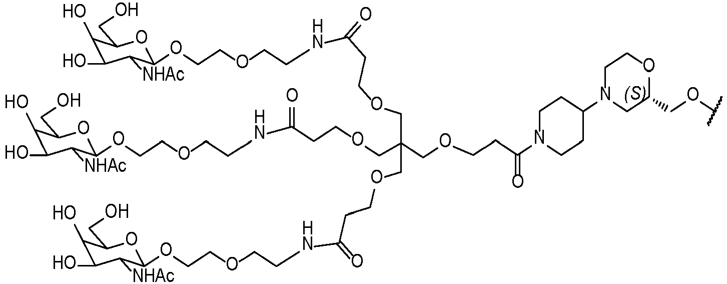 |
| 20 | 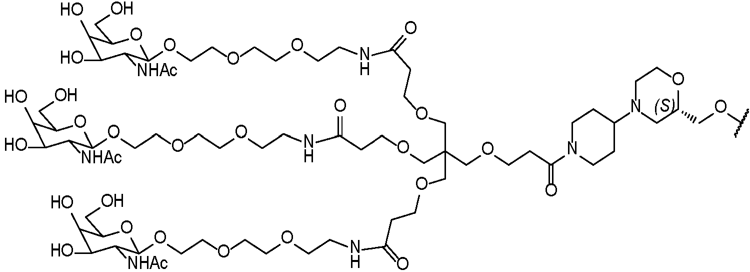 |
| 21 | 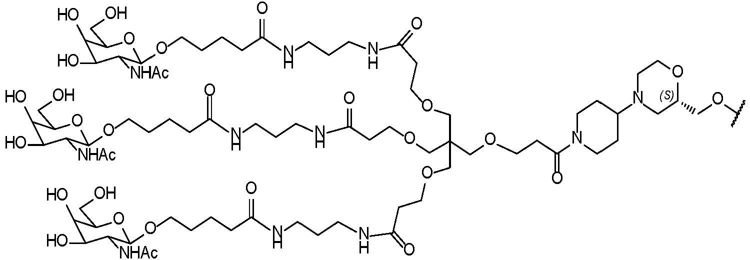 |
| 22 | 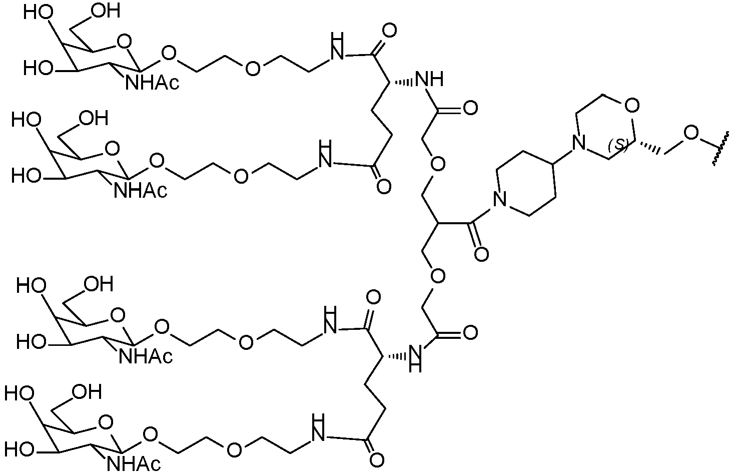 |
| 23-1 | 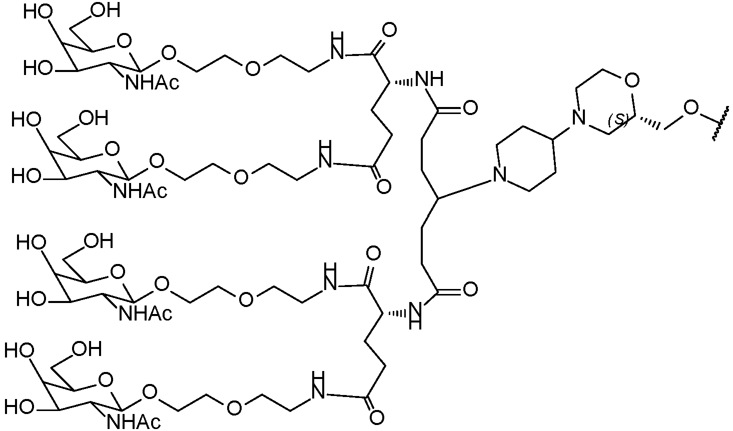 |
| 23-2 | 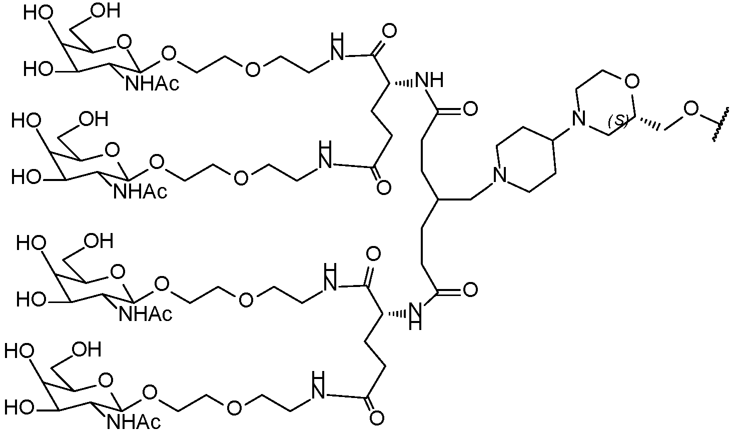 |
| 24 | 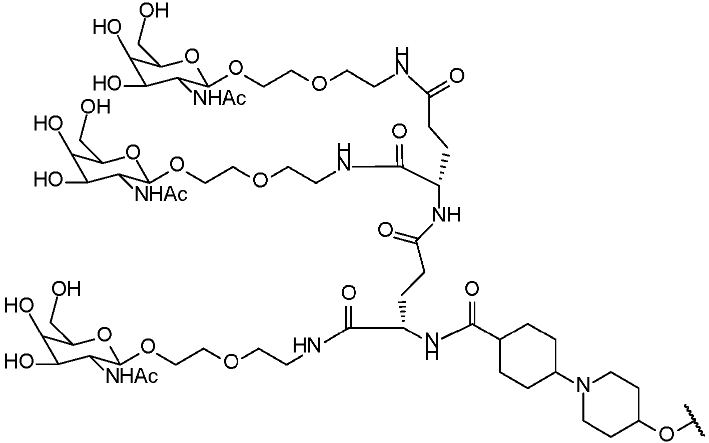 |
| 25 | 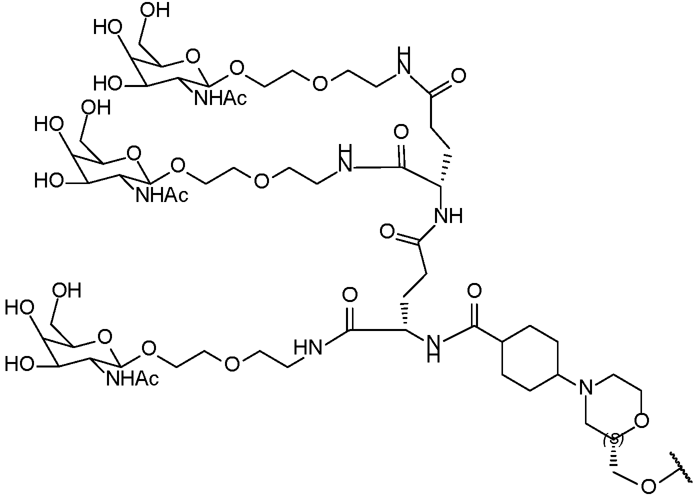 |
| 26 | 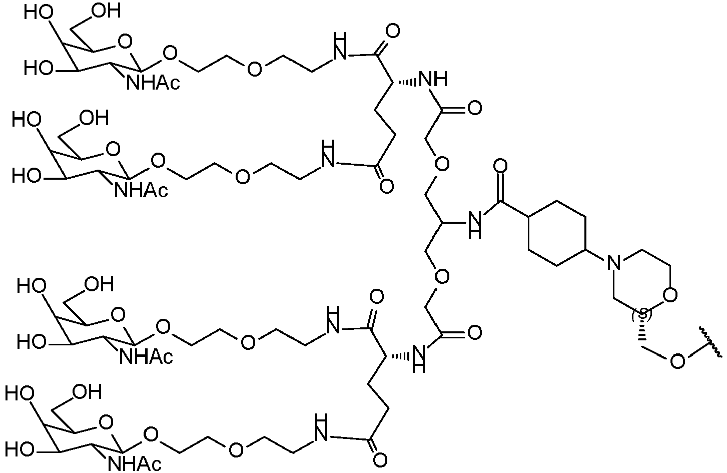 |
| 27 | 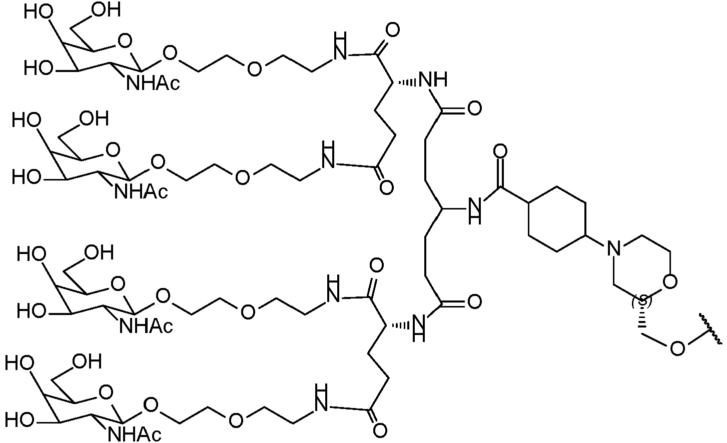 |
| 28 | 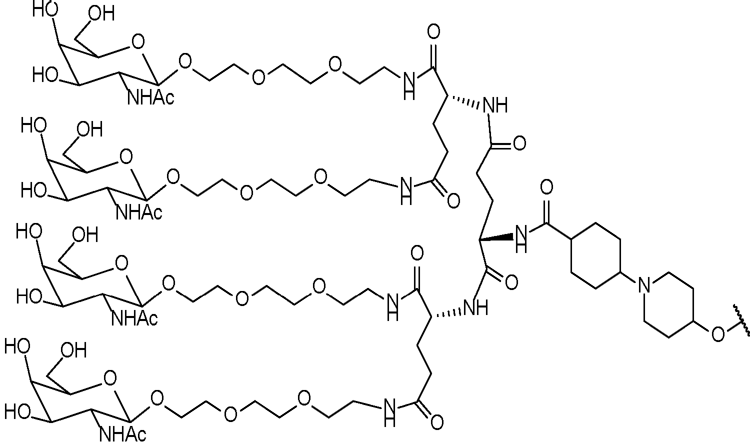 |
| 29 | 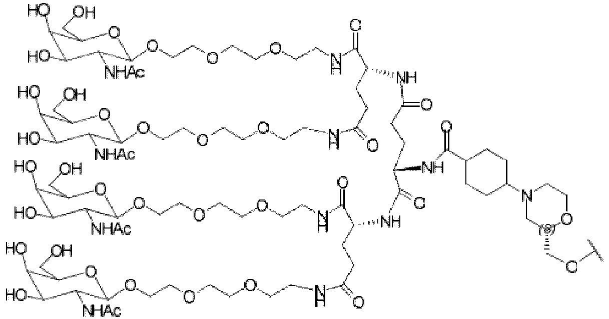 ∘ |

33. The siRNA according to any one of claims 1-32, wherein the ligand targets an asialoglycoprotein receptor (ASGPR).

34. The siRNA according to claim 14 or 15, wherein the ligand has the following structure: 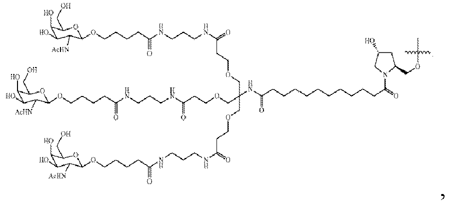 wherein 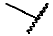 represents a position connected with the siRNA through the phosphate group or the thiophosphate group.

35. The siRNA according to claim 14 or 15, wherein the ligand has the following structure: 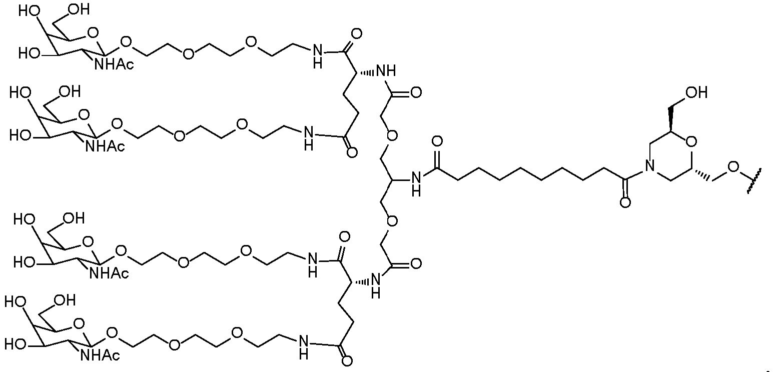 wherein 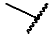 , represents a position connected with the sense strand of the siRNA through the phosphate group or the thiophosphate group.

36. The siRNA according to claim 14 or 15, wherein the ligand has the following structure: 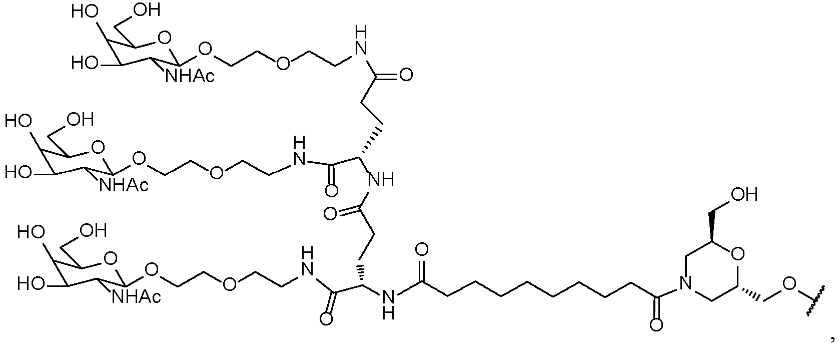 wherein 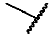 represents a position connected with the sense strand of the siRNA through the phosphate group or the thiophosphate group.

37. A cell, comprising the siRNA according to any one of claims 1-36.

38. A pharmaceutical composition, comprising the siRNA according to any one of claims 1-36 or the cell according to claim 37 and a pharmaceutically acceptable carrier or excipient.

39. A kit, comprising the siRNA according to any one of claims 1-36 or the cell according to claim 37.

40. A method for treating diseases or symptoms associated with expression of PCSK9 in a subject, wherein the method comprises a step of administering the siRNA according to any one of claims 1-36, the cell according to claim 37 or the pharmaceutical composition according to claim 38 to the subject.

41. The method according to claim 40, wherein the diseases or symptoms associated with expression of PCSK9 are cardiovascular diseases, and preferably, the cardiovascular diseases are selected from hyperlipidemia, hypercholesterolemia, coronary heart disease, myocardial infarction, stroke, or atherosclerosis.

42. The method according to claim 41, wherein the diseases or symptoms associated with expression of PCSK9 are hypercholesterolemia, and preferably, the hypercholesterolemia is selected from nonfamilial hypercholesterolemia, polygenic hypercholesterolemia, familial hypercholesterolemia, homozygous familial hypercholesterolemia, or heterozygous familial hypercholesterolemia.
